(19) Europäisches Patentamt
European Patent Office
Office européen des brevets

(11) Numéro de publication: **0 072 755 B1**

(12) **FASCICULE DE BREVET EUROPEEN**

(45) Date de publication du fascicule du brevet: **21.08.85**

(51) Int. Cl.⁴: **C 07 D 501/20**, C 07 D 498/04, A 61 K 31/545

(21) Numéro de dépôt: **82401531.7**

(22) Date de dépôt: **13.08.82**

(54) **Nouveaux derivés de la cephalosporine, leur préparation et les médicaments qui les contiennent.**

(30) Priorité: **17.08.81 FR 8115804**

(43) Date de publication de la demande: **23.02.83 Bulletin 83/8**

(45) Mention de la délivrance du brevet: **21.08.85 Bulletin 85/34**

(84) Etats contractants désignés: **AT BE CH DE FR GB IT LI LU NL SE**

(56) Documents cités:
**FR - A - 2 206 085**
**US - A - 3 962 223**

**Le dossier contient des informations techniques présentées postérieurement au dépôt de la demande et ne figurant pas dans le présent fascicule.**

(73) Titulaire: **RHONE-POULENC SANTE, Les Miroirs 18 Avenue d'Alsace, F-92400 Courbevoie Cedex (FR)**

(72) Inventeur: **Farge, Daniel, 30 rue des Pins Sylvestres, F-94320 Thiais (FR)**
Inventeur: **Le Roy, Pierre, 2 Allée des Cerisiers, F-94320 Thiais (FR)**
Inventeur: **Moutonnier, Claude, 3 rue Auguste Rodin, F-92350 Le Plessis-Robinson (FR)**
Inventeur: **Peyronel, Jean-François, 36 parc d'Ardenay, F-91120 Palaiseau (FR)**
Inventeur: **Plau, Bernard, 53 rue de Mesly, F-94000 Créteil (FR)**

(74) Mandataire: **Gaumont, Robert et al, RHONE-POULENC RECHERCHES Service Brevets Pharma 25, Quai Paul Doumer, F-92408 Courbevoie Cedex (FR)**

EP 0 072 755 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne des dérivés de thiazolylcéphalosporines de formule générale:

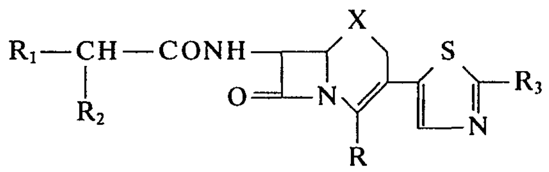

(I)

leurs sels, leur préparation et les compositions pharmaceutiques qui les contiennent.

Dans le brevet belge 793 448 ont été décrites des hétérocyclyl-3 céphalosporines actives sur germes gram-positifs et leur préparation.

La demande de brevet français 2 206 085 décrit entre autres des hétérocyclylcéphalosporines définies par une formule générale très vaste (et notamment des thiazolyl-3 céphalosporines), qui sont préparées par synthèse totale et qui sont actives sur germes gram-positifs et sur germes gram-négatifs.

La publication de T. Sugawara et coll., Chem. Pharm. Bull. 28 (7), 2116 (1980) fait état de thiadiazolylcéphalosporines dont l'intérêt principal est une bonne activité sur germes gram-négatifs.

Il a maintenant été trouvé qu'une classe particulière de thiazolyl-3 céphalosporines représentée ici par la formule générale (I) présente une activité antibactérienne spécifiquement sur les germes gram-positifs alors que son activité sur les germes gram-négatifs est quasiment inexistante. En outre cette nouvelle classe de thiazolyl-3 céphalosporines présente l'avantage d'une synthese facile.

Dans la formule générale (I), le symbole $R_1$ représente un radical hétérocyclyle choisi parmi thiényle, furyle ou dithiol-1,3 one-2 yle-4 ou un radical phényle, p. hydroxyphényle, phénoxy ou dichlorophénylthio et le symbole $R_2$ représente un atome d'hydrogène, ou le symbole $R_1$ représente un radical phényle ou p. hydroxyphényle et le symbole $R_2$ représente un radical amino, le symbole $R_3$ représente un atome d'hydrogène, un radical phényle (éventuellement substitué par un radical acylamino), alcoylthio, alcoylamino, dialcoylamino ou anilino, un radical acylamino, benzoylamino ou thénoylamino éventuellement substitué sur l'atome d'azote par un radical alcoyle ou phényle, un radical alcoyloxycarbonylamino, dialcoylaminoéthylamino, dialcoylaminométhylèneamino ou alcoylidènehydrazo, un radical acétamido substitué par un radical amino, amino-2 éthylthio ou L amino-2 éthylthio, ou un radical de structure $-A-R'_3$ dans lequel A représente un radical bivalent choisi parmi $-CH_2-$, $-NH-$ ou $-NHCO-$ et $R'_3$ représente un radical méthyl-1 pyridinio-3 (ou -4), benzoylméthyl-1 pyridinio-3 (ou -4) ou carboxyméthyl-1 pyridinio-3 (ou -4), le symbole R représente un radical carboxy, ou représente un radical carboxylato lorsque $R'_3$ est un radical pyridinio substitué, et le symbole X représente un atome de soufre ou d'oxygène.

Il est entendu que les radicaux et portions alcoyles et acyles cités ci-dessus sont droits ou ramifiés et contiennent 1 à 4 atomes de carbone.

Il est également entendu que, lorsque $R_2$ est un radical amino, les formes D et L des produits de formule générale (I) et leurs mélanges entrent dans le cadre de la présente invention.

A) Selon l'invention, les produits de formule générale (I) peuvent être préparés par action d'un acide de formule générale

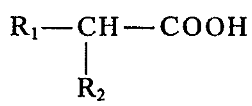

(II)

(dans laquelle $R_1$ et $R_2$ sont définis comme ci-dessus, et dont la fonction amine que peut représenter $R_2$ est préalablement protégée), ou d'un dérivé réactif de cet acide, sur un dérivé d'amino-7 céphalosporine de formule générale

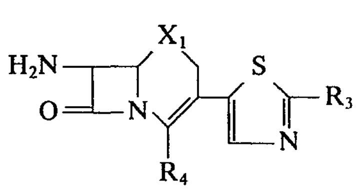

(III)

[dans laquelle $R_3$ qui est défini comme précédemment est protégé lorsqu'il contient un radical amino, $R_4$ est un radical carboxy libre ou protégé par un groupement facilement éliminable (par exemple méthoxyméthyle, t. butyle, trichloro-2,2,2 éthyle, benzhydryle, p. nitrobenzyle ou p. méthoxybenzyle) ou un radical carboxylato, et $X_1$ est défini comme X précédemment, ou représente un groupe sulfinyle], puis on réduit le cas échéant le sulfoxyde et élimine éventuellement les radicaux protecteurs.

La fonction amine représentée par $R_2$ (et le cas échéant la fonction amine contenue par $R_3$) peuvent

être protégées par tout groupement facilement éliminable utilisé en chimie peptidique, dont la mise en place et l'élimination n'affectent pas le reste de la molécule.

On peut utiliser notamment des groupements tels que t. butoxycarbonyle, vinyloxycarbonyle, trichloro-2,2,2 éthoxycarbonyle, chloracétyle, trichloracétyle, trifluoracétyle, trityle, benzyle, benzyloxycarbonyle, p. nitrobenzyloxycarbonyle, p. méthoxybenzyloxycarbonyle ou formyle, ou encore un radical diphénylphosphinoyle ou un radical tel que défini ci-après par la formule générale (XVI) qui peuvent être introduits par application de la méthode décrite par A. MORIMOTO et coll., J. Chem. Soc. Perkin I, 1109 (1980).

Lorsque $R_1$ contient un radical hydroxy, ce groupement peut être libre ou protégé. La protection s'effectue par exemple par des radicaux trityle, tétrahydropyrannyle, alcoyloxycarbonyle (par exemple t. butoxycarbonyle), aryloxycarbonyle (par exemple benzyloxycarbonyle) ou p. méthoxybenzyle.

Lorsque l'on utilise le produit de formule générale (II) sous sa forme acide, on effectue la condensation sur l'amino-7 céphalosporine de formule générale (III) dans laquelle $R_4$ est un radical carboxy protégé, ou un radical carboxylato, en présence d'un agent de condensation tel qu'un carbodiimide (par exemple dicyclohexylcarbodiimide), le NN'-carbonyldiimidazole ou l'éthoxy-2 éthoxycarbonyl-1 dihydro-1,2 quinoléine, à une température comprise entre −20 et 40° C, puis on élimine le cas échéant les groupements protecteurs.

Lorsque l'on veut utiliser un dérivé réactif de l'acide de formule générale (II), il est possible de mettre en oeuvre l'anhydride, un anhydride mixte, ou un ester réactif de formule générale

$$R_1—\underset{\underset{R_2}{|}}{CH}—COOZ \qquad (IV)$$

dans laquelle, $R_1$ et $R_2$ étant définis comme précédemment, Z représente un radical succinimido, benzotriazolyle-1, nitro-4 phényle, dinitro-2,4 phényle, pentachlorophényle ou phtalimido, étant entendu que lorsque $R_2$ est un radical amino, la fonction amine de tous ces dérivés réactifs est préalablement protégée.

Il est également avantageux d'utiliser un halogénure d'acide. Dans ce dernier cas, lorsque $R_2$ est amino, on peut faire réagir le chlorhydrate du chlorure d'acide ce qui évite, dans ce pas particulier, la protection préalable du radical amino.

Lorsque l'on met en oeuvre l'anhydride, un anhydride mixte ou un halogénure d'acide (qui peuvent être préparés in situ), on effectue la condensation dans un solvant organique inerte tel qu'un éther (par exemple tétrahydrofuranne ou dioxanne), un solvant chloré (par exemple chloroforme ou chlorure de méthylène), un amide (par exemple diméthylformamide ou diméthylacétamide) ou une cétone (par exemple acétone) ou dans des mélanges des solvants ci-dessus, en présence d'un accepteur d'acide tel qu'un époxyde (par exemple l'oxyde de propylène) ou tel qu'une base organique azotée comme la pyridine, la diméthylaminopyridine, la N-méthylmorpholine ou une trialcoylamine (par exemple triéthylamine) ou en présence d'un agent de silylation tel que le bistriméthylsilylacétamide, ou bien dans un milieu hydroorganique en présence d'un agent alcalin de condensation tel que le bicarbonate de sodium, et l'on opère à une température comprise entre −40 et 40°C, puis on remplace les groupements protecteurs par des atomes d'hydrogène.

Lorsque l'on met en oeuvre un ester réactif de formule générale (IV), on opère généralement en présence d'une amine tertiaire (par exemple triéthylamine) dans un solvant organique tel que le diméthylformamide à une température comprise entre 0 et 40°C, puis on remplace les groupements protecteurs par des atomes d'hydrogène.

Le cas échéant, la réduction du sulfoxyde s'effectue dans les conditions décrites dans la demande de brevet allemand 2 637 176.

L'élimination des groupements protecteurs d'acide peut s'effectuer par exemple:

- lorsqu'il s'agit d'un groupement t. butyle, p. méthoxybenzyle ou benzhydryle: par traitement en milieu acide, dans les conditions décrites ci-après pour l'élimination du radical trityle protecteur d'amino. Dans le cas du radical benzhydryle, on peut opérer en présence d'anisole ou par traitement par le chlorure d'aluminium dans les conditions décrites par T. Tsuji et coll., Tet. Lett., 30, 2793 (1979);
- lorsqu'il s'agit d'un groupement méthoxyméthyle: par traitement en milieu acide dilué;
- lorsqu'il s'agit d'un groupement trichloro-2,2,2 éthyle ou p. nitrobenzyle: par réduction (notamment traitement par le zinc dans l'acide acétique, ou lorsqu'il s'agit du groupement p. nitrobenzyle par hydrogénolyse).

L'élimination des groupements protecteurs d'amine peut s'effectuer par exemple:

- lorsqu'il s'agit d'un radical t. butoxycarbonyle, vinyloxycarbonyle, trityle, p. méthoxybenzyloxy-carbonyle ou formyle: par traitement en milieu acide. De préférence on utilise l'acide trifluoracétique en opérant à une température comprise entre 0 et 20°C, ou bien on utilise l'acide

formique, phosphorique ou polyphosphorique anhydres ou aqueux à une température comprise entre 20 et 60°C, ou encore l'acide paratoluènesulfonique ou méthanesulfonique dans l'acétone ou l'acétonitrile, à une température comprise entre 20°C et la température de reflux du mélange réactionnel. Dans ces conditions le produit de formule générale (I) peut être obtenue sous forme de trifluoracétate, de solvate avec l'acide formique, de phosphate, de méthanesulfonate ou de paratoluènesulfonate, dont on peut libérer la fonction amine par toute méthode connue en soi pour obtenir une amine à partir de l'un de ses sels sans toucher au reste de la molécule. On opère notamment par mise en contact avec une résine échangeuse d'ions ou par action d'une base organique;

- lorsqu'il s'agit d'un radical trichloro-2,2,2 éthoxycarbonyle ou p. nitrobenzyloxycarbonyle ou d'un radical de formule générale (XVI) dans laquelle R' est trichloro-2,2,2 éthyle ou nitrobenzyle: par réduction (notamment traitement par le zinc dans l'acide acétique);
- lorsqu'il s'agit d'un radical chloracétyle ou trichloracétyle: par application de la méthode décrite dans le brevet français publié sous le n° 2 243 199;
- lorsqu'il s'agit d'un radical benzyle ou benzyloxycarbonyle: par hydrogénation catalytique;
- lorsqu'il s'agit d'un radical trifluoracétyle: par traitement en milieu basique;
- lorsqu'il s'agit d'un radical diphénylphosphinoyle: selon la méthode décrite par P. Haake et coll., J. Am. Chem. Soc., 95, 8073 (1973);
- lorsqu'il s'agit d'un radical de formule générale (XVI): selon la méthode décrite dans le brevet belge 833 619.

L'élimination des groupements protecteurs du radical hydroxy s'effectue le cas échéant:

- lorsqu'il s'agit de groupements trityle, tétrahydropyrannyle ou méthoxybenzyle: par acidolyse, par exemple par l'acide trifluoracétique, l'acide formique aqueux ou non, ou l'acide paratoluènesulfonique;
- lorsqu'il s'agit des groupements alcoyloxycarbonyle ou aryloxycarbonyle: selon les méthodes décrites dans le brevet belge 871 213.

B) Selon l'invention, les dérivés de céphalosporine de formule générale (I) dans laquelle $R_1$, $R_2$ et X sont définis comme précédemment, $R_3$ est défini comme précédemment à l'exception de représenter un radical $-A-R'_3$ et R est un radical carboxy, peuvent être également préparés par action d'un produit de formule générale:

$$R_3CSNH_2 \qquad (V)$$

dans laquelle $R_3$ est défini comme précédemment (étant entendu que lorsqu'il contient un radical amino, ce dernier est protégé), sur un dérivé de céphalosporine de formule générale

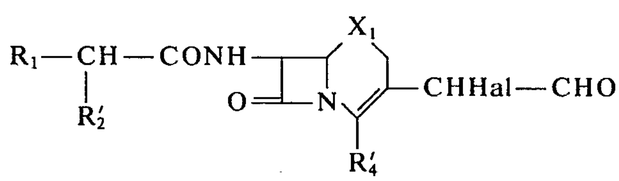

(VI)

(dans laquelle $R_1$ et $X_1$ sont définis comme précédemment, $R'_2$ représente un atome d'hydrogène ou un radical amino protégé, $R'_4$ représente un radical carboxy protégé tel que défini pour $R_4$ et Hal représente un atome d'halogène), puis éventuellement action d'un agent de déshydratation, puis le cas échéant du sulfoxyde et élimination des proupements protecteurs.

Lorsque $R'_2$ représente un radical amino protégé, il peut être protégé par exemple par un radical t. butoxycarbonyle, trichloro-2,2,2 éthoxycarbonyle, trityle, benzyle, benzyloxycarbonyle, formyle, trifluoracétyle, p. nitrobenzyloxycarbonyle, p. méthoxybenzyloxycarbonyle, diphénylphosphinoyle ou un radical de formule générale (XVI) tel que défini ci-après.

Le symbole Hal peut représenter un atome de chlore, de brome ou d'iode.

Lorsque $R_1$ contient un radical hydroxy, ce dernier peut être libre ou protégé. La protection et le déblocage s'effectuent dans les conditions décrites précédemment.

On opère généralement en milieu organique ou hydroorganique, par exemple dans des solvants (ou des mélanges de solvants) tels que des alcools (méthanol, éthanol), des éthers (tétrahydrofuranne, dioxanne), des cétones (acétone), des nitriles (acétonitrile), des amides secondaires (diméthylformamide, diméthylacétamide), des esters (acétate d'éthyle) ou des acides (acide acétique, acide formique), en présence ou non d'une base (soude potasse, carbonates, carbonates acides de métaux alcalins, sels d'acides carboxyliques et de métaux alcalins, amines tertiaires), à une température comprise entre −50°C et la température de reflux du mélange réactionnel.

Il est parfois préférable d'introduire un agent de déshydratation. C'est le cas notamment lorsque $R_3$

représente un radical phényle ou phényle substitué.

Parmi les agents de déshydratation utilisables, on peut citer: les halogénures d'acides sulfoniques (par exemple chlorure de tosyle, chlorure de méthanesulfonyle ou un halogénure du type $RSO_2Cl$ dans lequel R est alcoyle, trifluoro (ou trichloro) méthyle ou phényle éventuellement substitué par halogène, méthyle ou nitro), les halogénures de phosphoryle (par exemple oxychlorure de phosphore) ou le chlorure de sulfonyle, dans un solvant basique [pyridine, amide (par exemple diméthylformamide, diméthylacétamide, hexaméthylphosphorotriamide)] ou dans un solvant chloré (par exemple chloroforme, chlorure de méthylène), un éther (par exemple tétrahydrofuranne), un ester, une cétone, un nitrile, un solvant aromatique, en présence d'une amine tertiaire (par exemple pyridine, quinoléine, triéthylamine).

C) Selon l'invention, les produits de formule générale (I) pour lesquels $R_3$ représente un radical acylamino, benzoylamino ou thénoylamino éventuellement substitués sur l'atome d'azote, ou représente un radical alcoyloxycarbonylamino, dialcoylaminométhylèneamino, un radical acétamido substitué ou un radical de structure $-A-R'_3$ dans laquelle A est $-NHCO-$ et $R'_3$ est défini comme précédemment, peuvent être obtenus à partir de l'amine correspondante de formule générale

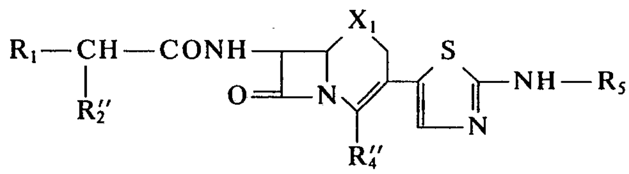

(VII)

dans laquelle $R_1$ et $X_1$ sont définis comme précédemment, $R''_2$ représente un radical amino protégé, $R''_4$ est un radical carboxy ou un radical carboxy protégé tel que défini pour $R'_4$ et $R_5$ est un atome d'hydrogène ou un radical alcoyle ou phényle, par toute méthode connue en soi pour former une fonction amide, carbamate ou amidine sans toucher au reste de la molécule, puis réduction le cas échéant du sulfoxyde obtenu et éventuellement élimination des radicaux protecteurs.

Le radical $R''_2$ protecteur d'amino peut être choisi parmi les groupements cités précédemment pour le procédé A. Il peut être éliminé après la réaction selon les méthodes citées précédemment en A/.

Lorsque $R_1$ contient un radical hydroxy, ce dernier peut être libre ou, de préférence, protégé. La protection et l'élimination des radicaux protecteurs s'effectuent comme décrit précédemment.

Lorsque l'on veut préparer un produit de formule générale (I) dans laquelle $R_3$ représente un radical acylamino, benzoylamino ou thénoylamino (substitués ou non), alcoyloxycarbonylamino, acétamido substitué ou un radical de structure $-A-R'_3$ dans laquelle A est $-NHCO-$, la réaction s'effectue avantageusement par action du chlorure d'acide, de l'anhydride ou du chloroformiate correspondant dans les conditions décrites précédemment pour la réaction du chlorure de l'acide de formule générale (II) sur le dérivé d'amino-7 céphalosporine de formule générale (III). Il est entendu que les restes d'amines primaires contenues le cas échéant dans le radical $R_3$, sont préalablement protégés.

Lorsque l'on veut préparer un produit de formule générale (I) dans laquelle $R_3$ est dialcoylaminométhylèneamino, la réaction s'effectue avantageusement par action de l'acétal du dialcoylformamide correspondant sur le dérivé de céphalosporine de formule générale (VII) dans laquelle $R''_4$ représente un radical carboxy protégé et $R_5$ représente un atome d'hydrogène.

D) Selon l'invention, les produits de formule générale (I) pour lesquels $R_1$, $R_2$ et X étant définis comme précédemment, $R_3$ est un radical de structure $-AR'_3$ et R est un radical carboxylato, peuvent être obtenus par action d'un halogénure de méthyle, de benzoylméthyle ou de carboxyméthyle (dont la fonction acide est libre ou protégée) sur un dérivé de céphalosporine de formule générale

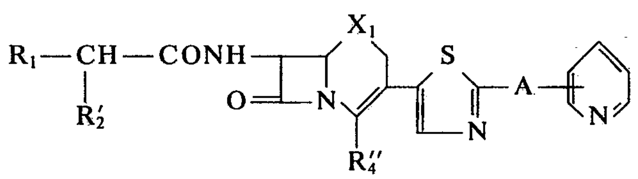

(VIII)

dans laquelle le radical A est substitué en position -3 ou -4 du noyau pyridyle, $R_1$, $R''_4$, $X_1$ et A sont définis comme précédemment et $R'_2$ est défini comme pour la formule générale (VII), puis le cas échéant réduit le sulfoxyde obtenu et élimine les radicaux protecteurs.

La réaction s'effectue généralement dans un solvant organique tel qu'un amide (par exemple diméthylformamide, hexaméthylphosphorotriamide ou diméthylacétamide), un nitrile (acétonitrile par exemple), une cétone (acétone par exemple) ou un dérivé nitré (nitrométhane, nitrobenzène par exemple) ou dans un mélange de tels solvants, à une température comprise entre 0 et 80°C.

La libération des radicaux protégés s'effectue dans les conditions décrites précédemment.

E) Selon l'invention, les produits de formule générale (I) pour lesquels $R_1$, $R_2$ et X étant définis comme précédemment, $R_3$ représente un radical acétamido substitué par un radical amino-2 éthylthio ou L amino-2 carboxy-2 éthylthio et R représente un radical carboxy, peuvent également être obtenus par action

de cystéamine ou de cystéine dont la fonction amine et le cas échéant acide sont préalablement protégées, sur une halogénoacétamidothiazolylcéphalosporine de formule générale

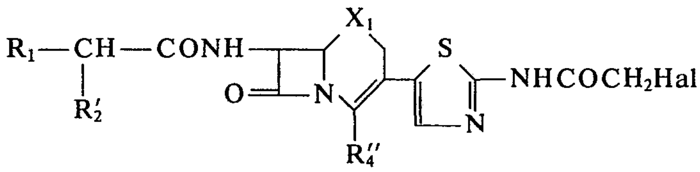

(IX)

[dans laquelle $R_1$, $R_4''$ et $X_1$ sont définis comme précédemment, $R_2'$ est défini comme pour la formule générale (VII) et Hal représente un atome de chlore, de brome ou d'iode], puis réduit le cas échéant le sulfoxyde obtenu et élimine les radicaux protecteurs.

La réaction s'effectue généralement dans un solvant organique par exemple un amide (diméthylformamide), un nitrile (acétonitrile) ou un mélange de tels solvants, à une température comprise entre −20°C et la température de reflux du mélange réactionnel. De préférence on opère en présence d'une base organique tertiaire par exemple la diisopropyléthylamine, la diéthylphénylamine ou la triéthylamine.

Il n'est pas absolument nécessaire d'avoir isolé le produit intermédiaire avant de procéder à la réduction.

L'élimination des radicaux protecteurs s'effectue dans les conditions décrites précédemment.

Les dérivés d'amino-7 céphalosporines de formule générale (III) peuvent être obtenus à partir d'un produit de formule générale

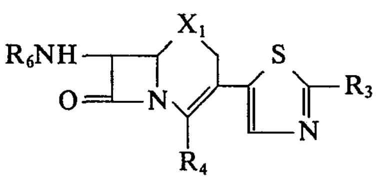

(X)

[dans laquelle $R_3$, $R_4$ et $X_1$ sont définis comme précédemment, étant entendu que lorsque $R_3$ contient un radical amino, ce dernier est protégé, et $R_6$ représente un radical facilement éliminable], par élimination du radical $R_6$, ou le cas échéant élimination successive ou simultanée du radical $R_6$ et du radical protecteur contenu par $R_4$ lorsque l'on veut obtenir un produit de formule générale (III) dont la fonction acide est libre à partir d'un produit de formule générale (X) dont la fonction acide est protégée.

Par radical $R_6$ facilement éliminable, on entend par exemple:

1. benzhydryle ou trityle,
2. un radical acyle de formule générale:

$$R_7CO— \qquad (XI)$$

dans laquelle $R_7$ représente

a) un atome d'hydrogène ou un radical alcoyle contenant 1 à 7 atomes de carbone, méthyle substitué par 1 à 3 atomes d'halogène,
b) un radical phényle (pouvant être jusqu'à 3 fois substitué par des atomes d'halogène ou des radicaux hydroxy, nitro, cyano, trifluorométhyle, alcoyle ou alcoyloxy) ou un radical thiényle-2 ou -3,
c) un radical de formule générale:

$$R_7'—Y—CH_2— \qquad (XIa)$$

dans laquelle $R_7'$ est un radical phényle pouvant être substitué par un atome d'halogène ou par un radical alcoyle, alcoyloxy ou hydroxy, et Y est un atome d'oxygène ou de soufre,
d) un radical arylalcoyle de formule générale:

$$R_7''CH_2— \qquad (XIb)$$

dans laquelle $R_7''$ est un radical phényle pouvant être jusqu'à 3 fois substitué par des radicaux hydroxy, alcoyle ou alcoyloxy ou un radical hétérocyclyle tel que thiényle-2 ou -3 ou furyle-2 ou -3,

3. un radical amino-5 adipoyle dont les fonctions amine et acide sont protégées par des radicaux

protecteurs tels que définis précédemment,

4. un radical de formule générale:

$R_8OCO—$ (XII)

dans laquelle $R_8$ est un radical alcoyle ramifié non substitué, un radical alcoyle droit ou ramifié portant un ou plusieurs substituants [tels que des atomes d'halogène ou des radicaux cyano, trialcoylsilyle, phényle ou phényle substitué (par un ou plusieurs atomes d'halogène ou radicaux alcoyle, alcoyloxy, nitro ou phényle)] ou un radical quinolyle,

5. un radical de formule générale:

$Ar—S(O)_n—$ (XIII) ou $ArSe—$ (XIV)

dans lesquelles le reste Ar est un radical phényle éventuellement substitué par un ou plusieurs atomes d'halogène ou radicaux nitro ou alcoyle et n est égal à 0 ou 1,

6. ou bien $R_6NH—$ peut être remplacé par un radical dialcoylaminométhylèneamino ou par un radical de formule générale:

$Ar'—CH{=}N—$ (XV)

dans laquelle Ar' est un radical phényle éventuellement substitué par un ou plusieurs radicaux tels que alcoyle, alcoyloxy, hydroxy ou nitro,

7. ou encore $R_6$ est un radical diphénylphosphinoyle ou un radical de formule générale:

$(R'O)_2P(\rightarrow O)—$ (XVI)

dans laquelle R' est alcoyle, trichloro-2,2,2 éthyle, phényle ou benzyle (ces deux derniers éventuellement substitués par un atome d'halogène ou par un radical alcoyle, alcoyloxy ou nitro) ou les radicaux R' forment ensemble un radical alcoylène contenant 2 ou 3 atomes de carbone.

Comme exemples de radicaux $R_6$ pouvant être utilisés, on peut citer les radicaux suivants:

formyle, acétyle, trichloracétyle, phénylacétyle,
phénoxyacétyle, benzoyle,
t.butoxycarbonyle
chloro-2 diméthyl-1,1 éthoxycarbonyle
trichloro-2,2,2 éthoxycarbonyle
trichloro-2,2,2 diméthyl-1,1 éthoxycarbonyle cyano-2 diméthyl-1,1, éthoxycarbonyle
triméthylsilyl-2 éthoxycarbonyle
benzyloxycarbonyle
p.méthoxybenzyloxycarbonyle
diméthoxy-3,5 benzyloxycarbonyle
p. nitrobenzyloxycarbonyle
diphénylméthoxycarbonyle
(biphénylyl-4)-2 isopropyloxycarbonyle
quinolyl-8 oxycarbonyle
o.nitrophénylthio
p.nitrophénylthio
diméthoxyphosphoryle
diéthoxyphosphoryle
diphénoxyphosphoryle
dibenzyloxyphosphoryle

Comme exemples de radicaux méthylèneamino définis précédemment en 6), ou peut citer:

diméthylaminométhylèneamino
diméthoxy-3,4 benzylidèneamino
nitro-4 benzylidèneamino

Il est entendu que le radical $R_6$ et le cas échéant le radical protecteur d'amino contenu par $R_3$ sont différents et choisis de telle manière qu'ils soient éliminables sélectivement.

L'élimination du radical protecteur peut s'effectuer par toute méthode connue pour libérer une fonction amine sans toucher au reste de la molécule.

On opère notamment dans les conditions décrites dans le brevet belge 883 415 ou rappelées ci-avant en A/.

Les dérivés de céphalosporine de formule générale (X) peuvent être obtenus selon la signification du symbole $R_3$ par analogie avex l'un des procédés B, C et D utilisés pour la préparation des produits selon l'invention, à savoir:

— soit par action d'un produit de formule générale (V) sur un dérivé de céphalosphorine de formule générale:

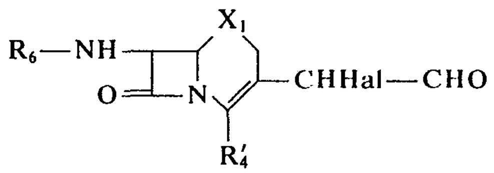

(VIa)

dans laquelle $R'_4$, $R_6$, $X_1$ et Hal sont définis comme précédemment, suivie éventuellement de l'addition d'un agent de déshydratation et éventuellement de la libération de la fonction acide lorsque l'on veut obenir un produit de formule générale (X) dans lequel $R_4$ est un radical carboxy:

— soit à partir d'un produit de formule générale:

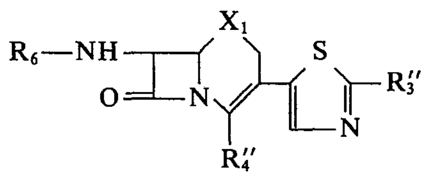

(XVII)

[dans laquelle $R''_4$, $R_6$ et $X_1$ sont définis comme précédemment, et $R''_3$ représente soit un radical de structure $-NHR_5$ dans laquelle $R_5$ est défini comme précédemment pour la formule générale (VII) soit un radical de structure

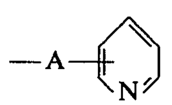

défini comme précédemment dans la formule générale (VIII)] en opérant, selon le cas, dans les conditions décrites dans le procédé C ou D.

Les conditions de mise en oeuvre de ces procédés sont identiques à celles utilisées pour la préparation des produits de formule générale (I).

Les produits de formule générale (V) et (Va) décrits ci-après, peuvent être préparés par action d'ammoniac sur l'isothiocyanate ou le dithiocarbamate correspondant, ou par action de sulfure d'hydrogène sur le nitrile correspondant, ou plus précisément selon les méthodes citées ci-après:

— lorsque $R_3$ représente un atome d'hydrogène: selon la méthode décrite par A. W. HOFMANN, Chem. Ber., 11, 340 (1878),
— lorsque $R_3$ est un radical acylaminophényle: par application de la méthode de L. Stephensen et coll., J. Chem. Soc. (C), 861 (1969),
— lorsque $R_3$ représente un radical alcoylthio: par action du dithiocarbamate d'ammonium sur l'halogénure d'alcoyle convenable,
— lorsque $R_3$ représente dialcoylamino: par application de la méthode de MAMELI, Ann. Chimica, 46, 545 (1956),
— lorsque $R_3$ représente acylamino: selon la méthode décrite par M. L. MOORE et F. S. CROSSLEY, J. Am. Chem. Soc. 62, 3274 (1940),
— lorsque $R_3$ ou $R''_3$ représente benzoylamino, thénoylamino, nicotinoylamino ou isonicotinoylamino: selon la méthode de W. H. PIKE, Chem. Ber., 6, 755 (1873),
— lorsque $R_3$ représente acylamino, benzoylamino ou thénoylamino substitués sur l'atome d'azote par un radical alcoyle: par analogie avec la méthode décrite par KURZER, J. Chem. Soc., 1957, 4461 à partir de N-cyanoacétamides, de N-cyanobenzamides ou de N-cyanothénoylamides obtenus respectivement par application de la méthode de R. HUFFMANN et F. C. SCHAEFER, J. Org. Chem., 28, 1816 (1963) ou de K. HARTKE et E. PALOU, Chem. Ber., 99 (10) 3155 (1966),
— lorsque $R_3$ est un radical acylamino, benzoylamino ou thénoylamino substitués sur l'atome d'azote

par un radical phényle: selon méthode décrite par P. K. SRIVASTAVA, Ind. J. Chem., 7 (4), 323 (1969),

- lorsque $R_3$ est un radical alcoyloxycarbonylamino: par application de la méthode décrite par R. E. DORAN, J. Chem. Soc., 69, 331 (1896),
- lorsque $R_3$ est un radical dialcoylaminoéthylamino: selon la méthode décrite dans la demande de brevet allemand 2 738 711,
- lorsque $R_3$ est un radical dialcoylaminométhylèneamino: par application de la méthode décrite par H. BREDERECK et coll., Ber., 97, 61 (1964),
- lorsque $R_3$ est alcoylidènehydrazo: par action de la thiosemicarbazide sur la cétone ou l'aldéhyde convenable.

Les dérivés de céphalosporine de formule générale (VI) et (VIa) dans lesquelles $X_1$ est autre que le radical sulfinyle peuvent être préparés par action d'un agent d'halogénation sur une énamine de formule générale:

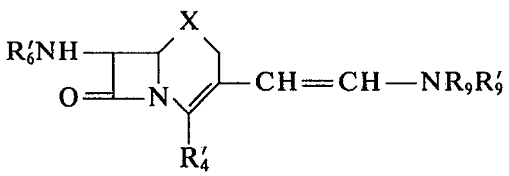

(XVIII)

[dans laquelle $R'_4$ et X sont définis comme précédemment, $R'_6$ représente un radical $R_6$ ou un radical $R_1CHR'_2$—CO— (dans lequel $R_1$ et $R'_2$ sont définis comme précédemment) et $R_9$ et $R'_9$, identiques ou différents, représentent des radicaux alcoyle (éventuellement substitués par un radical alcoyloxy ou dialcoylamino) ou phényle, ou forment ensemble avec l'atome d'azote auquel ils sont attachés, un hétérocyle à 5 ou 6 chaînons contenant éventuellement un autre hétéroatome choisi parmi l'azote, l'oxygène ou le soufre et éventuellement substitué par un radical alcoyle], suivie de l'hydrolyse du produit formé.

Par exemple on met en oeuvre une énamine de formule générale (XVIII) dans laquelle $R_9$ et $R'_9$ représentent chacun un radical méthyle.

Parmi les agents d'halogénation peuvent être cités: les halogènes, les N-haloamides [par exemple N-bromo (ou N-chloro) succinimide, N-bromo (ou N-chloro) acétamide, dibromohydantoïne], les hypohalogénites d'alcoyle (par exemple l'hypochlorite de t.butyle, l'hypobromite de t.butyle ou l'hypochlorite d'éthyle).

On opère généralement dans un solvant organique tel qu'un éther (par exemple tétrahydrofuranne, dioxanne), un solvant chloré (par exemple chlorure de méthylène, chloroforme), un ester (par exemple acétate d'éthyle), un alcool (par exemple méthanol, éthanol), un amide (par exemple diméthylformamide, diméthylacétamide), un nitrile (par exemple acétonitrile) ou une cétone (par exemple acétone), ou dans un mélange de ces solvants, à une température comprise entre −70 et 0°C.

L'hydrolyse s'effectue à une température comprise entre −70 et 20°C.

Il n'est pas indispensable d'isoler ni de purifier les produits de formules générales (VI) ou (VIa) pour les mettre en oeuvre selon l'invention.

Les dérivés de la céphalosphorine de formules générales (VI) et (VIa) dans lesquelles $X_1$ est un groupe sulfinyle, peuvent être obtenus par oxydation d'un produit de formule générale (VI) ou (VIa) dans laquelle $X_1$ est un atome de soufre, par application des méthodes décrites dans la demande de brevet allemand 2 637 176.

Les énamines de formule générale (XVIII) peuvent être préparées par application de la méthode décrite dans le brevet belge 883 416 à partir de dérivés de céphalosporines de formule générale:

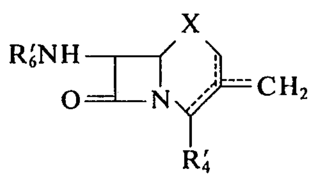

(XIX)

dans laquelle $R'_6$, $R'_4$ et X sont définis comme précédemment.

Les dérivés de céphalosporine de formule générale (XIX) dans laquelle X est un atome de soufre peuvent être obtenus comme décrit dans le brevet belge 883 416 ou, lorsque $R'_6$ est un radical facilement éliminable tel que défini précédemment pour $R_6$ en 7), par application de la méthode décrite par A. MORIMOTO et coll., J. C. S. Perkin I, 1109 (1980), à partir de l'halogénure correspondant $R'_6$-Hal [qui peut être lui-même obtenu selon l'une des méthodes décrites par K. SASSE, Methoden den Organischen Chemie, Vol. 12, part. 2, page 274, Houben Weyl, Georg Thieme Verlag, Stuttgart (1964)].

Les oxacéphalosporines de formule générale (XIX) peuvent être obtenues selon les méthodes décrites dans la littérature, par exemple:

- dans les brevets belges 863 998 et 848 288,
- dans le brevet américain 4 108 992 ou
- par Y. HAMASHIMA et coll., Tet. Let 4943 (1979), et
- par C. L. BRANCH et coll., J. C.S. Perkin I, 2268 (1979)
- ou à partir de l'oxacéphalosphorine de formule

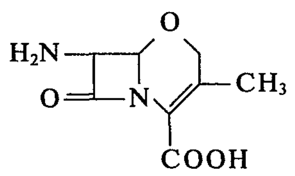

(XX)

par analogie avec les méthodes employées en chimie des céphalosporines, es décrites par exemple par
S. SEKI et coll., Tet. Lett., 33, 2915 (1977),
R. R. CHAUVETTE et coll., J. Org. Chem., 38 (17), 2994 (1973),
J. C. SHEEHAN et coll., J. Amer. Chem. Soc. 80, 1156 (1958),
E. H. FLYNN, Cephalosporins and Penicillins, Ac. Press (1972),
L. MORODER et coll., Hoppe Seyler's Z. Physiol. Chem. 357, 1651 (1976),
J. UGI et coll., Angew. Chem. Int. Ed. Engl. 17 (5), 361 (1978),
L. ZERVAS et coll., J. Amer. Chem. Soc. 85, 3660 (1963),
J. F. FITT, J. Org. Chem., 42 (15), 2639 (1977),
A. MORIMOTO et coll., J. Chem. Soc. Perkin I, 1109 (1980),
dans le brevet belge 788 885 ou dans Helv. Chim. Acta. 51, 924 (1968),
- lorsque $R'_6$ représente diphénylméthoxycarbonyle: par action de l'azidoformiate correspondant en milieu hydroorganique, en présence d'un bicarbonate alcalin,
- lorsque $R'_6$ représente quinolyl-8 oxycarbonyle: par action du carbonate correspondant en milieu hydroorganique basique,
- lorsque $R'_6NH-$ est remplacé par nitro-4 benzylidèneamino ou diméthoxy-3,4 benzylidèneamino: selon la méthode décrite par R. A. FIRESTONE, Tetrahedron Lett., 375 (1972).

Les dérivés de la céphalosporine de formules générales (VII), (VIII) et (IX) peuvent être préparés par application du procédé A décrit précédemment pour la préparation des produits selon l'invention, c'est-à-dire par acylation d'une amino-7 céphalosporine de formule générale

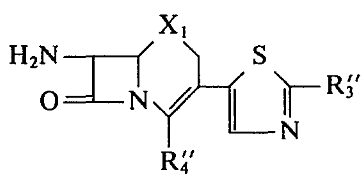

(IIIa)

dans laquelle $R''_4$ et $X_1$ sont définis comme précédemment et $R''_3$ représente un radical de structure $-NHR_5$ dans lequel $R_5$ est défini comme précédemment, un radical de structure

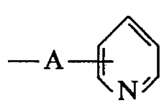

défini comme précédemment pour la formule générale (VIII), ou un radical halogénoacétamido tel que défini pour la formule générale (IX), puis éventuellement élimination des radicaux protecteurs.

Il est entendu que lorsque $R_5$ représente un atome d'hydrogène, le radical $-NHR_5$ est de préférence protégé. La protection s'effectue par un groupement tel que défini précédemment pour $R'_2$ dans les formules générales (VII), (VIII) et (IX).

L'acylation s'effectue dans les conditions décrites précédemment pour la préparation des produits selon l'invention par le procédé A.

L'élimination de radicaux protecteurs peut être effectuée dans les conditions décrites précédemment.

L'amino-7 céphalosporine de formule générale (IIIa) peut être obtenue à partir d'une céphalosporine de formule générale (XVII) par application des méthodes décrites pour la préparation de l'amino-7 céphalosporine de formule générale (III).

Il est entendu que lorsque $-NHR_5$ représente un radical amino ce dernier est de préférence protégé; la protection est effectuée par un groupement différent de $R_6$, de telle manière que le radical protecteur $R_6$ soit éliminable sélectivement.

La céphalosporine de formule générale (XVII) dans laquelle $R''_4$, $R_6$ et $X_1$ sont définis comme précé-

demment, et $R'_3$ représente un radical de structure $-NHR_5$ ($R_5$ étant défini comme précédemment), ou de structure

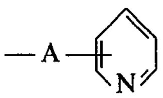

défini comme précédemment pour la formule général (VIII) peut être obtenue à partir d'un dérivé de céphalosporine de formule générale (VIa) par analogie avec la méthode décrite pour la préparation des produits selon l'invention par le procédé B.

La réaction s'effectue par action d'un produit de formule générale

$R''_3-CS-NH_2$ (Va)

dans laquelle $R''_3$ est défini comme ci-dessus, en opérant dans les conditions décrites précédemment pour le procédé B. Eventuellement on élimine le groupement protecteur contenu par $R'_4$.

La céphalosporine de formule générale (XVII) dans laquelle $R'_3$ est un radical halogénoacétamido peut être obtenue à partir d'un dérivé de céphalosphorine de formule générale (XVII) dans laquelle $R'_3$ est un radical $-NHR_5$, $R_5$ étant un atome d'hydrogène, par action d'un acide halogénoacétique ou d'un de ses dérivés, en opérant dans les conditions d'acylation décrites dans le procédé A.

Les dérivés de la céphalosporine de formules générales (VII) et (VIII) peuvent également être préparés à partir d'une céphalosporine de formule générale (VI), par application du procédé B dans les conditions décrites ci-dessus.

Les dérivés de la céphalosporine de formule générale (IX) peuvent également être obtenus à partir du dérivé correspondant de formule générale (VII) dans laquelle $R_5$ est un atome d'hydrogène, par analogie avec la préparation des produits de formule générale (XVII) décrite ci-dessus.

Les dérivés de la céphalosporine de formule générale (III), (IIIa), (VII), (VIII), (IX), (X) ou (XVII) dans lesquelles $X_1$ est un radical sulfinyle, peuvent être obtenus par oxydation des dérivés correspondants dans lesquels $X_1$ est un atome de soufre, dans les conditions décrites dans la demande de brevet allemand 2 637 176.

Les produits selon la présente invention peuvent être transformés en sels métalliques ou en sels d'addition avec les bases azotées selon les méthodes connues en soi. Ces sels peuvent être obtenus par action d'une base métallique (par exemple alcaline ou alcalino-terreuse), de l'ammoniac ou d'une amine sur un produit selon l'invention dans un solvant approprié tel qu'un alcool, un éther ou l'eau ou par réaction d'échange avec un sel d'un acide organique. Le sel formé précipite après concentration éventuelle de sa solution, il est séparé par filtration ou décantation. Il peut également être isolé de sa solution par évaporation du solvant, notamment par lyophilisation.

Les produits selon l'invention pour lesquels $R_2$ est un radical amino ou $R_3$ contient un substituant amino peuvent également être transformés en sels d'addition avec les acides; les produits selon l'invention pour lesquels $R_3$ est un radical $-AR'_3$ peuvent être transformés en sels d'addition avec les acides forts. Ces sels d'addition peuvent être obtenus par action produit sur des acides dans des solvants appropriés. Comme solvants organiques on utilise par exemple des alcools, des cétones, des éthers ou des solvants chlorés.

Comme exemples de sels pharmaceutiquement acceptables peuvent être cités les sels avec les métaux alcalins (sodium, potassium, lithium) ou avec les métaux alcalino-terreux (magnésium, calcium), les sels d'ammonium, les sels de bases azotées (éthanolamine, diéthanolamine, triméthylamine, triéthylamine, méthylamine, propylamine, diisopropylamine, NN-diméthyléthanolamine, benzylamine, dicyclohexylamine, N-benzyl-$\beta$-phénéthylamine, NN'-dibenzyléthylènediamine, N-méthylglucamine, diphénylènediamine, benzhydrylamine, quinine, choline, arginine, lysine, leucine, dibenzylamine) ou les sels d'addition avec des acides minéraux (chlorhydrates, bromhydrates, sulfates, nitrates, phosphates) ou organiques (succinates, fumarates, maléates, p.toluènesulfonates).

Les nouveaux produits selon la présente invention peuvent être éventuellement purifiés par des méthodes physiques telles que la cristallisation, l'ultrafiltration ou la chromatographie.

La famille de céphalosporine selon la présente invention, et leurs sels pharmaceutiquement acceptables, sont des agents antibactériens à spectre étroit particulièrement intéressants, qui manifestent une activité remarquable in vitro et in vivo sur les germes grampositifs, particulièrement sur staphylocoques et surtout sur streptocoques D.

La mise en évidence d'une famille de produits à spectre étroit est particulièrement remarquable puisque l'on connaît l'intérêt des spécialistes pour de tels produits.

In vitro, les produits de formule générale (I) se sont montrés actifs à une concentration comprise entre 0,03 et 8 $\mu g/cm^3$ sur des souches de staphylocoques sensibles à la pénicilline G (Staphylococcus aureus Smith), à une concentration comprise entre 0,00006 et 0,03 $\mu g/cm^3$ sur Streptococcus pyogenes Dig 7 et Streptococcus pneumoniae TIL et surtout à des concentrations comprises entre 4 et 60 $\mu g/cm^3$ sur Streptococcus faecium ATCC 9790.

In vivo les produits de formule générale (I) se sont montrés actifs sur les infections expérimentales de

la souris à Staphylococcus aureus Smith à une dose comprise entre 0,01 et 5 mg/kg par jour, par voie sous-cutanée.

Par ailleurs, la $DL_{50}$ des produits de formule générale (I) est comprise entre 0,5 g/kg et des doses supérieures à 2,5 g/kg par voie sous-cutanée chez la souris.

Parmi les produits selon l'invention, une sous-classe de produits intéressants peut être définie par la formule générale (I) dans laquelle les symboles $R_1$ et $R_2$ sont définis comme précédemment, le symbole $R_3$ représente un radical phényle, acylaminophényle, alcoylthio ou anilino, un radical benzoylamino ou thénoylamino éventuellement substitué sur l'atome d'azote (par un radical alcoyle ou phényle), un radical acylamino substitué sur l'atome d'azote (par un radical alcoyle ou phényle), un radical alcoyloxycarbonylamino, dialcoylaminoéthylamino, dialcoylaminométhylèneamino ou alcoylidènehydrazo, un radical acétamido substitué par un radical amino, amino-2 éthylthio ou L amino-2 carboxy-2 éthylthio, ou un radical de structure $-AR'_3$ dans lequel A représente un radical bivalent choisi parmi $-CH_2-$, $-NH-$ ou $NHCO-$ et $R'_3$ représente un radical méthyl-1 pyridinio-3 (ou −4), benzoylméthyl-1 pyridinio-3 (ou −4) ou carboxyméthyl-1 pyridinio-3 (ou −4), ou bien $R_1$ est un radical dithiol-1,3 one-2 yle-1, $R_2$ est un atome d'hydrogène et $R_3$ est un atome d'hydrogène, un radical alcoylamino, dialcoylamino ou acylamino et les symboles R et X sont définis comme précédemment.

D'un intérêt particulier sont aussi les produits de formule générale (I) dans laquelle $R_1$ représente un radical thiényle, furyle, phényle, p.hydroxyphényle, phénoxy ou dichloro-3,4 phénylthio et $R_2$ représente un atome d'hydrogène, ou bien $R_1$ représente un radical phényle ou p.hydroxyphényle et $R_2$ représente un radical amino, et $R_3$ représente un atome d'hydrogène, un radical alcoylamino, dialcoylamino ou acylamino, X est défini comme précédemment et R est un radical carboxy.

Et parmi les produits définis par la formule générale (I) plus spécialement intéressant sont les produits pour lesquels le symbole $R_1$ est un radical thiényle et le symbole $R_2$ est un atome d'hydrogène, ou bien le symbole $R_1$ est un radical phényle ou p.hydroxyphényle et le symbole $2_2$ est un radical amino, le symbole $R_3$ représente un atome d'hydrogène, un radical phényle, acylamino éventuellement substitué sur l'atome d'azote par un radical alcoyle, un radical dialcoylaminoéthylamino, un radical amino-2 éthylthioacetamido, L amino-2 carboxy-2 éthylthioacétamido, ou un radical de structure $-AR'_3$ tel que défini précédemment, et les symboles R et X sont définis comme précédemment.

Les exemples suivants, donnés à titre non limitatif, illustrent la présente invention.

## Exemple 1

A un solution de 2,27 g d'(acétamido-2 thiazolyl-5)-3 amino-7 benzhydryloxycarbonyl-2 oxo-8 thia-5 aza-1 bicyclo[4.2.0]octène-2 dans 27,5 $cm^3$ de tétrahydrofuranne sec à 2°C on ajoute en 5 minutes une solution de 0,55 $cm^3$ de chlorure de (thiényl-2) acétyle dans 5 $cm^3$ de tétrahydrofuranne sec puis, après 30 minutes, une solution de 0,63 $cm^3$ de triéthylamine dans 5 $cm^3$ de tétrahydrofuranne sec. Après 1 heures de réaction à 2°C le mélange réactionnel est filtré et le filtrat est dilué dans un mélange de 200 $cm^3$ d'acétate d'éthyle et 200 $cm^3$ de solution demi-saturée de bicarbonate de sodium. La couche organique est lavée par 100 $cm^3$ d'eau puis 100 $cm^3$ de solution saturée de chlorure de sodium, séchée sur sulfate de magnésium et concentrée à sec sous pression réduite (30 mm de mercure; 4 kPa) à 40°C. Le résidu est chromatographié sur une colonne (hauteur: 30 cm; diamètre: 5 cm) de silice (0,04—0,06 mm) en éluant sous 0,4 bar (40 kPa) par un mélange de cyclohexane et d'acétate d'éthyle 30—70 (en volumes) et en recueillant des fractions de 50 $cm^3$. Les fractions 15 à 22 contenant le produit pur sont rassemblées et concentrées sous pression réduite (30 mm de mercure; 4 kPa) à 40°C. On obtient 0,92 g d'(acétamido-2 thiazolyl-5)-3 benzhydryloxycarbonyl-2 oxo-8 (thiényl-2 acétamido)-7 thia-7 aza-1 bicyclo[4.2.0] octène-2 sous la forme d'une poudre jaune.

Spectre infra-rouge (KBr), bandes caractéristiques ($cm^{-1}$) 3280, 3200, 2500, 1785, 1770, 1725, 1695, 1670, 1650, 1550, 1530, 1495, 1450, 1220, 690.

Spectre de RMN du proton (350 MHz, $CDCl_3$, $\delta$ en ppm, J en Hz)

2,09 (s, 3H, $CH_3CO-$); 3,47 (AB limite, 2H, $-SCH_2$); 3,84 (s, 2H, $ArC\underline{H}_2CO-$);
5,04 (d, J = 4,5, 1H, H en 6); 5,88 (dd, J = 4,5 et 9, 1H, H en 7);
6,83 (s, 1H, $-OC\underline{H}Ar_2$); 6,9 à 7,0 (m, 2H, H en 3 et 4 du thiophène);
7,13 (s, 1H, H en 4 du thiazole);
7,10 à 7,45 (m, 16H, aromatiques, H en 5 du thiophène et $CON\underline{H}-C_7$).

Une solution de 0,85 g d'(acétamido-2 thiazolyl-5)-3 benzhydryloxycarbonyl-2 oxo-8 (thiényl-2 acétamido)-7 thia-5 aza-1 bicyclo[4.2.0] octène-2 dans 20 $cm^3$ d'acide formique est agitée pendant 30 minutes à 50°C puis concentrée à sec sous pression réduite (10 mm de mercure; 1,3 kPa). Le résidu est repris dans 50 $cm^3$ d'éthanol, agité pendant 5 minutes a 50°C puis le solvant est évaporé à sec sous pression réduite (30 mm de mercure; 4 kPa) à 40°C. Le résidu est repris dans 100 $cm^3$ d'éthanol; on agite pendant 10 minutes à 50°C puis essore le solide, qui est lavé par 3 fois 15 $cm^3$ d'éthanol et 3 fois 10 $cm^3$ d'éther éthylique. Après séchage, on obtient 0,53 g d'(acétamido-2 thiazolyl-5)- carboxy-2 oxo-8 (thiényl-2 acétamido)-7 thia-5 aza-1 bicyclo[4.2.0] octène-2 sous la forme d'une poudre jaune clair.

Spectre infra-rouge (KBr), bandes caractéristiques ($cm^{-1}$) 3260, 3200, 3100 à 2200, 1780, 1685, 1650, 1545, 1370, 700.

Spectre de RMN du proton (350 MHz, DMSO $d_6$, $\delta$ en ppm, J en Hz)

2,15 (s, 3H, $-CO-CH_3$); 3,74 et 3,80 (2d, J = 14, 2H, $-CO-CO_2-$);
3,76 et 3,88 (2d, J = 18, 2H, $-CH_2-S-$); 5,16 (d, J = 5, 1H, −H en 6);
5,72 (dd, J = 5 et 9, 1H, −H en 7); 6,90 à 7 (mt, 2H, =CH−CH= thiophène);
7,35 (dd, J = 4 et 1, 1H, =CH−S− thiophène); 7,50 (s,. 1H, −H thiazole);
9,16 (d, J = 9, 1H, −CO−NH−); 13,13 (s large, 1H, $-N\underline{H}-CO-CH_3$).

On obtient 4,25 g de sel de sodium de l'(acétamido-2 thiazolyl-5)-3 carboxy-2 oxo-8 (thiényl-2 acétamido)-7 thia-5 aza-1 bicyclo[4.2.0] octène-2 sous la forme d'un lyophilisat blanc en traitant, selon le mode opératoire décrit ci-après dans l'exemple 4, 5 g de l'acide correspondant par 0,85 g de bicarbonate de sodium dans 75 $cm^3$ d'eau et en chromatographiant la solution obtenue sur une colonne de 250 $cm^3$ de résine DUOLITE S 861 (préparée comme dans l'exemple 4), en éluant par 250 $cm^3$ d'eau distillée puis par des mélange d'eau et d'éthanol, successivement 95−5 en volumes (250 $cm^3$), 90−10 en volumes (250 $cm^3$) et 80−20 en volumes (1250 $cm^3$) et en recueillant des fractions de 60 $cm^3$, puis en réunissant et lyophilisant les fractions 5 à 24.

Spectre de RMN du proton (350 MHz, DMSO $d_6$, $\delta$ en ppm, J en Hz)

2,10 $\left(s, 3H, >N-CO-CH_3\right)$;

3,56 et 3,69 (2d, J = 18, 2H, $-S-CH_2-$);

3,73 et 3,80 $\left(2d, J = 14, 2H, het-CH_2-CON<\right)$;

5,02 (d, J = 5, 1H, −H en 6); 5,49 (dd, J = 9 et 5, 1H, −H en 7);
6,92 à 6,98 (mt, 2H, =CH−CH= du thiophène);
7,36 (dd, J = 4,5 et 1, 1H, =CH−S− du thiophène); 7,44 (s, 1H, −H du thiazole);
9,07 (d, J = 9, 1H, −CO−NH− en 7); 11,87 (s, 1H, Ar−NH−CO−).

L'(acétamido-2 thiazolyl-5)-3 amino-7 benzhydryloxycarbonyl-2 oxo-8 thia-5 aza-1 bicyclo[4.2.0] octène-2 peut être obtenu de la manière suivante:

4,6 g d'(acétamido-2 thiazolyl-5)-3 benzhydryloxycarbonyl-2 t.butoxycarbonylamino-7 oxo-8 thia-5 aza-1 bicyclo[4.2.0] octène-2 sont dissous dans 190 $cm^3$ d'acétonitrile à 35°C et traités par 2,9 g d'acide p.toluènesulfonique (monohydrate) en solution dans 20 $cm^3$ d'acétronitrile pendant 3 heures à 35°C, 16 heures à 25°C puis encore 6 heures à 35°C après addition de 0,95 g d'acide p.toluènesulfonique (monohydrate). Le mélange réactionnel est concentré partiellement sous pression réduite (30 mm de mercure; 4 kPa) à 30°C puis dilué par 200 $cm^3$ de chlorure de méthylène et 200 $cm^3$ de solution saturée de bicarbonate de sodium. La couche aqueuse est extraite par 100 $cm^3$ de chlorure de méthylène et les solutions organiques rassemblées sont lavées par 200 $cm^3$ d'eau puis séchées et concentrées sous pression réduite (30 mm de mercure; 4 kPa) à 30°C. Le résidu est séché sous pression réduite (10 mm de mercure; 1,3 kPa) à 25°C. On obtient 3,3 g d'(acétamido-2 thiazolyl-5)-3 amino-7 benzhydryloxycarbonyl-2 oxo-8 thia-5 aza-1 bicylo[4.2.0] octène-2 brut sous la forme d'une poudre beige.

Spectre infra-rouge ($CHBr_3$), bandes caracteristiques ($cm^{-1}$) 3440, 3400, 3340, 3260, 3160, 1780, 1720, 1695, 1670, 1560, 1515, 1495, 1450, 1370, 1220, 760.

Spectre de RMN du proton (350 MHz, $CDCl_3$, $\delta$ en ppm, J en Hz)

2,15 (s, 3H, $-CO-CH_3$); 3,57 et 3,74 (2d, J = 18, 2H, $-S-CH_2$);
4,84 (d, J = 4,5, 1H, −H en 7); 5,05 (d, J = 4,5, 1H, −H en 6);
6,92 (s, 1H, $-COO-C\underline{H}(C_6H_5)_2$); 7,03 (s, 1H, −H thiazole);
7,05 à 7,50 (mt, aromatiques); 11,45 (mf, 1H, −NH−CO−).

L'(acétamido-2 thiazolyl-5)-3 benzhydryloxycarbonyl-2 t.butoxycarbonylamino-7 oxo-8 thia-5 aza-1 bicyclo[4.2.0] octène-2 peut être obtenu selon l'un des procédés ci-après:

A) A une solution refroidie à 3°C de 5,87 g du mélange des épimères du benzhydryloxycarbonyl-2 t.butoxycarbonylamino-7 (bromo-1 oxo-2 éthyl)-3 oxo-8 thia-5 aza-1 bicyclo[4.2.0] octène-2 dans 58,7 $cm^3$ de tétrahydrofuranne sec on ajoute en 5 minutes une solution de 1,8 g d'acétylthiourée dans 20 $cm^3$ de tétrahydrofuranne sec. Le mélange réactionnel est ensuite agité pendant 4 heures 30 minutes à 25°C dilué 150 $cm^3$ d'acétate d'éthyle et 150 $cm^3$ de solution saturée de bicarbonate de sodium. La couche organique est lavée par 2 fois 100 $cm^3$ d'eau distillée puis par 100 $cm^3$ de solution saturée de chlorure de sodium et séchée. Après évaporation du solvant sous pression réduite (30 mm de mercure; 4 kPa) à 30°C le résidu est chromatographié sur une colonne (hauteur : 30 cm; diamètre : 6 cm) de silice (0,04−0,06 mm) en éluant sous une pression de 0,4 bar (40 kPa) par 1600 $cm^3$ de mélange de

cyclohexane et d'acétate d'éthyle 70–30 (en volumes) puis par 2000 $cm^3$ de mélange de cyclohexane et d'acétate d'éthyle 30–70 (en volumes) et en recueillant des fractions de 100 $cm^3$. Les fractions 27 à 33 contenant le produit pur sont rassemblées et évaporées à sec. On obtient 0,54 g d'(acétylamino-2 thiazolyl-5)-3 benzhydryloxycarbonyl-2 t. butoxycarbonylamino-7 oxo-8 thia-5 aza-1 bicyclo[4.2.0] octène-2 sous la forme d'une meringue jaune.

Rf = 0,45; chromatoplaque de gel de silice, éluant: cyclohexane-acétate d'éthyle 25–75 (en volumes).

Spectre infra-rouge ($CHBr_3$), bandes caractéristiques ($cm^{-1}$) 3400, 3270, 3220, 3180, 1790, 1730, 1700, 1545, 1510, 1495, 1455, 1370, 1230, 1165, 760, 745, 700.

Spectre de RMN du proton (350 MHz, DMSO $d_6$, $\delta$ en ppm, J en Hz)

1,42 (s, 9H, $(CH_3)_3C$–); 2,14 (s, 3H, $CH_3CO$–); 3,76 et 3,88 (AB, J = 18, 2H, –$SCH_2$–); 5,17 (d, J = 4, 1H, H en 6); 5,59 (dd, J = 4 et 9, 1H, H en 7); 6,81 (s, 1H, –$CHAr_2$); 7 à 7,4 (m, 11H, $H_4$ thiazole et aromatiques); 8,04 (d, J = 9, 1H, –CONH–$C_7$); 12,05 (s, 1H, –N<u>H</u>–$COCH_3$).

A une solution refroidie à –55°C de 2 g de benzhydryloxycarbonyl-2 t.butoxycarbonylamino-7 (diméthylamino-2 vinyl)-3 oxo-8 thia-5 aza-1 bicyclo[4.2.0] octène-2 (isomère E) dans 11 $cm^3$ de tétrahydrofuranne sec on ajoute goutte à goutte, en 5 minutes, une solution de 0,2 $cm^3$ de brome dans 2 $cm^3$ de chlorure de méthylène sec. Le mélange réactionnel est agité pendant 1 heures à –60°C puis versé dans un mélange de 200 $cm^3$ d'acétate d'éthyle et 200 $cm^3$ d'eau glacée. La couche organique est lavée par 100 $cm^3$ de solution demi-saturée de bicarbonate de sodium puis par 100 $cm^3$ d'eau et 100 $cm^3$ de solution demi-saturée de chlorure de sodium et séchée sur sulfate de sodium en présence de noir décolorant. Après filtration, le solvant est évaporé sous pression réduite (30 mm de mercure; 4 kPa) à 30°C. Le résidu est repris par 50 $cm^3$ d'oxyde d'isopropyle que l'on évapore sous pression réduite (30 mm de mercure; 4 kPa) à 30°C. On obtient 1,8 g de benzhydryloxycarbonyl-2 (bromo-1 oxo-2 éthyl)-3 t.butoxycarbonylamino-7 oxo-8 thia-5 aza-1 bicyclo[4.2.0] octène-2 sous la forme d'un solide beige clair (mélange des deux épimères du bromoaldéhyde).

Spectre infra-rouge ($CHBr_3$), bandes caractéristiques ($cm^{-1}$) 3420, 1790, 1725, 1505, 1455, 1390, 1370, 1245, 1225, 760, 745.

Spectre de RMN du proton ($CDCl_3$, 350 MHz, $\delta$ en ppm, J en Hz).

épimère A

1,42 (s, 9H, $(CH_3)_3C$–); 3,71 et 3,55 (AB, J = 17,5, 2H, –$SCH_2$–); 5,06 (d, J = 4, 1H, H en 6); 5,22 (d, J = 9, 1H, –NH–); 5,65 (dd, J = 4 et 9, 1H, H en 7); 6,01 (s, 1H, –C<u>H</u>Br–); 6,99 (s, 1H, –$CHAr_2$); 7,3 à 7,5 (m, 10H, aromatiques);

9,31 (s, 1H, –CHO).

épimère B

1,42 (s, 9H, $(CH_3)_3C$–); 3,35 et 3,65 (AB, J = 17,5, 2H, –$SCH_2$–); 5,01 (d, J = 4, 1H, H en 6); 5,29 (d, J = 9, 1H, –NH–); 5,72 (dd, J = 4 et 9, 1H, H en 7); 6,00 (s, 1H, –C<u>H</u>Br–); 6,92 (s, 1H, –$CHAr_6$); 7,3 à 7,5 (m, 10H, aromatiques); 9,30 (s, 1H, –CHO).

Le benzhydryloxycarbonyl-2 t.butoxycarbonylamino-7 (diméthylamino-2 vinyl)-3 oxo-8 thia-5 aza-1 bicyclo[4.2.0] octène-2 (isomère E) peut être préparé comme décrit dans le brevet belge 883 415.

B) A un solution refroidie à 0°C de 10 g d'(amino-2 thiazolyl-5)-3 benzhydryloxycarbonyl-2 t.butoxycarbonylamino-7 oxo-8 thia-5 aza-1 bicyclo[4.2.0] octène-2 dans 150 $cm^3$ de tétrahydrofuranne sec on ajoute goutte à goutte, en 5 minutes, une solution de 1,51 $cm^3$ de chlorure d'acétyle dans 10 $cm^3$ de tétrahydrofuranne sec puis une solution de 2,8 $cm^3$ de triéthylamine dans 5 $cm^3$ de tétrahydrofuranne sec. Le mélange réactionnel est agité pendant 1 heures 30 minutes vers 0–5°C puis traité de nouveau par 1,51 $cm^3$ de chlorure d'acétyle dans 10 $cm^3$ de tétrahydrofuranne. Après 1 heure 50 minutes à 5°C le mélange réactionnel est filtré, le filtrat concentré 50 $cm^3$ et dilué par 250 $cm^3$ d'acétate d'éthyle. La phase organique est lavée par 100 $cm^3$ de solution saturée de bicarbonate de sodium, 100 $cm^3$ d'eau distillée et 100 $cm^3$ de solution demi-saturée de chlorure de sodium puis séchée sur sulfate de magnésium et concentrée à sec. On obtient 10 g d'(acétylamino-2 thiazolyl-5)-3 benzhydryloxycarbonyl-2 t.butoxycarbonylamino-7 oxo-8 thia-5 aza-1 bicyclo[4.2.0] octène-2 brut sous la forme d'une meringue brune. Par cristallisation dans 25 $cm^3$ d'acétonitrile on obtient 5,9 g d'(acétylamino-2 thiazolyl-5)-3 benzhydryloxycarbonyl-2 t.butoxycarbonylamino-7 oxo-8 thia-5 aza-1 bicyclo[4.2.0] octène-2 pur, sous la forme d'une poudre cristalline blanchâtre dont les caractéristiques sont identiques à celles du produit obtenu dans la variante (A).

L'(amino-2 thiazolyl-5)-3 benzhydryloxycarbonyl-2 t.butoxycarbonylamino-7 oxo-8 thia-5 aza-1

bicyclo[4.2.0] octène-2 peut être obtenu de la manière suivant:

A une solution refroidie à −55°C de 5,35 g benzhydryloxycarbonyl-2 t.butoxycarbonylamino-7 (diméthylamino-2 vinyl)-3 oxo-8 thia-5 aza-1 bicyclo[4.2.0] octène-2 forme E dans 20 $cm^3$ de tétrahydrofuranne sec on ajoute en 5 minutes une suspension de 2,54 g d'iode dans 20 $cm^3$ de chlorure de méthylène sec. Le mélange réactionnel est agité pendant 2 heures en laissant progressivement remonter la température à 0°C puis traité par 0,36 $cm^3$ d'eau distillée et agité pendant 2 heures à 5°C. A 25 $cm^3$ de ce mélange réactionnel on ajoute une solution de 0,57 g de thiourée dans un mélange de 5 $cm^3$ de tétrahydrofuranne et de 2 $cm^3$ d'eau puis on laisse agiter pendant 16 heures à 25°C. On ajoute 0,57 g de thiourée et agite encore pendant 3 heures à 25°C puis on dilue par 150 $cm^3$ d'acétate d'éthyle. La phase organique est lavée successivement par 100 $cm^3$ d'eau distillée, 100 $cm^3$ de solution saturée de bicarbonate de sodium, 100 $cm^3$ d'eau distillée et 100 $cm^3$ de solution demi-saturée de chlorure de sodium. Après séchage sur sulfate de magnésium le solvant est évaporé sous pression réduite (30 mm de mercure; 4 kPa) à 30°C. Le résidu est chromatographié sur une colonne (hauteur: 30 cm; diamètre: 2 cm) de silice (0,04—0,06 mm) en éluant sous 0,4 bar (40 kPa) par un mélange de cyclohexane et d'acétate d'éthyle 45—55 (en volumes). Après évaporation des solvants on recueille 0,08 g d'(amino-2 thiazolyl-5)-3 benzhydryloxycarbonyl-2 t.butoxycarbonylamino-7 oxo-8 thia-5 aza-1 bicyclo[4.2.0] octène-2 sous la forme d'une meringue orangée dont les caractéristiques sont identiques à celles dur produit décrit ci-après à l'exemple 2.

## Exemple 2

Une solution de 2,9 g de (benzamido-2 thiazolyl-5)-3 benzhydryloxycarbonyl-2 t.butoxycarbonylamino-7 oxo-8 thia-5 aza-1 bicylo[4.2.0] octène-2 dans 29 $cm^3$ d'acétonitrile est traitée par 2,9 $cm^3$ d'acide méthanesulfonique selon le mode opératoire de l'exemple 15. On obtient 2 g d'amino-7 (benzamido-2 thiazolyl-5)-3 benzhydryloxycarbonyl-2 oxo-8 thia-5 aza-1 bicyclo[4.2.0] octène-2 brut sous la forme d'une meringue jaune.

Rf = 0,22 (chromatoplaque de gel de silice, éluant: mélange de cyclohexane et d'acétate d'éthyle 30—70 en volumes).

Ce produit est repris dans 50 $cm^3$ de tétrahydrofuranne sec et acylé par 0,53 $cm^3$ de chlorure de(thiényl-2)acétyle en présence de 0,6 $cm^3$ de triéthylamine en opérant selon le mode opératoire décrit dans l'exemple 15. Le produit brut est chromatographié sur une colonne (hauteur: 22 cm; diamètre: 4,4 cm) de gel de silice (0,04—0,06 mm) en éluant sous une pression de 0,4 bar (40 kPa) par un mélange de cyclohexane et d'acétate d'éthyle (50—50 en volumes) et en recueillant des fractions de 125 $cm^3$. Les fractions 7 à 16 contenant le produit pur sont concentrées à sec sous pression réduite (30 mm de mercure; 4 kPa) à 30°C. On obtient 1,5 g de (benzamido-2 thiazolyl-5)-3 benzhydryloxycarbonyl-2 oxo-8 (thiényl-2 acétamido)-7 thia-5 aza-1 bicyclo[4.2.0] octène-2 sous la forme d'une meringue jaune.

Spectre infra-rouge ($CHBr_3$), bandes caractéristiques ($cm^{-1}$) 3400, 3300—2500, 1785, 1725, 1675, 1600, 1580, 1535, 1505, 1490, 1450, 755, 740.

Spectre de RMN du proton (350 MHz, DMSO $d_6$, $\delta$ en ppm, J en Hz)

3,74 et 3,81 (2d, J = 14, 2H, $-CH_2-CO-N\langle$);

3,81 et 3,95 (2d, J = 18, 2H, $-CH_2-S-$); 5,24 (d, J = 5, 1H, −H en 6);
5,82 (dd, J = 5 et 8, −H en 7); 6,84 (s, 1H, $-COO-\underline{CH}(C_6H_5)_2$);
6,90 à 7 (mt, 2H, =CH—CH= du thiophène); 7 à 7,4 (mt, 10H, aromatiques);
7,35 (dd, J = 4 et 1, 1H, =CH—S— du thiophène); 7,42 (s, 1H, −H thiazole);
7,55 (t, J = 7,5, 2H, aromatiques en méta du benzamido);
7,66 (t, J = 7,5, 1H, aromatiques en para du benzamido);
8,08 (d, J = 7,5, 2H, aromatiques en ortho du benzamido); 9,23 (d, J = 8, 1H, —CO—NH—);
12,60 (s, 1H, $-\underline{NH}CO-C_6H_5$).

Une solution de 1,5 g de (benzamido-2 thiazolyl-5)-3 benzhydryloxycarbonyl-2 oxo-8 (thiényl-2 acétamido)-7 thia-5 aza-1 bicyclo[4.2.0] octène-2 dans un mélange de 43 $cm^3$ d'acide formique et 8 $cm^3$ d'eau distillée est chauffée pendant 30 minutes à 60°C puis diluée par 35 $cm^3$ d'eau distillée. Le précipité est essoré, lavé par 2 fois 10 $cm^3$ d'acide formique à 50% (en volumes) puis par 3 fois 75 $cm^3$ d'oxyde d'isopropyle et séché; on obtient 1 g de (benzamido-2 thiazolyl-5)-3 carboxy-2 oxo-8 (thiényl-2 acétamido)-7 thia-5 aza-1 bicyclo[4.2.0] octène-2 sous la forme d'un solide blanc.

Spectre infra-rouge (KBr), bandes caractéristiques ($cm^{-1}$) 3400, 3260, 3100—2100, 1785, 1665, 1605, 1585, 1550, 1495, 1450, 705.

Spectre de RMN du proton (350 MHz, DMSO $d_6$, $\delta$ en ppm, J en Hz)

3,73 et 3,82 (2d, J = 14, 2H, $-CH_2-CO-N\langle$);

3,80 et 3,95 (2d, J = 18, 2H, $-CH_2-S-$); 5,20 (d, J = 5, 1H, −H en 6);

5,75 (dd, J = 5 et 9, –H en 7); 6,9 à (mt, 2H, =CH–CH= du thiophène);
7,35 (dd, J = 4 et 1, 1H, =CH–S– du thiophène);
7,53 (t, J = 7,5, 2H, aromatiques en méta du benzamido); 7,63 (s, 1H, –H thiazole);
7,64 (t, J = 7,5, 1H, aromatiques en para du benzamido);
8,10 (d, J = 7,5, 2H, aromatiques en ortho du benzamido); 9,18 (d, J = 9, 1H, –CO–NH–);
11,60 (mf, 1H, –COOH); 12,45 (mf, 1H, –N$\underline{H}$–CO–$C_6H_5$).

En traitant 4,5 g d'(amino-2 thiazolyl-5)-3 benzhydryloxycarbonyl-2 t.butoxycarbonylamino-7 oxo-8 thia-5 aza-1 bicyclo[4.2.0] octène-2 par 1,11 $cm^3$ de chlorure de benzoyle dans 55 $cm^3$ de tétrahydrofuranne sec en présence de 1,23 $cm^3$ de triéthylamine selon le mode opératoire de l'exemple 1 (B), on obtient 1,8 g de (benzamido-2 thiazolyl-5)-3 benzhydryloxycarbonyl-2 t.butoxycarbonylamino-7 oxo-8 thia-5 aza-1 bicyclo[4.2.0] octène-2 sous la forme de cristaux de couleur crème.

Spectre infra-rouge (KBr), bandes caractéristiques ($cm^{-1}$) 3420, 3240, 1790, 1730, 1705, 1675, 1600, 1580, 1545, 1510, 1500, 1455, 1370, 1160, 760, 700.

Spectre de RMN du proton (350 MHz, DMSO $d_6$, $\delta$ en ppm, J en Hz)

1,45 (s, 9H, –$C(CH_3)_3$); 3,83 et 3,97 (2d, J = 18, 2H, –$CH_2$–S–); 5,24 (d, J = 5, 1H, –H en 6);
5,.67 (dd, J = 5 et 9, 1H, –H en 7); 6,91 (s, 1H, –COO–C$\underline{H}$($C_6H_5$)$_2$);
7,05 à 7,45 (mt, 10H, aromatiques); 7,49 (s, 1H, –H thiazole);
7,63 (t, J = 7,5, 2H, aromatiques en méta du benzamido);
7,72 (t, J = 7,5, 1H, aromatiques en para du benzamido); 8,13 (d, J = 9, 1H, –CO–NH–);
8,16 (d, J = 7,5, 2H, aromatiques en ortho du benzamido); 12,72 (s, 1H, –N$\underline{H}$–CO–$C_6H_5$).

Une solution de 107,13 g de benzhydryloxycarbonyl-2 t.butoxycarbonylamino-7 (diméthylamino-2 vinyl)-3 oxo-8 thia-5 aza-1 bicyclo[4.2.0] octène-2 isomère E dans 500 $cm^3$ de tétrahydrofuranne sec est refroidie à –50°C. On ajoute goutte à goutte en 35 minutes une solution de 10,25 $cm^3$ de brome dans 30 $cm^3$ de chlorure de méthylène sec. On laisse remonter en 1 heures la température jusqu'à –15°C puis ajoute 7,2 $cm^3$ d'eau distillée. Le mélange réactionnel est agité pendant 45 minutes entre –15°C et –5°C puis traité par une solution de 22,8 g de thiourée dans un mélange de 100 $cm^3$ de tétrahydrofuranne et de 20 $cm^3$ de'eau distillée. On agite pendant 3 heures à 25°C puis concentre à un volume résiduel de 350 $cm^3$ et dilue par 1500 $cm^3$ d'acétate d'éthyle. La couche organique est lavée successivement par 500 $cm^3$ de solution saturée de bicarbonate de sodium, 500 $cm^3$ de solution demi-saturée de bicarbonate de sodium, 2 fois 500 $cm^3$ d'eau distillée et 2 fois 500 $cm^3$ de solution demi-saturée de chlorure de sodium, séchée sur sulfate de sodium et concentrée à sec sous pression réduite (30 mm de mercure; 4 kPa) à 40°C. Le résidu est fixé sur 400 $cm^3$ de silice (0,2–0,063 mm) et chromatographié sur une colonne de 900 g de silice (0,2–0,063 mm) (diamètre: 9 cm) en éluant par des mélanges cyclohexane-acétate d'éthyle de compositions successives (en volumes) 70–30 (2 litres); 50–50 (6 litres); 40–60 (2 litres) et 20–80 (2 litres) puis par 4 litres d'acétate d'éthyle pur en recueillant des fractions de 800 $cm^3$. Les fractions 10 à 15 contenant le produit pur sont rassemblées et concentrées à sec sous pression réduite (30 mm de mercure; 4 kPa) à 40°C. On obtient 34,4 g d'(amino-2 thiazolyl-5)-3 benzhydryloxycarbonyl-2 t.butoxycarbonylamino-7 oxo-8 thia-5 aza-1 bicyclo[4.2.0] octène-2 sous la forme d'une meringue orangée.

Spectre infra-rouge ($CHBr_3$), bandes caractéristiques ($cm^{-1}$) 3480, 3420, 3380, 1780, 1720, 1600, 1495, 1455, 1390, 1370, 1240, 1220, 755, 740.

Spectre de RMN du proton (350 MHz, $CDCl_3$, $\delta$ en ppm, J en Hz)

1,47 (s, 9H, –$C(CH_3)_3$); 3,46 et 3,61 (2d, J = 18, 2H, –S–$CH_2$–);
5,01 (d, J = 4,5, 1H, –H en 6); 5,27 (mf, 2H, –$NH_2$); 5,57 (d, J = 9, 1H, –CO–NH);
5,64 (dd, J = 9 et 4,5, 1H, –H en 7); 6,85 (s, 1H, –COO–C$\underline{H}$($C_6H_5$)$_2$);
6,94 (s, 1H, –H thiazole); 7,10 à 7,50 (mt, 10H, aromatiques).

## Exemple 3

En opérant comme dans l'exemple 2 à partir de 4,5 g d'(amino-2 thiazolyl-5)-3 benzhydryloxycarbonyl-2 t.butoxycarbonylamino-7 oxo-8 thia-5 aza-1 bicyclo[4.2.0] octène-2, 1,23 $cm^3$ de triéthylamine et 1,4 g de chlorocarbonyl-2 thiophène on obtient, après purification sur gel de silice Merck (0,06–0,2 mm), 4,62 g de benzhydryloxycarbonyl-2 t.butoxycarbonylamino-7 oxo-8 [(thiényl-2 carbonylamino)-2 thiazolyl-5]-3 thia-5 aza-1 bicyclo[4.2.0] octène-2 la forme d'une poudre de couleur crème.

Spectre infra-rouge (KBr), bandes caractéristiques ($cm^{-1}$) 3420, 3230, 1790, 1730, 1700, 1660, 1545, 1505, 1165, 850, 740.

Spectre de RMN du proton (360 MHz, $CDCl_3$, $\delta$ en ppm, J en Hz)

1,49 (s, 9H, –$C(CH_3)_3$); 3,55 et 3,71 (2d, J = 18, 2H, –$SCH_2$–); 5,07 (d, J = 5, 1H, H en 6);
5,33 (d, J = 9, 1H, –CONH–); 5,72 (dd, J = 5 et 9, 1H, H en 7); 6,94 (s, 1H, –COOCH–);

7,0 (s large, 1H, H en 4 du thiophène); 7,69 (d, J = 4,5, 1H, H en 3 du thiophène); 7,74 (s large, 1H, H en 5 du thiophène).

On traite à 20°C pendant 30 minutes 4,62 g de benzhydryloxycarbonyl-2 t.butoxycarbonylamino-7 oxo-8 [(thiényl-2 carbonylamino)-2 thiazolyl-5]-3 thia-5 aza-1 bicyclo[4.2.0] octène-2 par 45 $cm^3$ d'acide trifluoroacétique. On concentre à sec à 20°C sous 0,05 mm de mercure (0,007 kPa) et reprend par 2 fois 50 $cm^3$ d'acétate d'éthyle en concentrant à sec à chaque fois à 30°C sous 20 mm de mercure (2,7 kPa). Le solide résiduel est trituré dans 100 $cm^3$ d'éther diéthylique. Après filtration on recueille 3,45 g d'amino-7 carboxy-2 oxo-8 [(thiényl-2 carbonylamino)-2 thiazolyl-5]-3 thia-5 aza-1 bicyclo[4.2.0] octène-2 à l'acétat de trifluoracétate sous la forme d'une poudre brute jaune.

Spectre infra-rouge (KBr), bandes caractéristiques ($cm^{-1}$) 3300—3200, 1790, 1670, 1555, 1200, 1150, 840, 800, 725.

En procédant comme dans l'exemple 6 à partir de 3,45 g de trifluoracétate d'amino-7 carboxy-2 oxo-8 [(thiényl-2 carbonylamino)-2 thiazolyl-5]-3 thia-5 aza-1 bicyclo[4.2.0] octène-2 et de 0,839 $cm^3$ de chlorure de thiényl-2 acétyle, on obtient 1,52 g d'une poudre brute de couleur crème. On la purifie à l'état de sel de sodium en solution aqueuse, par passage sur une colonne de 20 $cm^3$ de résine DUOLITE S 861 (diamètre de la colonne: 2 cm). On élue par 250 $cm^3$ d'eau, 250 $cm^3$ d'eau contenant 5% d'éthanol, 250 $cm^3$ d'eau contenant 10% d'éthanol et 250 $cm^3$ d'eau contenant 20% d'éthanol en recueillant des fractions de 30 $cm^3$. Les fractions 9 à 25 sont lyophilisées. On recueille 0,5 g de carboxy-2 oxo-8 [(thiényl-2)-2 acétamido]-7 [(thiényl-2 carbonylamino)-2 thiazolyl-5]-3 thia-5 aza-1 bicyclo[4.2.0] octène-2 à l'état de sel de sodium sous la forme d'un lyophilisat de couleur crème.

Spectre infra-rouge (KBr), bandes caractéristiques ($cm^{-1}$) 3300, 1755, 1650, 1610, 1555, 1505, 1410 et 730.

## Exemple 4

En traitant 5,56 g de benzhydryloxycarbonyl-2 t.butoxycarbonylamino-7 (N-méthylacétamido-2 thiazolyl-5)-3 oxo-8 thia-5 aza-1 bicyclo[4.2.0] octène-2 par 4 $cm^3$ d'acide méthanesulfonique dans 50 $cm^3$ d'acétonitrile selon le mode opératoire de l'exemple 15 on obtient 4,6 g d'amino-7 benzhydryloxycarbonyl-2 (N-méthylacétamido-2 thiazolyl-5)-3 oxo-8 thia-5 aza-1 bicyclo[4.2.0] octène-2 brut que l'on acyle par 1,25 $cm^3$ de chlorure de (thiényl-2) acétyle selon le mode opératoire décrit dans l'exemple 1. On chromatographie sur une colonne (hauteur: 30 cm; diamètre: 6 cm) de gel de silice (0,04—0,06 mm) en éluant sous une pression de 0,5 bar (50 kPa) par un mélange de cyclohexane et d'acétate d'éthyle (35—65 en volumes) et en recueillant des fractions de 100 $cm^3$. Les fractions 18 à 28 contenant le produit pur sont réunies et concentrées à sec sous pression réduite (30 mm de mercure) à 30°C. On obtient 3,34 g de benzhydryloxycarbonyl-2 (N-méthylacétamido-2 thiazolyl-5)-3 oxo-8 [(thiényl-2) acétamido]-7 thia-5 aza-1 bicyclo[4.2.0] octène-2 sous la forme d'une merigue de couleur crème.

Spectre infra-rouge ($CHCl_3$), bandes caractéristiques ($cm^{-1}$) 3400, 1790, 1730, 1680, 1510, 1465, 1380, 695.

Spectre de RMN du proton (350 MHz, $CDCl_3$, $\delta$ en ppm, J en Hz)

2,40 (s, 3H, $>N—CO—CH_3$);

3,53 et 3,68 (2d, J = 18, 2H, $—S—CH_2—$);

3,54 (s, 3H, $>NCH_3$);

3,88 (AB limite, 2H, hét$CH_2CON<$);

5,06 (d, J = 5, 1H, —H en 6); 5,92 (dd, J = 9 et 5, 1H, —H en 7);
6,33 (d, J = 9, 1H, —CO—NH—); 6,89 (s, 1H, $—COO—\underline{CH}(C_6H_5)_2$); 7,12 (s, 1H, —H du thiazole);
7 à 7,35 (mt, aromatiques et thiényle).

3,3 g de benzhydryloxycarbonyl-2 (N-méthylacétamido-2 thiazolyl-5)-3 oxo-8 [(thiényl-2) acétamido]-7 thia-5 aza-1 bicyclo[4.2.0] octène-2 sont dissous dans un mélange de 20 $cm^3$ d'acide formique et de 7 $cm^3$ d'anisole à 50°C. Après 20 minutes à cette température le mélange réactionnel est concentré à sec sous pression réduite (2 mm de mercure; 0,3 kPa) à 30°C. Le résidu est trituré avec 100 $cm^3$ d'éthanol absolu que l'on évapore sous pression réduite (30 mm de mercure; 4 kPa) à 30°C. Le résidu est trituré avec 150 $cm^3$ d'éthanol absolu à 50°C. L'insoluble est essoré, lavé par 50 $cm^3$ d'éthanol absolu, puis par 3 fois 50 $cm^3$ d'oxyde d'isopropyle puis séché. On obtient 1,44 g de carboxy-2 (N-méthylacétamido-2 thiazolyl-5)-3 oxo-8 [(thiényl-2) acétamido]-7 thia-5 aza-1 bicyclo[4.2.0] octène-2 sous la forme d'un solide blanc-crème que l'on redissout dans une solution de 0,25 g de bicarbonate de sodium dans 15 $cm^3$ d'eau distillée. La solution est filtrée puis versée sur une

colonne (diamètre: 2 cm) de 70 $cm^3$ de résine DUOLITE S 861 (préalablement lavée par 250 $cm^3$ d'alcool, 250 $cm^3$ d'eau distillée, puis par une solution de chlorure de sodium à 1% jusqu'à neutralité puis par de l'eau distillée jusqu'à élimination des ions chlorures). On élue successivement par 450 $cm^3$ d'eau distillée puis par 450 $cm^3$ de mélange eau-éthanol (95—5 en volumes) en recueillant des fractions de 30 $cm^3$. Les fractions 5 à 24 sont réunies et lyophilisées. On obtient 1,17 g de sel de sodium du carboxy-2 (N-méthylacétamido-2 thiazolyl-5)-3 oxo-8 [(thiényl-2) acétamido]-7 thia-5 aza-1 bicyclo[4.2.0] octène-2 sous la forme d'un lyophilisat jaune pâle.

Spectre infra-rouge (KBr), bandes caractéristiques ($cm^{-1}$) 3300, 1760, 1670, 1605, 1530, 1380, 700.

Spectre de RMN du proton (350 MHz, DMSO $d_6$, $\delta$ en ppm, J en Hz)

2,35 (s, 3H, $>N-CO-CH_3$);
3,56 et 3,68 (2d, J = 18, 2H, $-CH_2-S-$);
3,58 (s, 3H, $>N-CH_3$);
3,78 (AB limite, J = 14, 2H, hét$-CH_2-CO-N<$);
5,04 (d, J = 4,5, 1H, —H en 6); 5,50 (dd, J = 4,5 et 8, 1H, —H en 7);
6,92 à 6,97 (mt, 2H, =CH—CH= du thiophène);
7,37 (dd, J = 4,5 et 1, 1H, =CH—S— du thiophène); 7,51 (s, 1H, —H du thiazole);
9,09 (d, J = 8, 1H, —CO—NH— en 7).

Le benzhydryloxycarbonyl-2 t.butoxycarbonylamino-7 (N-méthylacétamido-2 thiazolyl-5)-3 oxo-8 thia-5 aza-1 bicyclo[4.2.0] octène-2 peut être préparé de la manière suivante:

7 g de benzhydryloxycarbonyl-2 t.butoxycarbonylamino-7 (méthylamino-2 thiazolyl-5)-3 oxo-8 thia-5 aza-1 bicyclo[4.2.0] octène-2 sont dissous dans 45 $cm^3$ de tétrahydrofuranne sec à 0°C et traités par une solution de 1,7 $cm^3$ de chlorure d'acétyle dans 5 $cm^3$ de tétrahydrofuranne sec puis par 3,1 $cm^3$ de triéthylamine en solution dans 5 $cm^3$ de tétrahydrofuranne. Le mélange réactionnel est agité pendant 6 heures à 0°C et 16 heures à 25°C puis dilué par 250 $cm^3$ d'acétate d'éthyle et lavé par 100 $cm^3$ d'eau additionnée de 50 $cm^3$ de solution saturée de bicarbonate de sodium, puis par 2 fois 250 $cm^3$ d'eau distillée et par 100 $cm^3$ de solution saturée de chlorure de sodium. Après séchage sur sulfate de magnésium, filtration et évaporation du solvant sous pression réduite (30 mm de mercure; 4 kPa) à 30°C le résidu obtenu est purifié par chromatographie sur une colonne (hauteur: 30 cm, diamètre: 6 cm) de gel de silice (0,04—0,06 mm) en éluant sous une pression de 50 kPa par un mélange de cyclohexane et d'acétate d'éthyle (35—65 en volumes) et en recueillant des fractions de 100 $cm^3$. Les fractions 10 à 18 contenant le produit pur sont réunies et concentrées à sec sous pression réduite (30 mm de mercure; 4 kPa) à 30°C. On obtient 5,33 g de benzhydryloxycarbonyl-2 t.butoxycarbonylamino-7 (N-méthylacétamido-2 thiazolyl-5)-3 oxo-8 thia-5 aza-1 bicyclo[4.2.0] octène-2 sous la forme d'une meringue de couleur crème.

Spectre infra-rouge ($CHCl_3$), bandes caractéristiques ($cm^{-1}$) 3340, 1785, 1720, 1670, 1505, 1460, 1380, 1370, 1160, 700.

Spectre de RMN du proton (350 MHz, $CDCl_3$, $\delta$ en ppm, J en Hz)

1,48 (s, 9H, $-C(CH_3)_3$);
2,4 (s, 3H, $>N-CO-CH_3$);
3,54 (s, 3H, $>N-CH_3$);
3,58 et 3,72 (2d, J = 18, 2H, $-CH_2-S-$); 5,07 (d, J = 5, 1H, —H en 6);
5,26 (d, J = 9, 1H, —CO—NH—); 5,7 (dd, J = 9, 1H, —H en 7); 6,9 (s, 1H, $-COO-\underline{CH}(C_6H_5)_2$);
7,05 à 7,45 (mt, aromatiques et —H du thiazole).

Le benzhydryloxycarbonyl-2 t.butoxycarbonylamino-7 (méthylamino-2 thiazolyl-5)-3 oxo-8 thia-5 aza-1 bicyclo[4.2.0] octène-2 peut être préparé en opérant selon le mode opératoire de l'exemple 2, mais en remplaçant la thiourée par de la N-méthylthiourée (4 g). A partir de 20 g de benzhydryloxycarbonyl-2 t.butoxycarbonylamino-7 (diméthylamino-2 vinyl)-3 oxo-8 thia-5 aza-1 bicyclo[4.2.0] octène-2 (forme E), et après purification par chromatographie sur une colonne (hauteur: 45 cm, diamètre: 4 cm) de gel de silice (0,2—0,06 mm) en éluant par des mélanges de cyclohexane et d'acétate d'éthyle successivement 50—50 en volumes (1,5 litre), 40—60 en volumes (1 litre), 20—80 en volumes (1,5 litre) et par 1 litre d'acétate d'éthyle en réunissant et évaporant à sec sous pression réduite (30 mm de

mercure; 4 kPa) à 30°C les fractions contenant le produit attendu (d'après examen en CCM), on obtient 4,74 g de benzhydryloxycarbonyl-2 t.butoxycarbonylamino-7 (méthylamino-2 thiazolyl-5)-3 oxo-8 thia-5 aza-1 bicyclo[4.2.0] octène-2 sous la forme d'une meringue brun clair.

Spectre infra-rouge ($CHCl_3$), bandes caractéristiques ($cm^{-1}$) 3440, 1785, 1725, 1560, 1510, 1460, 1396, 1370, 1160, 700.

Spectre de RMN du proton (350 MHz, DMSO $d_6$, $\delta$ en ppm, J en Hz)

1,44 (s, 9H, $(CH_3)_3C$—);
2,73 (d, J = 5, 3H, >N—$CH_3$);
3,72 et 3,80 (AB, J = 18, 2H, —$SCH_2$—); 5,16 (d, J = 4,5, 1H, H en 6);
5,49 (dd, J = 4,5 et 9, 1H, H en 7); 6,84 (s, 1H, —C$\underline{H}$$Ar_2$); 7,2 à 7,45 (mt, 11H, aromatiques);
7,79 (q, J = 5, 1H, —N$\underline{H}$$CH_3$); 8,04 (d, J = 9, 1H, —CON$\underline{H}$—).

## Exemple 5

En opérant selon le mode opératoire décrit dans l'exemple 15 mais à partir de 3,85 g de benzhydryloxycarbonyl-2 t.butoxycarbonylamino-7 oxo-8 (thiazolyl-5)-3 thia-5 aza-1 bicyclo[4.2.0] octène-2, on prépare le dérivé amino-7 par action de 3,8 $cm^3$ d'acide méthanesulfonique dans 38 $cm^3$ d'acétonitrile puis on acyle l'amino-7 benzhydryloxycarbonyl-2 oxo-8 (thiazolyl-5)-3 thia-5 aza-1 bicyclo[4.2.0] octène-2 brut obtenu par 0,86 $cm^3$ de chlorure de (thiényl-2) acétyle dans 65 $cm^3$ de tétrahydrofuranne sec en présence de 0,98 $cm^3$ de triéthylamine. Après chromatographie sur une colonne (hauteur: 30 cm; diamètre = 4 cm) de gel de silice (0,04—0,06 mm) en éluant sous une pression de 0,4 bar (40 kPa) par un mélange de cyclohexane et d'acétate d'éthyle 30—70 (en volumes), et concentration à sec sous pression réduite des fractions 11 à 20 (de 40 $cm^3$ chacune) contenant le produit pur, on obtient 2,67 g de benzhydryloxycarbonyl-2 oxo-8 (thiazolyl-5)-3 (thiényl-2 acétamido)-7 thia-5 aza-1 bicyclo[4.2.0] octène-2 sous la forme d'une meringue de couleur crème.

Spectre infra-rouge ($CHCl_3$), bandes caractéristiques ($cm^{-1}$) 3400, 1790, 1730, 1690, 1505, 695.

Spectre de RMN du proton (350 MHz, $CDCl_3$, $\delta$ en ppm, J en Hz)

3,51 et 3,71 (2d, J = 18, 2H, —S—$CH_2$—);
3,88 (s, 2H, —$CH_2$CO—N<);
5,09 (d, J = 5, 1H, —H en 6); 5,94 (dd, J = 5 et 9, 1H, —H en 7);
6,43 (d, J = 9, 1H, —CO—NH—); 6,90 (s, 1H, —COO—C$\underline{H}$$(C_6H_5)_2$);
6,98 à 7,05 (mt, $H_3$ et $H_4$ du thiophène);
7,05 à 7,45 (mt, 11H, aromatique et $H_5$ du thiophène); 7,55 (s, 1H, $H_4$ du thiazole);
8,59 (s, 1H, $H_2$ du thiazole).

En opérant selon le mode opératoire décrit dans l'exemple 1 mais à partir de 2,55 g de benzhydryloxycarbonyl-2 oxo-8 (thiazolyl-5)-3 (thiényl-2 acétamido)-7 thia-5 aza-1 bicyclo[4.2.0] octène-2, on obtient 1,03 g de carboxy-2 oxo-8 (thiazolyl-5)-3 (thiényl-2 acétamido-7 thia-5 aza-1 bicyclo[4.2.0] octène-2 sous la forme d'un solide beige.

Spectre infra-rouge (KBr), bandes caractéristiques ($cm^{-1}$) 3260, 3150—2200, 1780, 1655, 1540, 1220, 700.

Spectre de RMN du proton (350 MHz, DMSO $d_6$, $\delta$ en ppm, J en Hz)

3,80 et 3,87 (2 AB limite, 4H, —$CH_2$—CO—N< et —$CH_2$—S—);
5,20 (d, J = 4,5, 1H, —H en 6); 5,76 (dd, J = 9 et 4,5, 1H, —H en 7);
6,90 à 7 (mt, 2H, $H_3$ et $H_4$ du thiophène); 7,35 (dd, J = 5 et 1, 1H, H en 5 du thiophène);
7,91 (s, 1H, $H_4$ du thiazole); 9,09 (s, 1H, H en 2 du thiazole); 9,19 (d, J = 9, 1H, —CO—NH—);
13,56 (mf, 1H, —COOH).

Une solution de 2,34 g de benzhydryloxycarbonyl-2 (bromo-1 oxo-2 éthyl)-3 t.butoxycarbonylamino-7 oxo-8 thia-5 aza-1 bicyclo[4.2.0] octène-2 (mélange des bromoaldéhydes épimères) et de 0,3 g de thioformamide dans 23 $cm^3$ de tétrahydrofuranne sec est agitée pendant 3 heures à 20°C puis chauffée à 50°C pendant 90 minutes. Le mélange réactionnel est dilué par 160 $cm^3$ d'acétate d'éthyle et lavé par 100 $cm^3$ de solution saturée de bicarbonate de sodium puis par 100 $cm^3$ de solution demi-saturée de chlorure de sodium. Après séchage de la couche organique sur sulfate de magnésium et évaporation du solvant sous pression réduite (30 mm de mercure; 4 kPa) à 30°C, on soumet le résidu obtenu à une chromatographie sur une colonne (hauteur: 30 cm; diamètre: 2,5 cm) de gel de silice (0,4—0,06 cm) en éluant sous une pression de 0,8 bar (80 kPa) par un mélange de cyclohexane et

d'acétate d'éthyle 70—30 (en volumes) et en recueillant des fractions de 50 $cm^3$. Les fractions 16 à 25 contenant le produit pur sont réunies et concentrées à sec sous pression réduite (30 mm de mercure; 4 kPa) à 30°C et le résidu est trituré dans 50 $cm^3$ d'oxyde d'isopropyle. Après filtration et séchage, on obtient 0,85 g de benzhydryloxycarbonyl-2 t.butoxycarbonylamino-7 oxo-8 (thiazolyl-5)-3 thia-5 aza-1 bicyclo[4.2.0] octène-2 sous la forme d'un solide de couleur crème.

Spectre infra-rouge ($CHBr_3$), bandes caractéristiques ($cm^{-1}$) 3420, 1790, 1725, 1505, 1500, 1455, 1390, 1370, 870, 760, 740.

Spectre de RMN du proton (350 MHz, $CDCl_3$, $\delta$ en ppm, J en Hz)

1,49 (s, 9H, $-C(CH_3)_3$); 3,56 et 3,74 (2d, J = 18, 2H, $-S-CH_2$);
5,09 (d, J = 4,5, 1H, —H en 6); 5,32 (d, J = 9, 1H, —CO—NH—);
5,72 (dd, J = 9 et 4,5, 1H, —H en 7); 6,91 (s, 1H, $-COOC\underline{H}(C_6H_5)_2$);
7 à 7,4 (mt, 10H, aromatiques); 7,58 (s, 1H, $H_4$ du thiazole); 8,58 (s, 1H, $H_2$ du thiazole).

## Exemple 6

Une solution de 3,3 g d'amino-7 carboxy-2 oxo-8 (phényl-2 thiazolyl-5)-3 thia-5 aza-1 bicyclo[4.2.0] octène-2 dans un mélange de 35 $cm^3$ d'eau distillée et 24 $cm^3$ d'acétone contenant 2 g de bicarbonate de sodium est refroidie à —10°C puis traitée par une solution de 0,86 $cm^3$ de chlorure de (thiényl-2) acétyle dans 10 $cm^3$ d'acétone que l'on ajoute goutte à goutte en 8 minutes. Le mélange réactionnel est agité pendant 30 minutes à —10°C puis 3 heures à 20°C. L'acétone est évaporée sous pression réduite (30 mm de mercure; 4 kPa) à 30°C et l'on ajoute 150 $cm^3$ d'acétate d'éthyle et 100 $cm^3$ de solution saturée de bicarbonate de sodium. La couche aqueuse est extraite par 100 $cm^3$ d'acétate d'éthyle puis traitée par 1 g de noir décolorant et 5 g d'adjuvant de filtration (poudre de diatomées) et filtrée sur adjuvant de filtration en rinçant par 2 fois 50 $cm^3$ d'eau distillée. Les filtrats réunis sont acidifiés à pH = 2 par de l'acide chlorhydrique 4N. Le précipité est essoré, lavé par 50 $cm^3$ d'acétate d'éthyle puis trituré avex 100 $cm^3$ d'oxyde d'isopropyle et séché. On obtient 0,8 g de carboxy-2 oxo-8 (phényle-2 thiazolyl-5)-3 (thiényl-2 acétamido)-7 thia-5 aza-1 bicyclo[4.2.0] octène-2 sous la forme d'un solide de couleur crème.

Spectre infra-rouge (KBr), bandes caractéristiques ($cm^{-1}$) 3290, 2700—2200, 1780, 1730, 1690, 1530, 1450, 690.

Spectre de RMN du proton (350 MHz, DMSO $d_6$, $\delta$ en ppm, J en Hz)

3,74 et 3,82 (2d, J = 14, 2H, $-CH_2-CO-N<$);
3,86 et 3,96 (2D, J = 18, 2H, $-S-CH_2$); 5,23 (d, J = 4,5, 1H, —H en 6);
5,77 (dd, J = 4,5 et 9, 1H, —H en 7); 6,90 à 7 (mt, 2H, =CH—CH= du thiophène);
7,35 (dd, J = 4,5 et 1, 1H, =CH—S— du thiophène);
7,45 à 7,55 (mt, 3H, —H aromatiques du benzène en méta et en para du thiazole);
7,92 (d, J = 8, 2H, —H aromatiques du benzène en ortho du thiazole);
7,93 (s, 1H, —H du thiazole); 9,21 (d, J = 9, 1H, —CO—NH—).

On dissout 0,336 g de carboxy-2 oxo-8 (phényl-2 thiazolyl-5)-3 (thiényl-2 acétamido)-7 thia-5 aza-1 bicyclo[4.2.0] octène-2 dans une solution de 0,05 g de bicarbonate de sodium dans 10 $cm^3$ d'eau. Après lavage par 25 $cm^3$ d'acétate d'éthyle la phase aqueuse est filtrée puis lyophilisée. On obtient 0,32 g de sel de sodium du carboxy-2 oxo-8 (phényl-2 thiazolyl-5)-3 (thiényl-2 acétamido)-7 thia-5 aza-1 bicyclo[4.2.0] octène-2 sous la forme d'un lyophilisat beige.

Spectre infra-rouge (KBr), bandes caractéristiques ($cm^{-1}$) 3280, 1760, 1665, 1605, 1545, 1495, 1450, 1395, 690.

Spectre de RMN du proton (DMSO $d_6$, $\delta$ en ppm, J en Hz)

3,68 et 3,81 (2d, J = 16, 2H, $-SCH_2-$); 3,77 (s, 2H, $-CH_2CO-$); 5,08 (d, J = 5, 1H, H en 6);
5,54 (dd, J = 5 et 8, 1H, H en 7); 6,9 à 7 (mt, 2H, $H_3$ et $H_4$ du thiényle);
7,36 (dd, J = 1 et 5, 1H, $H_5$ du thiényle); 7,4 à 7,5 (mt, 3H, $H_3$, $H_4$ et du phényle);
7,85 (d, J = 7,5, 2H, $H_2$ et $H_6$ du phényle); 7,90 (s, 1H, H en 4 du thiazole);
9,15 (d, J = 9, 1H, —CONH—).

L'amino-7 carboxy-2 oxo-8 (phényl-2 thiazolyl-5)-3 thia-5 aza-1 bicyclo[4.2.0] octène-2 peut être obtenu de la manière suivante:

On traite 5,7 g de benzhydryloxycarbonyl-2 t.butoxycarbonylamino-7 oxo-8 (phényl-2 thiazolyl-5)-3 thia-5 aza-1 bicyclo[4.2.0] octène-2 par 57 $cm^3$ d'acide trifluoroacétique pendant 20 minutes à 20°C puis concentre le mélange réactionnel à sec sous pression réduite (0,2 mm de mercure; 0,03 kPa) à 30°C. Le résidu est repris par 100 $cm^3$ d'acétate d'éthyle que l'on évapore sous pression résuite (30 mm de mercure; 4 kPa) à 30°C. Le résidu est trituré avec 100 $cm^3$ d'oxyde d'isopropyle. Le solide est essoré, lavé par 3 fois 50 $cm^3$ d'oxyde d'isopropyle et séché sous pression réduite (0,2 mm de mercure; 0,03 kPa) à 20°C. On obtient 3,3 g d'amino-7 carboxy-2 oxo-8 (phényl-2 thiazolyl-5)-3 thia-5

aza-1 bicyclo[4.2.0] octène-2 sous la forme d'un solide ocre.

Spectre infra-rouge (KBr), bandes caractéristiques ($cm^{-1}$) 3300 à 2000, 1785, 1615, 1400, 760, 690.

Spectre de RMN du proton (350 MHz, DMSO $d_6$, $\delta$ en ppm, J en Hz)

3,82 et 3,92 (d, J = 18, 2H, $-S-CH_2-$); 4,92 (d, J = 4,5, 1H, –H en 7);
5,13 (d, J = 4,5, 1H, –H en 6);
7,40 à 7,60 (mt, –H aromatiques du benzène en méta et en para du thiazole);
7,85 à 8 (mt, –H aromatiques du benzène en ortho du thiazole et –H thiazole).

On agite à 20°C pendant 1 heure 20 minutes une solution de 5,87 g de benzhydryloxycarbonyl-2 (bromo-1 oxo-2 éthyl)-3 t.butoxycarbonylamino-7 oxo-8 thia-5 aza-1 bicyclo[4.2.0] octène-2 (mélange des épimères du bromoaldéhyde) et de 1,5 g de thiobenzamide dans 60 $cm^3$ de tétrahydrofuranne sec puis on ajoute au mélange réactionnel refroidi à 2°C 0,85 $cm^3$ de chlorure de méthanesulfonyle puis en 5 minutes 5,6 $cm^3$ de triéthylamine. Après 2 heures d'agitation à 2°C le mélange réactionnel est versé dans un mélange de 100 $cm^3$ d'acétate d'éthyle et de 50 $cm^3$ d'acide chlorhydrique 1 N. La phase organique est lavée par 100 $cm^3$ de solution saturée de bicarbonate de sodium puis par 100 $cm^3$ de solution saturée de chlorure de sodium, séché sur sulfate de magnésium et concentrée à sec sous pression réduite (30 mm de mercure; 4 kPa) à 30°C. Le résidu est chromatographié sur une colonne (hauteur: 25 cm; diamètre: 5 cm) de gel de silice (0,04–0,06 mm) en éluant sous une pression de 0,4 bar (40 kPa) par un mélange de cyclohexane et d'acétate d'éthyle 80–20 (en volumes) et en recueillant des fractions des 125 $cm^3$. Les fractions 17 à 22 contenant le produit pur sont réunies et concentrées à sec sous pression réduite (30 mm de mercure; 4 kPa) à 30°C. On obtient 1 g de benzhydryloxycarbonyl-2 t.butoxycarbonylamino-7 oxo-8 (phényl-2 thiazolyl-5)-3 thia-5 aza-1 bicyclo[4.2.0] octène-2 sous la forme d'une meringue jaune.

Spectre infra-rouge ($CHBr_3$), bandes caractéristiques ($cm^{-1}$) 3420, 1790, 1720, 1595, 1495, 1450, 1500, 1420, 1390, 1370, 1235, 760, 740.

Spectre de RMN du proton (350 MHz, $CDCl_3$, $\delta$ en ppm, J en Hz)

1,48 (s, 9H, $-C(CH_3)_3$); 3,62 et 3,78 (2d, J = 18, 2H, $-CH_2-S-$);
5,10 (d, J = 5, 1H, –H en 6); 5,29 (d, J = 9, 1H, –CO–NH–);
5,73 (dd, J = 5 et 9, 1H, –H en 7); 6,96 (s, 1H, $-COO\underline{C}\underline{H}(C_6H_5)_2$);
7 à 7,5 (mt, aromatiques); 7,54 (s, 1H, –H thiazole);
7,77 (mt, 2H, –H aromatiques du phényle en ortho du thiazole).

## Exemple 7

En traitant 3,4 g d'[(acétamido-4 phényle)-2 thiazolyl-5]-3 benzhydryloxycarbonyl-2 t.butoxycarbonylamino-7 oxo-8 thia-5 aza-1 bicyclo[4.2.0] octène-2 par 3,4 $cm^3$ d'acide méthanesulfonique dans 15 $cm^3$ d'acétonitrile selon le mode opératoire décrit dans l'exemple 15 on obtient 2,9 g d'amino-7 [(acétamido-4 phényl)-2 thiazolyl-5]-3 benzhydryloxycarbonyl-2 oxo-8 thia-5 aza-1 bicyclo[4.2.0] octène-2 brut que l'on acyle par 0,62 $cm^3$ de chlorure de (thiényl-2) acétyle selon le mode opératoire décrit dans l'exemple 1. Le produit brut obtenu est purifié par cristallisation dans 200 $cm^3$ d'acétonitrile. On obtient 1,33 g d'[(acétamido-4 phényl)-2 thiazolyl-5]-3 benzhydryloxycarbonyl-2 oxo-8 [(thiényl-2) acétamido]-7 thia-5 aza-1 bicyclo[4.2.0] octène-2 sous la forme d'une poudre cristalline beige.

Spectre infra-rouge (KBr), bandes caractéristiques ($cm^{-1}$) 3280, 1785, 1730, 1680, 1600, 1530, 1230, 850, 760, 750, 700.

Spectre de RMN du proton (350 MHz, $CDCl_3$, $\delta$ en ppm, J en Hz)

2,25 $\left(s, 3H, >N-CO-CH_3\right)$;

3,56 et 3,72 (2d, J = 18, 2H, $-S-CH_2-$);

3,89 $\left(s, 2H, hét-CH_2-CON<\right)$;

5,09 (d, J = 4,5, 1H, –H en 6); 5,94 (dd, J = 9 et 4,5, 1H, –H en 7);
6,36 (d, J = 9, 1H, –CONH–); 6,94 (s, 1H, $-COO-C\underline{H}(C_6H_5)_2$);
7 à 7,45 (mt, aromatiques, –H du thiophène); 7,47 (s, 1H, –H du thiazole);
7,58 (d, J = 7,5, 2H, –H aromatiques en méta du thiazole);
7,70 (d, J = 7,5, 2H, –H aromatiques en ortho du thiazole).

Selon le mode opératoire décrit dans l'exemple 4 on traite 1,5 g d'[(acétamido-4 phényl)-2 thiazolyl-5]-3 benzhydryloxycarbonyl-2 oxo-8 [(thiényl-2) acétamido]-7 thia-5 aza-1 bicyclo[4.2.0] octène-2 par 25 $cm^3$ d'acide formique et 5 $cm^3$ d'anisole à 50°C pendant 20 minutes. On obtient 0,99 g d'[(acétamido-4 phényl)-2 thiazolyl-5]-3 carboxy-2 oxo-8 [(thiényl-2) acétamido]-7 thia-5 aza-1 bicyclo[4.2.0] octène-2 sous la forme d'un solide jaune.

Spectre infra-rouge (KBr), bandes caractéristiques ($cm^{-1}$) 3400, 3320, 2650—1850, 1790, 1695, 1670, 1600, 1540, 840, 705.

Spectre de RMN du proton (350 MHz, DMSO $d_6$, $\delta$ en ppm, J en Hz)

2,09 (s, 3H, $>N-CO-CH_3$);

3,74 et 3,82 (2d, J = 14, 2H, hét$-CH_2-CO-N<$);

3,86 et 3,96 (2d, J = 18, 2H, $-S-CH_2-$); 5,21 (d, J = 4,5, 1H, —H en 6);
5,76 (dd, J = 8 et 4,5, 1H, —H en 7); 6,94 à 7 (mt, 2H, =CH—CH= du thiophène);
7,37 (dd, J = 4,5 et 1, 1H, =CH—S— du thiophène);
7,71 (d, J = 8, 2H, —H aromatiques en méta du thiazole);
7,82 (d, J = 8, 2H, —H aromatiques en ortho du thiazole); 7,88 (s, 1H, —H du thiazole);
9,22 (d, J = 8, 1H, —NH—CO— en 7); 10,20 (s, 1H, Ar—NH—CO—).

Selon le mode opératoire décrit dans l'exemple 4, on traite 0,97 g de cet acide par 0,15 g de bicarbonate de sodium dans 30 $cm^3$ d'eau et on chromatographie la solution obtenue sur une colonne de 60 $cm^3$ de résine DUOLITE S 861 (préparée comme dans l'exemple 4) en éluant par un mélange d'eau et d'éthanol (90—10 en volumes) et en recueillant des fractions de 15 $cm^3$. On réunit et lyophilise les fractions 3 à 32. On obtient 0,34 g de sel de sodium de l'[(acétamido-4 phényl)-2 thiazolyl-5]-3 carboxy-2 oxo-8 [(thiényl-2) acétamido]-7 thia-5 aza-1 bicyclo[4.2.0] octène-2 sous forme d'un lyophilisat jaune clair.

Spectre de RMN du proton (350 MHz, DMSO $d_6$, $\delta$ en ppm, J en Hz)

2,07 (s, 3H, $>N-CO-CH_3$);

3,69 et 3,78 (2d, J = 18, 2H, $-S-CH_2-$);

3,75 et 3,81 (2d, J = 14, 2H, hét$-CH_2-CO-N<$);

5,07 (d, J = 5, 1H, —H en 6); 5,52 (dd, J = 9 et 5, 1H, —H en 7);
6,93 à 6,97 (mt, 2H, =CH—CH= du thiophène);
7,36 (dd, J = 5 et 1, 1H, =CH—S— du thiophène);
7,71 (d, J = 8, 2H, —H aromatiques en méta du thiazole);
7,77 (d, J = 8, 2H, —H aromatiques en ortho du thiazole); 7,85 (s, 1H, —H du thiazole);
9,11 (s, 1H, —CO—NH—); 10,5 (s, 1H, Ar—NH—CO—).

L'[(acétamido-4 phényl)-2 thiazolyl-5]-3 benzhydryloxycarbonyl-2 t.butoxycarbonylamino-7 oxo-8 thia-5 aza-1 bicyclo[4.2.0] octène-2 peut être préparé de la manière suivante:

Selon le mode opératoire de l'exemple 6 on traite 8,16 g de mélange des épimères du benzhydryloxycarbonyl-2 (bromo-1 oxo-2 éthyl)-3 t.butoxycarbonylamino-7 oxo-8 thia-5 aza-1 bicyclo[4.2.0] octène-2 par 2,7 g de paraacétamidothiobenzamide préparé selon la méthode de L. STEPHENSON, W. K. WARBURTON et M. J. WILSON, J. Chem. Soc. (C), 861 (1969) dans 50 $cm^3$ de tétrahydrofuranne sec puis par 1,18 $cm^3$ de chlorure de méthanesulfonyle et 4,2 $cm^3$ de triéthylamine. Le produit brut est chromatographié sur une colonne (hauteur: 35 cm, diamètre: 6 cm) de gel de silice (0,04—0,06 mm) en éluant sous une pression de 0,5 bar (50 kPa) par un mélange de cyclohexane et d'acétate d'éthyle (40—60 en volumes) et en recueillant des fractions de 100 $cm^3$. Les fractions 28 à 36 contenant le produit pur sont réunies et concentrées à sec sous pression réduite (30 mm de mercure; 4 kPa) à 30°C. On obtient 3,43 g de meringue jaune constituée principalement d'[(acétamido-4 phényl)-2 thiazolyl-5]-3 benzhydryloxycarbonyl-2 t.butoxycarbonylamino-7 oxo-8 thia-5 aza-1 bicyclo[4.2.0] octène-2.

Spectre de RMN du proton (350 MHz, $CDCl_3$, $\delta$ en ppm, J en Hz)

1,47 (s, 9H, $-C(CH_3)_3$);

2,22 (s, 3H, $>N-CO-CH_3$);

3,61 et 3,75 (2d, J = 18, 2H, $-CH_2-S-$); 5,09 (d, J = 5, 1H, —H en 6);
5,29 (d, J = 9, 1H, —CO—NH—); 5,73 (dd, J = 9 et 5, 1H, —H en 7);
6,95 (s, 1H, $-COO-\underline{CH}(C_6H_5)_2$); 7 à 7,45 (mt, aromatiques); 7,49 (s, 1H, —H du thiazole);
7,59 (d, J = 8, 2H, —H aromatiques en méta du thiazole);
7,70 (d, J = 8, 2H, —H aromatiques en ortho du thiazole).

Exemple 8

On traite 4 g de benzhydryloxycarbonyl-2 t.butoxycarbonylamino-7 (méthylthio-2 thiazolyl-5)-3 oxo-8 thia-5 aza-1 bicyclo[4.2.0] octène-2 en solution dans 40 $cm^3$ d'acétonitrile à 25°C par 4 $cm^3$ d'acide méthanesulfonique selon le mode opératoire de l'exemple 15. On obtient 3,3 g d'amino-7 benzhydryloxycarbonyl-2 (méthylthio-2 thiazolyl-5)-3 oxo-8 thia-5 aza-1 bicyclo[4.2.0] octène-2 brut sous la forme d'une huile orangée.

Spectre infra-rouge ($CHBr_3$), bandes caractéristiques ($cm^{-1}$) 3400, 3320, 1780, 1720, 1495, 1450, 1220, 760, 755.

On dissout 3,3 g d'amino-7 benzhydryloxycarbonyl-2 (méthylthio-2 thiazolyl-5)-3 oxo-8 thia-5 aza-1 bicyclo[4.2.0] octène-2 octène-2 brut dans 30 $cm^3$ de tétrahydrofuranne pour effecteur l'acylation par 0,83 $cm^3$ de chlorure de (thiényl-2) acétyle en présence de 0,94 $cm^3$ de triéthylamine selon le mode opératoire décrit dans l'exemple 1. Le produit brut obtenu est chromatographié sur une colonne (hauteur: 30 cm; diamètre: 4 cm) de gel de silice (0,04–0,06 mm) en éluant, sous une pression de 1 bar (100 kPa), par un mélange de cyclohexane et d'acétate d'éthyle 70–30 (en volumes) et en recueillant des fractions de 50 $cm^3$. Les fractions 16 à 27 contenant le produit pur sont rasemblées et concentrées à sec sous pression réduite (30 mm de mercure; 4 kPa) à 30°C et le résidu est trituré avec 30 $cm^3$ d'oxyde d'isopropyle. On obtient 1,6 g de benzhydryloxycarbonyl-2 (méthylthio-2 thiazolyl-5)-3 oxo-8 (thiényl-2 acétamido)-7 thia-5 aza-1 bicyclo[4.2.0] octène-2 sous la forme d'un solide jaune.

Spectre infra-rouge ($CHBr_3$), bandes caractéristiques ($cm^{-1}$) 3400, 1785, 1720, 1680, 1620, 1505, 1450, 1430, 1220, 955, 740.

Spectre de RMN du proton (350 MHz, $CDCl_3$, $\delta$ en ppm, J en Hz)

2,56 (s, 3H, $-S-CH_3$); 4,46 et 3,65 (2d, J = 18, 2H, $-S-CH_2$);
3,86 $\left(s, 2H, -CH_2-CO-N\langle\right)$;
5,05 (d, J = 5, 1H, –H en 6); 5,90 (dd, J = 5 et 9, 1H, –H en 7);
6,45 (d, J = 9, 1H, –CO–NH–); 6,93 (s, 1H, $-COO-\underline{CH}(C_6H_5)_2$);
6,98 à 7,05 (mt, 2H, =CH–CH= du thiophène);
7,10 à 7,5 (mt, 2H, aromatiques, –H du thiazole, =CH–S– du thiophène).

A une solution de 1,61 g de benzhydryloxycarbonyl-2 (méthylthio-2 thiazolyl-5)-3 oxo-8 (thiényl-2 acétamido)-7 thia-5 aza-1 bicyclo[4.2.0] octène-2 dans 40 $cm^3$ de chlorure de méthylène sec contenant 4 $cm^3$ d'anisole et refroidie à –8°C on ajoute en 10 minutes une solution de 1,04 g de chlorure d'aluminium dans 25 $cm^3$ de nitrométhane et agite le mélange réactionnel pendant 2 heures à une température comprise entre –8°C et 0°C. Le mélange réactionnel est dilué par 200 $cm^3$ d'acétate d'éthyle et 200 $cm^3$ d'eau distillée acidifiée par 3 gouttes d'acide chlorhydrique 4N. Après filtration pour éliminer un insoluble, on décante la couche organique qui est lavée par 50 $cm^3$ d'eau distillée puis extraite par 80 $cm^3$ de solution à 5% de bicarbonate de sodium. La solution aqueuse est lavée par 50 $cm^3$ d'acétate d'éthyle puis acidifiée à 0°C à pH 3 par de l'acide chlorhydrique 4N.

Le précipité est essoré, lavé par 2 fois 20 $cm^3$ d'eau et séché. On obtient 0,8 g de carboxy-2 (méthylthio-2 thiazolyl-5)-3 oxo-8 (thiényl-2 acétamido)-7 thia-5 aza-1 bicyclo[4.2.0] octène-2 sous la forme d'un solide blanc.

Spectre infra-rouge (KBr), bandes caractéristiques ($cm^{-1}$) 3270, 3120–2200, 1780, 1725, 1650, 1540, 705.

Spectre de RMN du proton (350 MHz, DMSO $d_6$, $\delta$ en ppm, J en Hz)

2,67 (s, 3H, $-S-CH_3$);
3,75 $\left(AB\ limite, 2H, -CH_2-CO-N\langle\right)$;
3,81 (AB limite, 2H, $-S-CH_2-$); 5,18 (d, J = 5, 1H, –H en 6);
5,73 (dd, J = 9 et 5, 1H, –H en 7); 6,90 à 7 (mt, 2H, =CH–CH= du thiophène);
7,35 (dd, J = 4 et 1, 1H, =CH–S– du thiophène); 7,7 (s, 1H, –H du thiazole);
9,18 (d, J = 9, 1H, –CO–NH–).

Le benzhydryloxycarbonyl-2 t.butoxycarbonylamino-7 (méthylthio-2 thiazolyl-5)-3 oxo-8 thia-5 aza-1 bicyclo[4.2.0] octène-2 peut être préparé de la manière suivante:

On porte à la température de reflux une solution de 9 g de dithiocarbamate de méthyle dans 250 $cm^3$ de tétrahydrofuranne puis ajoute une solution de 44 g de benzhydryloxycarbonyl-2 (bromo-1 oxo-2 éthyl)-3 t.butoxycarbonylamino-7 oxo-8 thia-5 aza-1 bicyclo[4.2.0] octène-2 dans 100 $cm^3$ d'éthanol. Le mélange réactionnel est chauffé pendant 65 minutes à 66°C puis dilué par 2 litres d'eau et 1 litre d'acétate d'éthyle. La phase aqueuse est décantée et lavée par 250 $cm^3$ d'acétate d'éthyle. Les phases organiques rassemblées sont lavées par 1 litre de solution demi-saturée de chlorure de sodium puis

séchées sur sulfate de sodium et concentrées à sec sous pression réduite (30 mm de mercure; 4 kPa) à 30°C. Le résidu est chromatographié sur une colonne (diamètre: 8 cm) de 1 litre de gel de silice (0,2—0,06 mm) en recueillant des fractions de 500 $cm^3$. On élue successivement par 3,5 litre de chlorure de méthylène puis par 8 litre de mélange de chlorure de méthylène et d'acétate d'éthyle 90—10 (en volumes). Les fractions 14 à 16 contenant le produit pur sont réunies et concentrées à sec sous pression réduite (30 mm de mercure; 4 kPa) à 30°C. Le résidu est trituré avec de l'oxyde d'isopropyle puis filtré et séché; on obtient 7,3 g de benzhydryloxycarbonyl-2 t.butoxycarbonylamino-7 (méthylthio-2 thiazolyl-5)-3 oxo-8 thia-5 aza-1 bicyclo[4.2.0] octène-2 sous la forme d'un solide de couleur crème.

Spectre infra-rouge ($CHCl_3$), bandes caractéristiques ($cm^{-1}$) 3440, 1790, 1720, 1505, 1455, 1390, 1370, 1155, 695.

Spectre de RMN du proton (350 MHz, $CDCl_3$, $\delta$ en ppm, J en Hz)

1,47 (s, 9H, $-C(CH_3)_3$); 2,56 (s, 3H, $-S-CH_3$); 3,50 et 3,68 (2d, J = 18, 2H, $-S-CH_2-$);
5,06 (d, J = 5, 1H, —H en 6); 5,30 (d, J = 9, 1H, —CO—NH—);
5,71 (dd, J = 9 et 5, 1H, —H en 7); 6,96 (s, 1H, $-COO-\underline{C}\underline{H}(C_6H_5)_2$);
7 à 7,60 (mt, 11H, aromatiques et —H thiazole).

Exemple 9

En opérant selon le mode opératoire décrit dans l'exemple 15 on effectue le déblocage de 5 g de benzhydryloxycarbonyl-2 t.butoxycarbonylamino-7 (diméthylamino-2 thiazolyl-5)-3 oxo-8 thia-5 aza-1 bicyclo[4.2.0] octène-2 par 5 $cm^3$ d'acide méthanesulfonique dans 50 $cm^3$ d'acétonitrile, suivi de l'acylation de l'amino-7 benzhydryloxycarbonyl-2 (diméthylamino-2 thiazolyl-5)-3 oxo-8 thia-5 aza-1 bicyclo[4.2.0] octène-2 brut obtenu (Rf = 0,32; chromatoplaque de gel de silice; éluant: acétate d'éthyle) par 1,04 $cm^3$ de chlorure de (thiényl-2) acétyle dans 40 $cm^3$ de tétrahydrofuranne en présence de 1,18 $cm^3$ de triéthylamine puis de la chromatographie sur une colonne (hauteur: 30 cm; diamètre: 5 cm) de gel de silice (0,04—0,06 mm) en éluant sous une pression de 0,8 bar (80 kPa) par des mélanges de cyclohexane et d'acétate d'éthyle (successivement 3 litres 50—50 en volumes et 2 litres 40—60 en volumes) en recueillant des fractions de 100 $cm^3$ et évaporant à sec les fractions 33 à 44 sous pression réduite (30 mm de mercure; 4 kPa) à 30°C. On obtient 2,4 g de benzhydryloxycarbonyl-2 (diméthylamino-2 thiazolyl-5)-3 oxo-8 (thiényl-2 acétamido)-7 thia-5 aza-1 bicyclo[4.2.0] octène-2 sous la forme d'un solide jaune.

Spectre infra-rouge ($CHBr_3$), bandes caractéristiques ($cm^{-1}$) 3400, 3330, 1780, 1720, 1680, 1565, 1510, 1475, 1455, 1420, 1410, 755, 740.

Spectre de RMN du proton (350 MHz, $CDCl_3$, $\delta$ en ppm, J en Hz)

2,95 (s, 6H, $-N(CH_3)_2$); 3,47 et 3,54 (2d, J = 18, 2H, $-CH_2-S-$);
3,95 (s, 2H, $-CH_2-CO-N<$);
5,03 (d, J = 4,5, 1H, —H en 6); 5,82 (dd, J = 9 et 4,5, 1H, —H en 7);
6,54 (d, J = 9, 1H, —CO—NH—); 6,94 (s, 1H, $-COO-\underline{C}\underline{H}(C_6H_5)_2$);
6,95 à 7,05 (mt, 2H, =CH—CH= du thiophène); 7,03 (s, 1H, —H thiazole);
7,10 à 7,40 (mt, 11H, aromatiques et =CH—S— du thiophène).

On traite 2,4 g de benzhydryloxycarbonyl-2 (diméthylamino-2 thiazolyl-5)-3 oxo-8 (thiényl-2 acétamido)-7 thia-5 aza-1 bicyclo[4.2.0] octène-2 par 25 $cm^3$ d'acide formique selon le mode opératoire décrit dans l'exemple 17 et obtient 1,05 g de carboxy-2 (diméthylamino-2 thiazolyl-5)-3 oxo-8 (thiényl-2 acétamido)-7 thia-5 aza-1 bicyclo[4.2.0] octène-2 à l'état de sel interne, sous la forme d'un solide jaune.

Spectre infra-rouge (KBr), bandes caractéristiques ($cm^{-1}$) 3280, 3100—2200, 1775, 1695, 1665, 1630, 1560, 1535, 1415, 1240, 700.

Spectre de RMN du proton (350 MHz, DMSO $d_6$, $\delta$ en ppm, J en Hz)

3,04 (s, 6H, $-N(CH_3)_2$); 3,70 et 3,81 (2d, J = 18, 2H, $-CH_2-S-$);
3,78 (s, 2H, $-CH_2-CO-N<$);
5,13 (d, J = 4,5, 1H, —H en 6); 5,63 (dd, J = 4,5 et 8,5, 1H, —H en 7);
6,90 à 7 (mt, 2H, =CH—CH= du thiophène); 7,30 (s, 1H, —H du thiazole);
7,35 (dd, J = 4 et 1, 1H, =CH—S— du thiophène); 9,14 (d, J = 8,5, 1H, —CO—NH—).

On opère selon le mode opératoire de l'exemple 2 mais en remplaçant la thiourée par 5,25 g de N,N-diméthylthiourée et à partir de 21,4 g de benzhydryloxycarbonyl-2 t.butoxycarbonylamino-7 (diméthylamino-2 vinyl)-3 oxo-8 thia-5 aza-1 bicyclo[4.2.0] octène-2 (forme E). On chromatographie

le produit obtenu sur une colonne (diamètre: 4,5 cm) contenant 500 $cm^3$ de gel de silice (0,2—0,06 mm) en éluant par 5 litres de chlorure de méthylène puis par des mélanges de chlorure de méthylène et d'acétate d'éthyle 99—1 (en volumes, 5 litres) puis 97—3 (en volumes, 7,5 litres) et en recueillant des fractions de 500 $cm^3$, et on concentre à sec les fractions 13 à 34 sous pression réduite (30 mm de mercure; 4 kPa) à 30°C. On triture le résidu avec de l'oxyde d'isopropyle (100 $cm^3$) et obtient 13,8 g de benzhydryloxycarbonyl-2 t.butoxycarbonylamino-7 (diméthylamino-2 thiazolyl-5)-3 oxo-8 thia-5 aza-1 bicyclo[4.2.0] octène-2 sous la forme d'un solide jaune.

Spectre infra-rouge ($CHBr_3$), bandes caractéristiques ($cm^{-1}$) 3420, 1780, 1720, 1555, 1500, 1455, 1420, 1390, 1370, 1235, 760, 740.

Spectre de RMN du proton (350 MHz, $CDCl_3$, $\delta$ en ppm, J en Hz)

1,48 (s, 9H, $-C(CH_3)_3$); 2,95 (s, 6H, $-N(CH_3)_2$); 3,56 et 3,67 (2d, J = 18, 2H,. $-CH_2-S-$);
5,05 (d, J = 4,5, 1H, —H en 6); 5,27 (d, J = 9, 1H, —CO—NH—);
5,64 (dd, J = 4,5 et 9, 1H, —H en 7); 6,97 (s, 1H, $-COO-\underline{CH}(C_6H_5)_2$); 7,05 (s, 1H, —H thiazole);
7,15 à 7,45 (mt, 10H, aromatiques).

Exemple 10

On traite 2,3 g d'(anilino-2 thiazolyl-5)-3 benzhydryloxycarbonyl-2 t.butoxycarbonylamino-7 oxo-8 thia-5 aza-1 bicyclo[4.2.0] octène-2 par 2,3 $cm^3$ d'acide méthanesulfonique dans 23 $cm^3$ d'acétonitrile selon le mode opératoire décrit dans l'exemple 2. On obtient 1,85 g d'amino-7 (anilino-2 thiazolyl-5)-3 benzhydryloxycarbonyl-2 oxo-8 thia-5 aza-1 bicyclo[4.2.0] octène-2 brut que l'on acyle par 0,445 $cm^3$ de chlorure de (thiényl-2) acétyle dans 25 $cm^3$ de tétrahydrofuranne en présence de 0,505 $cm^3$ de triéthylamine en opérant selon le mode opératoire décrit dans l'exemple 1. Le produit brut obtenu est chromatographié sur une colonne (hauteur: 30 cm; diamètre: 2,5 cm) de gel de silice (0,04—0,06 mm) en éluant sous une pression de 1,2 bar (120 kPa) par 3 litres de mélange de cyclohexane et d'acétate d'éthyle 70—30 (en volumes) en recueillant des fractions de 50 $cm^3$. Les fractions 20 à 50 sont réunies et concentrées à sec. Après trituration avec de l'éther isopropylique on obtient 0,9 g d'(anilino-2 thiazolyl-5)-3 benzhydryloxycarbonyl-2 oxo-8 (thiényl-2 acétamido)-7 thia-5 aza-1 bicyclo[4.2.0] octène-2 sous la forme d'un solide jaune.

Spectre infra-rouge (KBr), bandes caractéristiques ($cm^{-1}$) 3380, 1785, 1720, 1675, 1600, 1530, 1500, 1455, 750, 700, 620, 605.

Spectre de RMN du proton (350 MHz, $CDCl_3$, $\delta$ en ppm, J en Hz)

3,51 (AB limite, 2H, $-S-CH_2-$);
3,84 (s, 2H, $-CH_2-CO-N\langle$);
5,04 (d, J = 4,5, 1H, —H en 6); 5,84 (dd, J = 4,5 et 9, 1H, —H en 7);
6,92 (s, 1H, $-COO-\underline{CH}(C_6H_5)_2$); 6,95 à 7,03 (mt, 2H, =CH—CH= du thiophène);
7,05 à 7,40 (mt, 18H, aromatiques, —H thiazole, =CH—S— du thiophène et —CO—NH—);
8,65 (mf, 1H, —NH—).

On traite 0,9 g d'(anilino-2 thiazolyl-5)-3 benzhydryloxycarbonyl-2 oxo-8 (thiényl-2 acétamido)-7 thia-5 aza-1 bicyclo[4.2.0] octène-2 par 9 $cm^3$ d'acide formique selon le mode opératoire décrit dans l'exemple 1 et obtient 0,4 g d'(anilino-2 thiazolyl-5)-3 carboxy-2 oxo-8 (thiényl-2 acétamido)-7 thia-5 aza-1 bicyclo[4.2.0] octène-2 sous la forme d'un solide jaune.

Spectre infra-rouge (KBr), bandes caractéristiques ($cm^{-1}$) 3400, 3280, 3150—2000, 1770, 1665, 1625, 1605, 1530, 1500, 1455, 1400, 750, 700.

Spectre de RMN du proton (350 MHz, DMSO $d_6$, $\delta$ en ppm, J en Hz)

3,79 (AB limite, 2H, $-CH_2-CO-N\langle$);
3,76 et 3,87 (2d, J = 18, 2H, $-S-CH_2-$); 5,16 (d, J = 5, 1H, —H en 6);
5,68 (dd, J = 5 et 9, 1H, —H en 7); 6,85 à 7 (mt, 2H, =CH—CH= du thiophène);
7,20 à 7,4 (mt, 5H, —H aromatiques en méta et para de l'anilino, =CH—S— du thiophène et —H du thiazole);
7,58 (d, J = 7,5, 2H, —H aromatiques en ortho de l'anilino); 9,15 (d, J = 9, 1H, —CO—NH—);
10,27 (s large, 1H, —NH—).

A une solution de 10,3 g de benzhydryloxycarbonyl-2 t.butoxycarbonylamino-7 (chloro-1 oxo-2 éthyl)-3 oxo-8 thia-5 aza-1 bicyclo[4.2.0] octène-2 (mélange des épimères du chloroaldéhyde) dans un mélange de 100 $cm^3$ de tétrahydrofuranne et de 40 $cm^3$ d'éthanol on ajoute 3,34 g de N-phénylthiourée puis on chauffe à la température de reflux du mélange réactionnel pendant 135 minutes. On dilue par 250 $cm^3$ d'acétate d'éthyle et 500 $cm^3$ de solution demi-saturée de bicarbonate de sodium. La

couche organique est lavée par 250 $cm^3$ de solution demi-saturée de chlorure de sodium puis séchée sur sulfate de magnésium et concentrée à sec sous pression réduite (30 mm de mercure; 4 kPa) à 30°C.

Le résidu obtenu est chromatographié sur une colonne (hauteur: 30 cm; diamètre: 7 cm) de gel de silice (0,04—0,06 mm) en éluant sous une pression de 0,7 bar (70 kPa) par un mélange de cyclohexane et d'acétate d'éthyle 70—30 (en volumes). Après avoir recueilli 1,5 litre d'éluat on recueille des fractions de 100 $cm^3$, réunit les fractions 20 et 21 et concentre à sec sous pression réduite (30 mm de mercure; 4 kPa) à 30°C. Le résidu est trituré avec 30 $cm^3$ d'oxyde d'isopropyle. On obtient 2,7 g d'(anilino-2 thiazolyl-5)-3 benzhydryloxycarbonyl-2 t.butoxycarbonylamino-7 oxo-8 thia-5 aza-1 bicyclo[4.2.0] octène-2 sous la forme d'un solide jaune.

Spectre infra-rouge ($CHBr_3$), bandes caractéristiques ($cm^{-1}$) 3420, 1785, 1720, 1600, 1540, 1500, 1495, 1455, 1390, 1370, 750.

Spectre de RMN du proton (350 MHz, $CDCl_3$, $\delta$ en ppm, J en Hz)

1,47 (s, 9H, $-C(CH_3)_3$); 3,52 et 3,67 (2d, J = 18, 2H, $-S-CH_2-$); 5,03 (d, J = 4,5, 1H, —H en 6); 5,41 (d, J = 9, 1H, —CO—NH—); 5,66 (dd, J = 4,5 et 9, 1H, —H en 7); 6,95 (s, 1H, $-COO-\underline{CH}(C_6H_5)_2$); 7,05 (s, 1H, —H thiazole); 7,05 à 7,4 (mt, 15H, aromatiques); 8,91 (s large, 1H, —NH—).

En opérant de façon similaire à l'exemple 1 mais en traitant une solution de 21,4 g de benzhydryloxycarbonyl-2 t.butoxycarbonylamino-7 (diméthylamino-2 vinyl)-3 oxo-8 thia-5 aza-1 bicyclo[4.2.0] octène-2 (forme E) dans 100 $cm^3$ de tétrahydrofuranne sec à —60°C par 40 $cm^3$ d'une solution chlorométhylénique de chlore à 10% (poids/volume), on obtient 21,6 g de benzhydryloxycarbonyl-2 t.butoxycarbonylamino-7 (chloro-1 oxo-2 éthyl)-3 oxo-8 thia-5 aza-1 bicyclo[4.2.0] octène-2 (mélange des deux épimère du chloroaldéhyde).

Spectre infra-rouge ($CHBr_3$), bandes caractéristiques ($cm^{-1}$) 3420, 1785, 1720, 1505, 1450, 1390, 1365, 755, 740.

Spectre de RMN du proton (350 MHz, $CDCl_3$, $\delta$ en ppm, J en Hz)

épimère A

1,44 (s, 9H, $(CH_3)_3C-$); 3,46 et 3,60 (AB, J = 18, 2H, $-SCH_2-$); 5,06 (d, J = 5, 1H, H en 6); 5,24 (d, J = 9, 1H, —NH—); 5,67 (dd, J = 5 et 9, 1H, H en 7); 5,90 (s, 1H, —CHCl—); 6,96 (s, 1H, $-CHAr_2$); 7,20 à 7,60 (m, 10H, aromatiques); 9,38 (s, 1H, —CHO).

épimère B

1,44 (s, 9H, $(CH_3)_3C-$); 3,23 et 3,63 (AB, J = 18, 2H, $-SCH_2-$); 5,0 (d, J = 5, 1H, H en 6); 5,28 (d, J = 9, 1H, —NH—); 5,71 (dd, J = 5 et 9, 1H, H en 7); 5,94 (s, 1H, —CHCl—); 6,91 (s, 1H, $-CHAr_2$); 7,20 à 7,60 (m, 10H, aromatiques); 9,45 (s, 1H, —CHO).

## Exemple 11

A une solution de 6,2 g de benzhydryloxycarbonyl-2 t.butoxycarbonylamino-7 (méthoxycarbonylamino-2 thiazolyl-5)-3 oxo-8 thia-5 aza-1 bicyclo[4.2.0] octène-2 dans 60 $cm^3$ d'acétonitrile on ajoute 6 $cm^3$ d'acide méthanesulfonique. Après 15 minutes à 20°C le mélange réactionnel est dilué par 150 $cm^3$ de chlorure de méthylène et 200 $cm^3$ de solution demi-saturée de bicarbonate de sodium. La couche aqueuse est lavée par 3 fois 50 $cm^3$ de chlorure de méthylène et les phases organiques rassemblées sont lavée par 2 fois 100 $cm^3$ d'eau distillée, séchées sur sulfate de magnésium et concentrées à sec sous pression réduite (30 mm de mercure; 4 kPa) à 30°C. On obtient 5,2 g d'amino-7 benzhydryloxycarbonyl-2 (méthoxycarbonylamino-2 thiazolyl-5)-3 oxo-8 thia-5 aza-1 bicyclo[4.2.0] octène-2 brut que l'on redissout dans 50 $cm^3$ de tétrahydrofuranne sec. La solution obtenue est refroidie à 4°C et traitée par une solution de 1,23 $cm^3$ de chlorure de (thiényl-2) acétyle dans 10 $cm^3$ de tétrahydrofuranne sec puis par une solution de 1,4 $cm^3$ de triéthylamine dans 5 $cm^3$ de tétrahydrofuranne sec selon le mode opératoire décrit dans l'exemple 1. Le produit brut est chromatographié sur une colonne (hauteur: 32 cm; diamètre: 6 cm) de gel de silice (0,04—0,06 mm) en éluant sous une pression de 0,4 bar (40 kPa) par un mélange de cyclohexane et d'acétate d'éthyle 45—55 (en volumes) et en recueillant des fractions de 100 $cm^3$. Les fractions 8 à 21 contenant le produit pur sont rassemblées et concentrées à sec sous pression réduite (30 mm de mercure; 4 kPa) à 30°C. On obtient 1,91 g de benzhydryloxycarbonyl-2 (méthoxycarbonylamino-2 thiazolyl-5)-3 oxo-8 (thiényl-2 acétamido)-7 thia-5 aza-1 bicyclo[4.2.0] octène-2 sous la forme d'un solide blanchâtre.

Spectre infra-rouge ($CHBr_3$), bandes caractéristiques ($cm^{-1}$) 3400, 3335, 3100—2500, 1780, 1730, 1690, 1545, 1515, 1500, 1460, 1430, 1255, 1240, 705.

Spectre de RMN du proton (350 MHz, $CDCl_3$, $\delta$ en ppm, J en Hz)

3,35 et 3,63 (2d, J = 16, 2H, $-S-CH_2-$); 3,54 (s, 3H, $-COO-CH_3$); 3,85 (s, 2H, $-CH_2-CO-$); 5,00 (d, J = 4, 1H, –H en 6); 5,82 (dd, J = 4 et 9, 1H, –H en 7); 6,86 (s, 1H, $-COO-C\underline{H}(C_6H_5)_2$); 6,80 à 6,95 (mt, 2H, =CH–CH= thiophène); 7,18 (d, J = 4, =CH–S– thiophène); 7,20 à 7,60 (mt, aromatiques, –H thiazole, –CO–NH–);

12,98 $\left(\text{mf étalé, 1H, } -NH-\underset{\underset{O}{\|}}{C}-O-\right)$;

On traite 1,82 g de benzhydryloxycarbonyl-2 (méthoxycarbonylamino-2 thiazolyl-5)-3 oxo-8 (thiényl-2 acétamido)-7 thia-5 aza-1 bicyclo[4.2.0] octène-2 par 20 $cm^3$ d'acide formique selon le mode opératoire décrit dans l'exemple 1. On obtient 0,9 g de carboxy-2 (méthoxycarbonylamino-2 thiazolyl-5)-3 oxo-8 (thiényl-2 acétamido)-7 thia-5 aza-1 bicyclo[4.2.0] octène-2 sous la forme d'un solide jaune.

Spectre infra-rouge (KBr), bandes caractéristiques ($cm^{-1}$) 3400, 3260, 3100–2100, 1785, 1730, 1660, 1550, 1530, 1240, 705.

Spectre de RMN du proton (350 MHz, DMSO $d_6$, $\delta$ en ppm, J en Hz)

3,77 $\left(\text{s, 3H, } -\underset{\underset{O}{\|}}{C}-O-CH_3\right)$;

3,74 et 3,82 (2d, J = 14, 2H, $-CH_2-CO-$); 3,79 et 3,89 (2d, J = 16, 2H, $-CH_2-S-$); 5,17 (d, J = 4, 1H, –H en 6); 5,72 (dd, J = 4 et 9, 1H, –H en 7); 6,9 à 7,05 (mt, 2H, =CH–CH= thiophène); 7,34 (dd, J = 4 et 1, 1H, =CH–S– thiophène); 7,44 (s, 1H, –H thiazole); 9,17 (d, J = 9, 1H, –CO–NH–);

11,35 $\left(\text{mf, 1H, } -COOH \text{ ou } -NH-\underset{\underset{O}{\|}}{C}-O-\right)$;

13,52 $\left(\text{mf étalé, 1H, } -NH-\underset{\underset{O}{\|}}{C}-O- \text{ ou } -COOH\right)$.

On traite 1,13 g d'(amino-2 thiazolyl-5)-3 benzhydryloxycarbonyl-2 t.butoxycarbonylamino-7 oxo-8 thia-5 aza-1 bicyclo[4.2.0] octène-2 selon le mode opératoire décrit dans l'exemple 1 (B) en remplaçant le chlorure d'acétyle par 0,19 $cm^3$ de chloroformiate de méthyle. Le produit brut obtenu est chromatographié sur une colonne (hauteur: 30 cm; diamètre: 3 cm) de gel de silice (0,04–0,06 mm) en éluant sous une pression de 0,4 bar (40 kPa) par un mélange de cyclohexane et d'acétate d'éthyle 40–60 (en volumes) et en recueillant des fractions de 50 $cm^3$, qui sont analysées par chromatographie sur couche mince de gel de silice (éluant: mélange de cyclohexane et d'acétate d'éthyle 40–60 (en volumes). Les fractions contenant le produit attendu pur (Rf = 0,3) sont réunies et concentrées à sec sous pression réduite (30 mm de mercure; 4 kPa) à 30°C. On obtient 0,73 g de benzhydryloxycarbonyl-2 t.butoxycarbonylamino-7 (méthoxycarbonylamino-2 thiazolyl-5)-3 oxo-8 thia-5 aza-1 bicyclo[4.2.0] octène-2 sous la forme d'une meringue jaune.

Spectre infra-rouge ($CHBr_3$), bandes caractéristiques ($cm^{-1}$) 3420, 3100–2500, 1785, 1725, 1720, 1575, 1500, 1455, 1430, 1390, 1370, 1240, 760, 745, 605.

Spectre de RMN du proton (350 MHz, $CDCl_3$, $\delta$ en ppm, J en Hz)

1,48 (s, 9H, $-C(CH_3)_3$); 3,57 et 3,72 (2d, J = 18, 2H, $-S-CH_2-$);

3,86 $\left(\text{s, 3H, } -\underset{\underset{O}{\|}}{C}-O-CH_3\right)$;

5,08 (d, J = 5, 1H, –H en 6); 5,37 (d, J = 9, 1H, –CO–NH–); 5,71 (dd, J = 5 et 9, 1H –H en 7); 6,93 (s, 1H, $-COO-C\underline{H}(C_6H_5)_2$); 7,07 (s, –H thiazole); 7,05 à 7,40 (mt, aromatiques);

11,26 $\left(\text{mf, 1H, } -NH-\underset{\underset{O}{\|}}{C}-O-\right)$.

Exemple 12

Selon le mode opératoire décrit dans l'exemple 15, on traite 0,77 g de benzhydryloxycarbonyl-2 t.butoxycarbonylamino-7 (diméthylaminométhylèneamino-2 thiazolyl-5)-3 oxo-8 thia-5 aza-1 bicyclo[4.2.0] octène-2 (forme E) par 0,8 $cm^3$ d'acide méthanesulfonique dans 8 $cm^3$ d'acétonitrile. On obtient 0,65 g d'amino-7 benzhydryloxycarbonyl-2 (diméthylaminométhylèneamino-2 thiazolyl-5)-3 oxo-8 thia-5 aza-1 bicyclo[4.2.0] octène-2 (forme E) sous la forme d'une meringue brune brute (Rf = 0,1 chromatoplaque de gel de silice; éluant: acétate d'éthyle) que l'on redissout dans 15 $cm^3$ de tétrahydrofuranne et on l'acyle, selon le mode opératoire décrit dans l'exemple 1, par 0,15 $cm^3$ de chlorure de (thiényl-2) acétyle en présence de 0,17 $cm^3$ de triéthylamine. On chromatographie sur une colonne (hauteur: 28 cm; diamètre: 2 cm) de gel de silice (0,04—0,06 mm) en éluant sous une pression de 0,8 bar (80 kPa) par de l'acétate d'éthyle et en recueillant des fractions de 30 $cm^3$. Les fractions 11 à 19 contenante le produit pur sont réunies et concentrées à sec sous pression réduite (30 mm de mercure; 4 kPa) à 30°C. On obtient 0,13 g de benzhydryloxycarbonyl-2 (diméthylaminométhylèneamino-2 thiazolyl-5)-3 oxo-8 (thiényl-2 acétamido)-7 thia-5 aza-1 bicyclo[4.2.0] octène-2 (forme E) sous la forme d'une poudre jaune.

Spectre infra-rouge ($CHBr_3$), bandes caractéristiques ($cm^{-1}$) 3400, 1780, 1720, 1680, 1615, 1505, 1490, 1450.

Spectre de RMN du proton (350 MHz, $CDCl_3$, $\delta$ en ppm, J en Hz)

3,08 et 3,11 (2s, 6H, $-N(CH_3)_2$); 3,51 et 3,65 (2d, J = 18, 2H, $-CH_2-S-$);
3,86 (s, 2H, $-CH_2-CO-N<$);
5,04 (d, J = 4,5, 1H, –H en 6); 5,87 (dd, J = 9 et 4,5, 1H, –H en 7);
6,38 (d, J = 9, 1H, –CO–NH–); 6,93 (s, 1H, $-COOC\underline{H}(C_6H_5)_2$);
6,97 à 7,03 (mt, 2H, =CH–CH= du thiophène); 7,03 (s, 1H, –H du thiazole);
7,1 à 7,4 (mt, aromatiques);
7,95 (s, 1H, $-N=CH-N<$).

Selon le mode opératoire décrit dans l'exemple 1, on traite 0,1 g de benzhydryloxycarbonyl-2 (diméthylaminométhylèneamino-2 thiazolyl-5)-3 oxo-8 (thiényl-2 acétamido)-7 thia-5 aza-1 bicyclo[4.2.0] octène-2 par 15 $cm^3$ d'acide formique. On obtient 0,045 g de carboxy-2 (diméthylaminométhylèneamino-2 thiazolyl-5)-3 oxo-8 (thiényl-2 acétamido)-7 thia-5 aza-1 bicyclo[4.2.0] octène-2 sous la forme d'une poudre jaune.

Spectre infra-rouge (KBr), bandes caractéristiques ($cm^{-1}$) 3260, 3100—2000, 1770, 1680, 1630, 1530, 1390, 700.

Spectre de RMN du proton (350 MHz, DMSO $d_6$, $\delta$ en ppm, J en Hz)

2,97 et 3,12 (2s, 6H, $-N(CH_3)_2$);
3,65 à 3,85 (mf, 4H, $-CH_2-S-$ et $-CH_2-CO-N<$);
5,12 (d, J = 4,5, 1H, –H en 6); 5,67 (dd, J = 4,5 et 9, 1H, –H en 7);
6,9 à 7 (mt, 2H, =CH–CH= du thiophène);
7,3 à 7,45 (mt, 2H, =CH–S– du thiophène et –H du thiazole);
8,27 (s, 1H, $-N=CH-N<$);
9,15 (d, J = 9, 1H, –CO–NH–).

Le benzhydryloxycarbonyl-2 t.butoxycarbonylamino-7 (diméthylaminométhylèneamino-2 thiazolyl-5)-3 oxo-8 thia-5 aza-1 bicyclo[4.2.0] octène-2 peut être préparé selon l'une ou l'autre des méthodes suivantes:

A) On prépare une solution de benzhydryloxycarbonyl-2 (bromo-1 oxo-2 éthyl)-3 t.butoxycarbonylamino-7 oxo-8 thia-5 aza-1 bicyclo[4.2.0] octène-2 brut dans 50 $cm^3$ de tétrahydrofuranne, en traitant, selon le mode opératoire décrit dans l'exemple 2, 13,4 g de benzhydryloxycarbonyl-2 t.butoxycarbonylamino-7 (diméthylamino-2 vinyl)-3 oxo-8 thia-5 aza-1 bicyclo[4.2.0] octène-2 par 1,3 $cm^3$ de brome en solution dans 5 $cm^3$ de chlorure de méthylène puis par 0,9 $cm^3$ d'eau. On ajoute à −40°C une solution de 3,28 g de diméthylaminométhylènethiourée dans un mélange de 10 $cm^3$ d'eau distillée et de 50 $cm^3$ de tétrahydrofuranne puis agite le mélange réactionnel pendant 3 heures en laissant remonter la température jusqu'à 15°C. Le mélange réactionnel est dilué par 600 $cm^3$ d'acétate d'éthyle et lavé par 800 $cm^3$ d'eau additionnée de 100 $cm^3$ de solution saturée de bicarbonate de sodium, puis par 3 fois 200 $cm^3$ d'eau distillée et par 200 $cm^3$ de solution saturée de chlorure de sodium. Après séchage

sur sulfate de magnésium la phase organique est concentrée à sec sous pression réduite (30 mm de mercure; 4 kPa) à 30°C et le résidu est chromatographié sur une colonne (hauteur: 30 cm; diamètre: 5 cm) de gel de silice (0,04—0,06 mm) en éluant sous une pression de 0,6 bar (60 kPa) par de l'acétate d'éthyle et en recueillant des fractions de 70 $cm^3$. Les fractions 19 à 30 contenant le produit pur sont réunies et concentrées à sec sous pression réduite (30 mm de mercure; 4 kPa) à 30°C. On obtient 0,78 g de benzhydryloxycarbonyl-2 t.butoxycarbonylamino-7 (diméthylaminométhylèneamino-2 thiazolyl-5)-3 oxo-8 thia-5 aza-1 bicyclo[4.2.0] octène-2 sous la forme d'une meringue orangée.

Spectre infra-rouge ($CHCl_3$), bandes caractéristiques ($cm^{-1}$) 3440, 1770, 1725, 1625, 1500, 1460, 1375, 1160, 700.

Spectre de RMN du proton (350 MHz, $CDCl_3$, $\delta$ en ppm, J en Hz)

1,47 (s, 9H, $-C(CH_3)_3$); 3,08 et 3,13 (2s, 6H, $-N(CH_3)_2$);
3,58 et 3,71 (2d, J = 18, 2H, $-S-CH_2$); 5,06 (d, J = 4,5, 1H, –H en 6);
5,27 (d, J = 9, 1H, –CO–NH–); 5,67 (dd, J = 9 et 4,5, 1H, –H en 7);
6,95 (s, 1H, $-COO-C\underline{H}(C_6H_5)_2$); 7,07 (s, 1H, –H du thiazole);
7,10 à 7,50 (mt, aromatiques);
7,95 (s, 1H, $-N{=}CH-N\langle$).

B) Une solution de 4 g d'(amino-2 thiazolyl-5)-3 benzhydryloxycarbonyl-2 t.butoxycarbonylamino-7 oxo-8 thia-5 aza-1 bicyclo[4.2.0] octène-2 dans 50 $cm^3$ de tétrahydrofuranne est traitée par 1,13 g de diméthoxy(diméthylamino)méthane. Le mélange réactionnel est agité pendant 25 minutes à 20°C, puis dilué par 150 $cm^3$ d'acétate d'éthyle, lavé par 4 fois 150 $cm^3$ d'eau distillée et par 150 $cm^3$ de solution saturée de chlorure de sodium et séché sur sulfate de magnésium. Le résidu obtenu après évaporation du solvant sous pression réduite (30 mm de mercure; 4 kPa) à 30°C est chromatographié sur une colonne (hauteur: 28 cm; diamètre: 5 cm) de gel de silice (0,04—0,06 mm) en éluant sous une pression de 0,6 bar (60 kPa) par de l'acétate d'éthyle et en recueillant des fractions de 100 $cm^3$. Les fractions 9 à 23 contenant le produit pur sont réunies et concentrées à sec sous pression réduite (30 mm de mercure; 4 kPa) à 30°C. On obtient 2,25 g de benzhydryloxycarbonyl-2 t.butoxycarbonylamino-7 (diméthylaminométhylèneamino-2 thiazolyl-5)-3 oxo-8 thia-5 aza-1 bicyclo[4.2.0] octène-2 (forme E) sous la forme d'une meringue orangée dont les caractéristiques sont identiques à celles du produit obtenu en A).

Exemple 13

En traitant 4,6 g de benzhydryloxycarbonyl-2 t.butoxycarbonylamino-7 (isopropylidènehydrazo-2 thiazolyl-5)-3 oxo-8 thia-5 aza-1 bicyclo[4.2.0] octène-2 par 4,5 $cm^3$ d'acide méthanesulfonique dans 40 $cm^3$ d'acétonitrile selon le mode opératoire décrit dans l'exemple 15, on obtient 3,8 g d'amino-7 benzhydryloxycarbonyl-2 (isopropylidènehydrazo-2 thiazolyl-5)-3 oxo-8 thia-5 aza-1 bicyclo[4.2.0] octène-2 brut que l'on acyle selon le mode opératoire décrit dans l'exemple 1 par 0,91 $cm^3$ de chlorure de (thiényl-2) acétyle. Le produit brut est chromatographié sur une colonne (hauteur: 35 cm; diamètre: 5 cm) de gel de silice (0,04—0,06 mm) en éluant sous une pression de 0,5 bar (50 kPa) par un mélange de cyclohexane et d'acétate d'éthyle (50—50 en volumes) et en recueillant des fractions de 90 $cm^3$. Les fractions 23 à 29 contenant le produit pur sont réunies et concentrées à sec sous pression réduite (30 mm de mercure; 4 kPa) à 30°C. On obtient 0,78 g de benzhydryloxycarbonyl-2 (isopropylidènehydrazo-2 thiazolyl-5)-3 oxo-8 [(thiényl-2) acétamido]-7 thia-5 aza-1 bicyclo[4.2.0] octène-2 sous la forme d'une meringue jaune.

Spectre infra-rouge ($CHCl_3$), bandes caractéristiques ($cm^{-1}$) 3400, 3320, 3160, 3100—2500, 1780, 1720, 1680, 1545, 1510, 695.

Spectre de RMN du proton (350 MHz, $CDCl_3$, $\delta$ en ppm, J en Hz)

1,85 et 2,05 (2s, 6H, $-N{=}C(CH_3)_2$);

3,50 (mf, 2H, $-S-CH_2-$);

3,85 (s, 2H, hét$-CH_2-CO-N\langle$);

5,04 (d, J = 4,5, 1H, –H en 6); 5,84 (dd, J = 4,5 et 9,5, 1H, –H en 7);
6,93 (s, $-COO-C\underline{H}(C_6H_5)_2$); 6,9 à 7 (mt, =CH–CH= du thiophène);
6,09 à 7,4 (mt, aromatiques, –H du thiazole, =CH–S– du thiophène, –CO–NH– en 7 et –NH–N=).

Selon le mode opératoire de l'exemple 1, on traite 0,73 g de benzhydryloxycarbonyl-2 (isopropylidènehydrazo-2 thiazolyl-5)-3 oxo-8 [(thiényl-2) acétamido]-7 thia-5 aza-1 bicyclo[4.2.0] octène-2 par 20 $cm^3$ d'acide formique à 90% et l'on obtient 0,28 g de carboxy-2 (isopropylidènehydrazo-2 thiazolyl-5)-3 oxo-8 [(thiényl-2) acétamido]-7 thia-5 aza-1 bicyclo[4.2.0] octène-2 sous la forme d'un solide brun.

Spectre infra-rouge (KBr), bandes caractéristiques ($cm^{-1}$) 3260, 1770, 1650, 1615, 1530, 1370, 700.

Spectre de RMN du proton (350 MHz, DMSO $d_6$, $\delta$ en ppm, J en Hz)

1,90 et 1,95 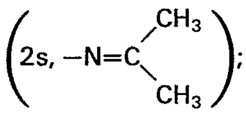;

3,71 et 3,84 (2d, J = 18, 2H, $-S-CH_2$);

3,78 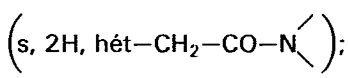;

5,13 (d, J = 4,5, 1H, —H en 6); 5,65 (mf, 1H, —H en 7);
6,93 à 7 (mt, 2H, =CH—CH= du thiophène);
7,28 à 7,4 (mt, 3H, =CH—S— du thiophène, —H du thiazole, —NH—N=);
9,15 (d, J = 9, 1H, —CO—NH— en 7).

Le benzhydryloxycarbonyl-2 t.butoxycarbonylamino-7 (isopropylidènehydrazo-2 thiazolyl-5)-3 oxo-8 thia-5 aza-1 bicyclo[4.2.0] octène-2 peut être obtenu de la manière suivante:

Selon le mode opératoire de l'exemple 2, mais en remplaçant la thiourée par la thiosemicarbazide de l'acétone (3,28 g) on traite 13,4 g de benzhydryloxycarbonyl-2 t.butoxycarbonylamino-7 (diméthylamino-2 vinyl)-3 oxo-8 thia-5 aza-1 bicyclo[4.2.0] octène-2 (forme E) et l'on chromatographie sur une colonne (hauteur: 35 cm, diamètre: 4 cm) de gel de silice (0,06—0,2 mm) en éluant par un mélange de cyclohexane et d'acétate d'éthyle (50—50 en volumes) et en recueillant des fractions de 100 $cm^3$. Les fractions 11 à 17 contenant le produit pur sont réunies et concentrées à sec sous pression réduite (30 mm de mercure; 4 kPa) à 30°C. On obtient 4,73 g de benzhydryloxycarbonyl-2 t.butoxycarbonylamino-7 (isopropylidènehydrazo-2 thiazolyl-5)-3 oxo-8 thia-5 aza-1 bicyclo[4.2.0] octène-2 sous la forme d'une meringue beige.

Spectre infra-rouge ($CHCl_3$), bandes caractéristiques ($cm^{-1}$) 3440, 3360, 3160, 1780, 1720, 1550, 1505, 1455, 1390, 1370, 1160, 700.

Spectre de RMN du proton (350 MHz, $CDCl_3$, $\delta$ en ppm, J en Hz)

1,48 (s, 9H, $-C(CH_3)_3$);

1,83 et 2,04 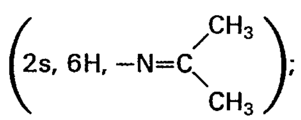;

3,53 et 3,67 (2d, J = 18, 2H, $-S-CH_2$); 5,03 (d, J = 4,5, 1H, —H en 6);
5,41 (d, J = 9, 1H, —CO—NH— en 7); 5,66 (dd, J = 4,5 et 9, 1H, —H en 7);
6,92 (s, 1H, $-COO-\underline{CH}(C_6H_5)_2$); 6,96 (s, 1H, —H du thiazole);
7,1 à 7,4 (mt, aromatiques + —NH—N=).

## Exemple 14

On acyle 3,4 g d'(acétamido-2 thiazolyl-5)-3 amino-7 benzhydryloxycarbonyl-2 oxo-8 thia-5 aza-1 bicyclo[4.2.0] octène-2 (obtenu comme décrit dans l'exemple 1) par 1,95 g de chlorure de (dithiol-1,3 one-2 yl-4) acétyle dans 75 $cm^3$ de tétrahydrofuranne sec en présence de 1,4 $cm^3$ de triéthylamine en opérant selon le mode opératoire décrit dans l'exemple 1. Le produit brut est chromatographié sur une colonne (hauteur: 30 cm; diamètre: 6 cm) de gel de silice (0,04—0,06 mm) en éluant sous une pression de 0,4 bar (40 kPa) par un mélange de cyclohexane et d'acétate d'éthyle 20—80 (en volumes) en recueillant des fractions de 100 $cm^3$. Les fractions 11 à 20 sont réunies et concentrées à sec sous pression réduite (30 mm de mercure; 4 kPa) à 30°C. On obtient 1,87 g du produit attendu que l'on cristallise dans 90 $cm^3$ d'acétonitrile. On obtient 0,8 g d'(acétamido-2 thiazolyl-5)-3 benzhydryloxycarbonyl-2 (dithiol-1,3 one-2 yl-4 acétamido)-7 oxo-8 thia-5 aza-1 bicyclo[4.2.0] octène-2 sous la forme de cristaux blancs.

Spectre infra-rouge (KBr), bandes caractéristiques ($cm^{-1}$) 3280, 1780, 1730, 1695, 1640, 1545, 1225, 700.

Spectre de RMN du proton (350 MHz, DMSO $d_6$, $\delta$ en ppm, J en Hz)

2,14 (s, 3H, $-CO-CH_3$);

3,67 $\left(s,\ 2H,\ -CH_2-CO-N\langle\right)$;

3,79 et 3,92 (2d, J = 18, 2H, $-CH_2-S-$); 5,23 (d, J = 4,5, 1H, –H en 6);
5,8 (dd, J = 4,5 et 9, 1H, –H en 7); 6,83 (s, 1H, $-COOC\underline{H}(C_6H_5)_2$);
7,01 (s, 1H, $-S-CH=$); 7,03 à 7,4 (mt, 11H, aromatiques et –H thiazole);
9,3 (d, J = 9, 1H, –CO–NH–); 12,06 (s, 1H, $-N\underline{H}-CO-CH_3$).

En opérant selon le mode opératoire décrit dans l'exemple 2 mais à partir de 0,8 g d'(acétamido-2 thiazolyl-5)-3 benzhydryloxycarbonyl-2 (dithiol-1,3 one-2 yl-4 acétamido)-7 oxo-8 thia-5 aza-1 bicyclo[4.2.0] octène-2, on obtient 0,2 g d'(acétamido-2 thiazolyl-5)-3 carboxy-2 (dithiol-1,3 one-2 yl-4 acétamido)-7 oxo-8 thia-5 aza-1 bicyclo[4.2.0] octène-2 sous la forme d'un solide beige.

Spectre infra-rouge (KBr), bandes caractéristiques ($cm^{-1}$) 3270, 3100 à 2100, 1785, 1685, 1640, 1545.

Spectre de RMN du proton (350 MHz, DMSO $d_6$, $\delta$ en ppm, J en Hz)

2,14 (s, 3H, $-CO-CH_3$);

3,65 $\left(s,\ 2H,\ -CH_2-CO-N\langle\right)$;

3,77 et 3,91 (2d, J = 18, 2H, $-CH_2-S-$); 5,18 (d, J = 5, 1H, –H en 6);
5,68 (dd, J = 5 et 9, 1H, –H en 7); 7,01 (s, 1H, $-S-CH=$);
7,52 (s, 1H, H thiazole); 9,27 (d, J = 9, 1H, –CO–NH–);
12,15 (s large, 1H, $-N\underline{H}-CO-CH_3$).

## Exemple 15

Une solution de 6,06 g d'(acétamido-2 thiazolyl-5)-3 benzhydryloxycarbonyl-2 t.butoxycarbonylamino-7 oxo-8 thia-5 aza-1 bicyclo[4.2.0] octène-2 dans 100 $cm^3$ d'acétonitrile est agitée avec 6 $cm^3$ d'acide méthanesulfonique pendant 30 minutes à 20°C puis diluée par 200 $cm^3$ d'acétate d'éthyle et agitée avec 300 $cm^3$ de solution saturée de bicarbonate de sodium. La couche aqueuse est extraite par 100 $cm^3$ d'acétate d'éthyle et les solutions organiques jointes sont lavées par 2 fois 250 $cm^3$ de solution demi-saturée de chlorure de sodium puis séchées sur sulfate de magnésium et concentrées à sec sous pression réduite (30 mm de mercure; 4 kPa) à 40°C. On obtient 4,9 g d'(acétamido-2 thiazolyl-5)-3 amino-7 benzhydryloxycarbonyl-2 oxo-8 thia-5 aza-1 bicyclo[4.2.0] octène-2 sous la forme d'une meringue jaune. Ce produit est redissous dans 75 $cm^3$ de tétrahydrofuranne sec. La solution refroidie vers 0°C est traitée successivement par 1,54 g de chlorure de phénylacétyle et par 1,4 $cm^3$ de triéthylamine puis agitée pendant 1 heure entre 0 et 4°C. Le mélange réactionnel est dilué par 100 $cm^3$ d'acétate d'éthyle et 200 $cm^3$ de solution demi-saturée de bicarbonate de sodium. La couche organique est décantée, lavée par 100 $cm^3$ d'eau puis par 100 $cm^3$ de solution demi-saturée de chlorure de sodium, séchée sur sulfate de magnésium et concentrée à sec pression réduite (30 mm de mercure; 4 kPa) à 40°C. Le résidu est repris par 25 $cm^3$ de chlorure de méthylène. Le précipité est essoré puis repris par 80 $cm^3$ d'acétonitrile bouillant. Après refroidissement le précipité est essoré, lavé par 10 $cm^3$ d'acétonitrile et séché sous pression réduite (2 mm de mercure; 0,27 kPa) à 25°C. On obtient ainsi 2,65 g d'(acétamido-2 thiazolyl-5)-3 benzhydryloxycarbonyl-2 oxo-8 phénylacétamido-7 thia-5 aza-1 bicyclo[4.2.0] octène-2 sous la forme d'un solide blanc.

Spectre infra-rouge (KBr), bandes caractéristiques ($cm^{-1}$) 3410, 3370, 3170, 1780, 1730, 1720, 1700, 1680, 1655, 1540, 1525, 1515, 1495, 1455, 1230, 760, 740, 700.

Spectre de RMN du proton (350 MHz, $CDCl_3-DMSO\ d_6$, $\delta$ en ppm, J en Hz)

2,25 $\left(s,\ 3H,\ \rangle N-CO-CH_3\right)$;

3,57 et 3,68 $\left(2d,\ J = 14,\ 2H,\ -CH_2CO-N\langle\right)$;

3,63 et 3,75 (2d, J = 18, 2H, $-CH_2-S-$); 5,14 (d, J = 4,5, 1H, –H en 6);
5,84 (dd, J = 4,5 et 9, 1H, –H en 7); 6,88 (s, 1H, $-COO-C\underline{H}(C_6H_5)_2$);
7,12 (s, 1H, –H du thiazole); 7 à 7,5 (mt, 15H, aromatiques);
9,08 (d, J = 9, 1H, –CO–NH–);
11,95 (s large, 1H, $-N\underline{H}-CO-CH_3$).

De façon similaire à l'exemple 1 on traite 2,65 g d'(acétamido-2 thiazolyl-5)-3 benzhydryloxycarbonyl-2 oxo-8 phénylacétamido-7 thia-5 aza-1 bicyclo[4.2.0] octène-2 et obtient 1,7 g de l'acide correspondant, qui est purifié de la façon suivante: on dissout le produit dans une solution à 5% de bicarbonate de sodium. La solution aqueuse est lavée par de l'acétate d'éthyle puis acidifiée à pH = 4 par de l'acide chlorhydrique 1 N. Le précipité est essoré, lavé par 10 $cm^3$ d'eau, 10 $cm^3$ d'éthanol et 10 $cm^3$ d'éther éthylique et séché. On obtient ainsi 1,55 g d'(acétamido-2 thiazolyl-5)-3 carboxy-2 oxo-8 phénylacétamido-7 thia-5 aza-1 bicyclo[4.2.0] octène-2 sous la forme d'une solide blanc.

Spectre infra-rouge (KBr), bandes caractéristiques ($cm^{-1}$) 3280, 3200, 3100–2300, 1785, 1710, 1695, 1650, 1540, 1520, 1495, 1450.

Spectre de RMN du proton (350 MHz, DMSO $d_6$, $\delta$ en ppm, J en Hz)

2,18 (s, 3H, >N–CO–$CH_3$);

3,53 et 3,63 (2d, J = 14, 2H, –$CH_2$–CO–N<);

3,78 et 3,93 (2d, J = 18, 2H, –$CH_2$–S–); 5,17 (d, J = 4,5, 1H, –H en 6);
5,74 (dd, J = 4,5 et 8, 1H, –H en 7); 7,15 à 7,45 (mt, 5H, aromatiques);
7,52 (s, 1H, –H du thiazole); 9,17 (d, J = 9, 1H, –CO–NH–);
12,19 (s large, 1H, –NH–CO–$CH_3$); 13,54 (mf, 1H, –COOH).

En opérant selon le mode opératoire de l'exemple 1 on dissout 1,1 g d'(acétamido-2 thiazolyl-5)-3 carboxy-2 oxo-8 phénylacétamido-7 thia-5 aza-1 bicyclo[4.2.0] octène-2 dans une solution de 0,25 g de bicarbonate de sodium et 100 $cm^3$ d'eau distillée et on chromatographie sur une colonne (diamètre: 1,8 cm, hauteur: 27 cm) de résine DUOLITE S 841 (préalablement lavée comme décrit dans l'exemple 4) en éluant par 300 $cm^3$ d'eau distillée puis par des mélanges d'eau et d'éthanol 95–5 (200 $cm^3$), 90–10 (200 $cm^3$), 80–20 (200 $cm^3$) et 60–40 (200 $cm^3$) (en volumes) et en recueillant des fractions de 50 $cm^3$. Les fractions 8 à 20 sont réunies et lyophilisées après que l'on ait évaporé l'alcool sous pression réduite (30 mm de mercure; 4 kPa) à 30°C. On obtient 1,1 g de sel de sodium de l'(acétamido-2 thiazolyl-5)-3 carboxy-2 oxo-8 phénylacétamido-7 thia-5 aza-1 bicyclo[4.2.0] octène-2 sous le forme d'un lyophilisat blanc.

Spectre infra-rouge (KBr), bandes caractéristiques ($cm^{-1}$) 3400–2500, 1760, 1670, 1605, 1550, 1395, 1370, 1305, 1010.

Spectre de RMN du proton (350 MHz, DMSO $d_6$, $\delta$ en ppm, J en Hz)

2,11 (s, 3H, $CH_3CO$–); 3,50 et 3,59 (2d, J = 14, 2H, $C_6H_5\underline{CH_2}CO$–);
3,59 et 3,70 (2d, J = 16, 2H, –$SCH_2$–); 5,02 (d, J = 5, 1H, H en 6);
5,50 (dd, J = 9 et 5, 1H, H en 7); 7,20 à 7,35 (mt, 5H, phényle);
7,46 (s, 1H, H en 4 du thiazole); 9,07 (d, J = 9, 1H, –CON$\underline{H}$–$C_7$); 11,90 (s large, 1H, $CH_3CON\underline{H}$–).

## Exemple 16

A une solution refroidie à 3°C de 2,6 g d'amino-7 benzhydryloxycarbonyl-2 [(diméthylamino-2 éthylamino)-2 thiazolyl-5]-3 oxo-8 thia-5 aza-1 bicyclo[4.2.0] octène-2 dans 60 $cm^3$ de dichlorométhane sec, on ajoute 0,65 $cm^3$ de triéthylamine. Au mélange obtenu, on ajoute goutte à goutte une solution de 1,2 g de chlorure de (dichloro-3,4 phényl)thioacétyle dans 20 $cm^3$ de dichlorométhane. L'addition terminée, on agite le mélange à 23°C pendant 2 heures 30 minutes. Le mélange réactionnel est alors dilué par 250 $cm^3$ de dichlorméthane puis on lave la phase organique successivement par 200 $cm^3$ d'eau distillée, 200 $cm^3$ d'une solution saturée de bicarbonate de sodium et 200 $cm^3$ d'une solution saturée de chlorure de sodium. La phase organique est séchée sur sulfate de magnésium anhydre, filtrée et concentrée à sec sous pression réduite (30 mm de mercure; 4 kPa). Le résidu est purifié par chromatographie sur gel de silice (hauteur de silice 18 cm, diamètre de la colonne 3 cm) (0,04–0,06 mm) en éluant sous une pression de 50 kPa par un mélange de dichlorométhane et de méthanol (90–10 en volumes) et en recueillant des fractions de 30 $cm^3$. Les fractions 11 à 17 contenant le produit sont réunies et concentrées à sec sous pression réduite (30 mm de mercure; 4 kPa) à 30°C. On obtient 1,5 g de benzhydryloxycarbonyl-2 (dichloro-3,4-phénylthio)acétamido-7 [(diméthylamino-2 éthylamino)-2 thiazolyl-5]-3 oxo-8 thia-5 aza-1 bicyclo[4.2.0] octène-2, sous la forme d'une meringue jaune.

Spectre de RMN du proton (250 MHz, $CDCl_3$, $\delta$ en ppm, J en Hz)

2,33 (s, 6H, –$N(CH_3)_2$);

2,60 (t, J = 6, 2H, >N–$CH_2$–);

3,24 (mt, 2H, –NH–$\underline{CH_2}$); 3,46 et 3,59 (2d, J = 18, 2H, –S–$CH_2$–);

3,64 et 3,75 (2d, J = 15, 2H, $-S-CH_2-C(=O)-N<$);

5,03 (d, J = 5, 1H, –H en 6); 5,78 (dd, J = 9 et 5, 1H, –H en 7);

6,09 (mf, 1H, >NH);

6,96 (s, 1H, –H du thiazole ou $-COO-C\underline{H}(C_6H_5)_2$);
6,97 (s, 1H, $-COO-C\underline{H}(C_6H_5)_2$) ou –H du thiazole);
7,10 à 7,35 (mt, aromatique et –H aromatiques en 6);
7,39 (d, J = 8,5, 1H, –H aromatique en 5);
7,45 (d, J = 2, 1H, –H aromatique en 2);

7,47 (d, J = 9, 1H, $-NH-\overset{|}{C}=O$).

On agite pendant 30 minutes à 50°C un mélange de 1,5 g de benzhydryloxycarbonyl-2 (dichloro-3,4 phénylthio)acétamido-7 [(diméthylamino-2 éthylamino)-2 thiazolyl-5]-3 oxo-8 thia-5 aza-1 bicyclo[4.2.0] octène-2, 5 $cm^3$ d'anisole et 25 $cm^3$ d'acide formique. Le mélange est concentré à sec sous pression réduite (0,1 mm de mercure; 0,013 kPa) puis repris par trois fois 50 $cm^3$ d'éthanol et concentré à sec sous pression réduite (30 mm de mercure; 4 kPa) à 30°C. Le solide obtenu est lavé par 50 $cm^3$ d'acétone, puis 50 $cm^3$ d'éther éthylique. Il est séché sous pression réduite (0,1 mm de mercure; 0,013 kPa) pour donner 1 g de carboxy-2 (dichloro-3,4 phénylthio)acétamido-7 [(diméthylamino-2 éthylamino)-2 thiazolyl-5]-3 oxo-8 thia-5 aza-1 bicyclo[4.2.0] octène-2 sous la forme d'une poudre jaune.

Spectre infra-rouge (KBr), bandes caractéristiques ($cm^{-1}$) 3260, 1765, 1675, 1605, 1530, 1460, 1380, 815.

Spectre de RMN du proton (250 MHz, DMSO $d_6$, $\delta$ en ppm, J en Hz)

2,69 (s, 6H, $-N(CH_3)_2$); 3,15 (mf, $=N-CH_2-$); 3,52 (mf, $-NH-C\underline{H_2}$);
3,62 et 3,88 (2d, J = 18, 2H, $-S-CH_2-$); 3,82 (s, 2H, $-S-CH_2-CO-N=$);
5,05 (d, J = 5, 1H, –H en 6); 5,58 (dd, J = 8 et 5, 1H, –H en 7);
7,24 (s, 1H, –H du thiazole); 7,35 (dd, J = 8,5 et 2, 1H, –H aromatique en 6);
7,55 (d, J = 8,5, 1H, –H aromatique en 5); 7,65 (d, J = 2, 1H, –H aromatique en 2);
9,07 (mf, 1H, =N–H); 9,15 (d, J = 8, 1H, –CONH–).

(250 MHz, DMSO $d_6$ + 1 γ de $CF_3COOD$, $\delta$ en ppm, J en Hz)

2,87 (s, –6H, $-N(CH_3)_2$); 3,37 (t, J = 6, 2H, $=N-CH_2-$); 3,74 (t, J = 6, 2H, $-NHCH_2$).

L'amino-7 benzhydryloxycarbonyl-2 [(diméthylamino-2 éthylamino)-2 thiazolyl-5]-3 oxo-8 thia-5 aza-1 bicyclo[4.2.0] octène-2 peut être obtenu de la manière suivante:

A une suspension de 0,3 g de benzhydryloxycarbonal-2 t.butoxycarbonylamino-7 [(diméthylamino-2 éthylamino)-2 thiazolyl-5]-3 oxo-8 thia-5 aza-1 bicyclo[4.2.0] octène-2 dans 3 $cm^3$ d'acétonitrile, on ajoute 0,3 $cm^3$ d'acide méthanesulfonique. La solution jaune obtenue est agitée à 23°C pendant 5 minutes et est versée sur un mélange de 50 $cm^3$ d'une solution saturée de bicarbonate de sodium et de 50 $cm^3$ d'acétate d'éthyle. La phase organique est séchée sur sulfate de magnésium anhydre, filtrée et concentrée sous pression réduite (20 mm de mercure; 2,7 kPa). On obtient ainsi 0,25 g d'amino-7 benzhydryloxycarbonyl-2 [(diméthylamino-2 éthylamino)-2 thiazolyl-5]-3 oxo-8 thia-5 aza-1 bicyclo[4.2.0] octène-2 sous la forme d'une meringue jaune.

Spectre infra-rouge (KBr), bandes caractéristiques ($cm^{-1}$) 3400, 1770, 1720, 1620, 1540, 1490, 1450, 1220, 760, 745 et 700.

Spectre de RMN du proton (250 MHz, $CDCl_3$, $\delta$ en ppm, J en Hz)

2,26 (s, 6H, $-N(CH_3)_2$);

2,53 (t, J = 6, 2H, $>N-CH_2-$);

3,17 (mt, 2H, $-NH-C\underline{H_2}-$); 3,50 et 3,66 (2d, J = 18,.2H, $-S-CH_2-$);
4,77 (d large, J = 5, 1H, –H en 7); 5,0 (d, J = 5, 1H, –H en 6);

6,0 (mf, >NH);

6,90 (s, 1H, –H thiazole ou $-COOC\underline{H}(C_6H_5)_2$); 6,95 (s, 1H, $-COOC\underline{H}(C_6H_5)_2$) ou –H thiazole); 7,05 à 7,4 (mt, aromatiques, $-NH_2$).

Le benzhydryloxycarbonyl-2 t.butoxycarbonylamino-7 [(diméthylamino-2 éthylamino)-2 thiazolyl-5]-3 oxo-8 thia-5 aza-1 bicyclo[4.2.0] octène-2 peut être obtenu de la manière suivante:

A une solution de 88 g de benzhydryloxycarbonyl-2 (bromo-1 oxo-2 éthyl)-3 t.butoxycarbonylamino-7 oxo-8 thia-5 aza-1 bicyclo[4.2.0] octène-2 dans 500 $cm^3$ de tétrahydrofuranne anhydre, on ajoute un mélange de 22 g de (diméthylamino-2 éthyl)-1 thiourée dans 200 $cm^3$ de dichlorométhane. On agite pendant 24 heures à 25°C et on verse le mélange réactionnel sur un mélange de 1,5 litre d'acétate d'éthyle et 1,5 litre de solution saturée de bicarbonate de sodium. La phase organique est lavé par 1 litre de solution saturée de bicarbonate de sodium, 1 litre d'eau distillée et 1 litre de solution saturée de chlorure des sodium, puis séchée sur sulfate de magnésium anhydre. Après filtration et concentration du filtrat sous pression réduite (20 mm de mercure; 2,7 kPa), le résidu est purifié par chromatographie sur gel de silice (0,063–0,2 mm) [hauteur de silice: 38 cm, diamètre de la colonne: 10 cm] en éluant par 5 litres de dichlorométhane puis 11 litres d'un mélange dichlorométhane-méthanol (90–10 en volumes) en recueillant des fractions de 1 litre. En concentrant à sec les fractions 14 à 16, on obtient 15 g d'une meringue jaune qui est dissoute dans 100 $cm^3$ d'acétonitrile chaud. En refroidissant, on obtient 3 g de benzhydryloxycarbonyl-2 t.butoxycarbonylamino-7 [(diméthylamino-2 éthylamino)-2 thiazolyl-5]-3 oxo-8 thia-5 aza-1 bicyclo[4.2.0] octène-2 sous la forme de cristaux blancs (F. inst. = 163°C Kofler).

Spectre infra-rouge (KBr), bandes caractéristiques ($cm^{-1}$) 3380, 3200, 1780, 1720, 1710, 1555, 1525, 1370, 1165, 755, 740 et 700.

Spectre de RMN du proton (250 MHz, $CDCl_3$, $\delta$ en ppm, J en Hz)

1,47 (s, 9H, $-C(CH_3)_3$); 2,27 (s, 6H, $-N(CH_3)_2$);
2,53 (t, J = 6, 2H, $>N-CH_2-$);
3,18 (mt, 2H, $-NH-C\underline{H}_2-$); 3,52 et 3,66 (2d, J = 18, 2H, $-S-CH_2-$);
5,04 (d, J = 5, 1H, –H en 6); 5,40 (d, J = 9, 1H, –CONH–);
5,64 (dd, J = 9 et 5, 1H, –H en 7);
5,93 (mf, 1H, $>NH$);
6,96 (s, 1H, –H thiazole ou $-COO-C\underline{H}(C_6H_5)_2$);
6,97 (s, 1H, $-COOC\underline{H}(C_6H_5)_2$ ou –H thiazole);
7,15 à 7,40 (mt, aromatiques).

La (diméthylamino-2 éthyl)-1 thiourée peut être obtenue de la manière suivante:

On chauffe pendant 36 heures au reflux une solution de 139 g de N[(diméthylamino)-2 éthyl] dithiocarbamate de méthyle dans 600 $cm^3$ d'éthanol et de 275 $cm^3$ de solution aqueuse d'ammoniaque à 28%. Le mélange est concentré sous pression réduite (20 mm de mercure; 2,7 kPa) et le résidu cristallisé est lavé par 200 $cm^3$ d'éther éthylique. On obtient 54 g de (diméthylamino-2 éthyl)-1 thiourée sous la forme de cristaux blancs.

Spectre infra-rouge (KBr), bandes caractéristiques ($cm^{-1}$) 3306, 2790, 1640, 1560 et 1350.

Le N[(diméthylamino)-2 éthyl] dithiocarbamate de méthyle peut être obtenu selon la méthode décrite dans la demande de brevet allemand 2 738 711.

## Exemple 17

A une solution de 3,2 g d'(acétamido-2 thiazolyl-5)-3 amino-7 benzhydryloxycarbonyl-2 oxo-8 thia-5 aza-1 bicyclo[4.2.0] octène-2 brut (tel qu'obtenu dans l'exemple 1) dans 50 $cm^3$ de chlorure de méthylène on ajoute 1,59 g de D N-t.butoxycarbonylphénylglycine. On refroidit la solution à 4°C et ajoute en 10 minutes une solution de 1,43 g de NN'dicyclohexylcarbodiimide dans 10 $cm^3$ de chlorure de méthylène. Après 50 minutes vers 4°C le mélange réactionnel est filtré puis concentré à sec sous pression réduite (30 mm de mercure; 4 kPa) à 30°C. Le résidu est repris dans 150 $cm^3$ d'éthyle et la solution est lavée successivement par 50 $cm^3$ d'acide chlorhydrique 0,1 N, 50 $cm^3$ de solution demi-saturée de carbonate de sodium puis par 2 fois 50 $cm^3$ d'eau et 50 $cm^3$ de solution saturée de chlorure de sodium, séchée sur sulfate de magnésium, filtrée et concentrée à sec sous pression réduite (30 mm de mercure; 4 kPa) à 40°C. Le résidu (produit attendu brut) est purifié par cristallisation dans 90 $cm^3$ d'acétonitrile. On obtient 2,38 g d'(acétylamino-2 thiazolyl-5)-3 benzhydryloxycarbonyl-2 [(D) $\alpha$-t.butoxycarbonylamino-2 phénylacétamido]-7 oxo-8 thia-5 aza-1 bicyclo[4.2.0] octène-2 sous la forme d'une poudre cristalline beige clair.

Spectre infra-rouge ($CHBr_3$), bandes caractéristiques ($cm^{-1}$) 3400, 3260, 3160, 1780, 1715, 1690, 1560, 1500, 1490, 1450, 1390, 1370, 760, 740.

Spectre de RMN du proton (350 MHz, DMSO $d_6$, $\delta$ en ppm, J en Hz)

1,4 (s, 9H, $-C(CH_3)_3$); 2,13 (s, 3H, $-CO-CH_3$); 3,67 et 3,81 (2d, J = 18, 2H, $-S-CH_2-$);
5,12 (d, J = 5, 1H, –H en 6);
5,35 (d, J = 8, 1H, 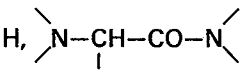);
5,54 (d, J = 8, 0,5H, 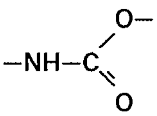 en partie échangée);
5,83 (dd, J = 5 et 9, 1H, –H en 7); 6,82 (s, 1H, $-COO-\underline{C}H(C_6H_5)_2$);
7 à 7,5 (mt, 16H, aromatiques et –H du thiazole); 9,26 (d, J = 9, 1H, –CO–NH–);
12,04 (s, 1H, $-N\underline{H}-CO-CH_3$).

2,25 g d'(acétylamino-2 thiazolyl-5)-3 benzhydryloxycarbonyl-2 [(D) $\alpha$-t.butoxycarbonylamino-phénylacétamido]-7 oxo-8 thia-5 aza-1 bicyclo[4.2.0] octène-2 sont dissous dans 20 $cm^3$ d'acide trifluoroacétique. La solution est agitée à 25°C pendant 20 minutes puis concentrée à sec sous pression réduite (5 mm de mercure; 0,7 kPa) à 40°C. Le résidu est repris par 30 $cm^3$ d'oxyde d'isopropyle. On évapore à sec de nouveau sous pression réduite (30 mm de mercure; 4 kPa) à 40°C. Cette opération est répétée 4 fois puis le résidu est triuiré avec 100 $cm^3$ d'éther éthylique puis essoré, lavé par 3 fois 30 $cm^3$ d'éther éthylique puis séché. On obtient 1,45 g de trifluoroacétate d'(acétylamino-2 thiazolyl-5)-3 [(D) $\alpha$-aminophénylacétamido]-7 carboxy-2 oxo-8 thia-5 aza-1 bicyclo[4.2.0] octène-2 sous la forme d'une poudre beige.

Spectre infra-rouge (KBr), bandes caractéristiques ($cm^{-1}$) 3180, 3100, 2150, 1775, 1680, 1455, 1200, 800, 720, 700.

Spectre de RMN du proton (350 MHz, DMSO $d_6$, $\delta$ en ppm, J en Hz)

2,13 (s, 3H, $-CO-CH_3$); 3,66 et 3,78 (2d, J = 18, 2H, $-S-CH_2-$);
5,01 (s, 1H, 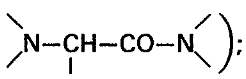);
5,11 (d, J = 4,5, 1H, –H en 6); 5,81 (mf, 1H, –H en 7);
7,35 à 7,65 (mt, 6H, aromatiques et –H thiazole); 8 à 10 (mf étalé, 1H, –COOH);
9,58 (d, J = 8, 1H, –CO–NH–); 12,14 (mf, 1H, $-NH-CO-CH_3$).

On redissout 1,2 g de ce trifluoroacétate dans 100 $cm^3$ d'eau et la solution est lavée par 3 fois 20 $cm^3$ d'acétate d'éthyle puis décantée, traitée par 0,5 g de noir décolorant, filtrée et agitée avec 20 $cm^3$ de résine Amberlite IR 45 ($OH^-$) (préalablement lavée à l'eau à jusqu'à neutralité) jusqu'à ce que le pH de la solution aqueuse atteigne 5,5. Après élimination de la résine par filtration, la solution aqueuse est lyophilisée. On obtient 0,58 g d'(acétylamino-2 thiazolyl-5)-3 [(D) $\alpha$-aminophénylacétamido]-7 carboxy-2 oxo-8 thia-5 aza-1 bicyclo[4.2.0] octène-2 sous la forme d'un lyophilisat blanc.

Spectre infra-rouge (KBr), bandes caractéristiques ($cm^{-1}$) 3240, 3100–2300, 1765, 1685, 1600, 1545, 1510, 1455, 1370, 720, 700.

Spectre de RMN ($CF_3CO_2H$) conforme à celui du trifluoroacétate.

## Exemple 18

En opérant selon le mode opératoire décrit dans l'exemple 17 on acyle 4,9 g d'amino-7 benzhydryloxycarbonyl-2 oxo-8 (thiazolyl-5)-3 thia-5 aza-1 bicyclo[4.2.0] octène-2 par 2,74 g de (D)N-t.butoxycarbonylphénylglycine en présence de 2,5 g de N,N' dicyclohexylcarbodiimide dans 40 $cm^3$ de chlorure de méthylène. Après chromatographie sur une colonne (hauteur: 30 cm, diamètre: 4 cm) de gel de silice (0,04–0,06 mm) en éluant sous une pression de 1 bar (100 kPa) par un mélange de cyclohexane et d'acétate d'éthyle (60–40 en volumes) et en recueillant des fractions de 100 $cm^3$, les fractions 15 à 24 contenant le produit pur sont réunies et concentrées à sec sous pression réduite (30 mm de mercure; 4 kPa) à 30°C. On obtient 2,4 g de benzhydryloxycarbonyl-2 oxo-8 [(D)$\alpha$-t.butoxycarbonylamino-phénylacétamido]-7 (thiazolyl-5)-3 thia-5 aza-1 bicyclo[4.2.0] octène-2 sous la forme d'un solide de couleur crème.

Spectre infra-rouge ($CHBr_3$), bandes caractéristiques ($cm^{-1}$) 3400, 1788, 1715, 1690, 1490, 1450, 1390, 1365, 755, 740.

Spectre de RMN du proton (350 MHz, $CDCl_3$, $\delta$ en ppm, J en Hz)

1,43 (s, J = 9, $-C(CH_3)_3$); 3,42 et 3,63 (2d, J = 18, 2H, $-S-CH_2$);
5,02 (d, J = 5, 1H, —H en 6);

5,23 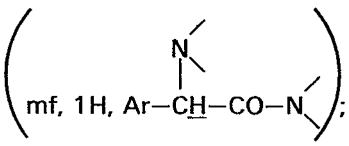;

5,65 (d, J = 6, 1H, —NH—COO—); 5,89 (dd, J = 5 et 9, 1H, —H en 7); 6,65 (mf, 1H, —CO—NH—);
6,89 (s, 1H, $-COO-C\underline{H}(C_6H_5)_2$); 7 à 7,45 (mt, aromatiques); 7,53 (s, 1H, =CH—N= du thiazole);
8,56 (s, 1H, —N=CH—S— du thiazole).

Selon le mode opératoire de l'exemple 17, on traite 2,4 g de benzhydryloxycarbonyl-2 [(D)α-t.butoxycarbonylaminophénylacétamido]-7 oxo-8 (thiazolyl-5)-3 thia-5 aza-1 bicyclo[4.2.0] octène-2 par 24 $cm^3$ d'acide trifluoroacétique. On obtient 1,25 g de trifluoroacétate de [(D)α-aminophénylacétamido]-7 carboxy-2 oxo-8 (thiazolyl-5)-3 thia-5 aza-1 bicyclo[4.2.0] octène-2 sous la forme d'un solide de couleur crème.

Spectre infra-rouge (KBr), bandes caractéristiques ($cm^{-1}$) 3200, 3150—2200, 1770, 1680, 1620, 1550, 1205, 1140, 800, 725.

Spectre de RMN du proton (350 MHz, $CF_3COOD$, δ en ppm, J en Hz)

3,60 et 4,01 (2d, J = 19, 2H, $-CH_2-S-$); 5,38 (d, J = 5, 1H, —H en 6);

5,55 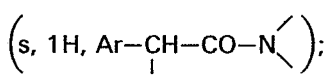;

5,98 (d, J = 5, —H en 7); 7 à 7,5 (mt, 5H, aromatiques); 8,32 (s, 1H, H en 4 du thiazole);
9,97 (s, 1H, H en 2 du thiazole).

L'amino-7 benzhydryloxycarbonyl-2 oxo-8 (thiazolyl-5)-3 thia-5 aza-1 bicyclo[4.2.0] octène-2 peut être obtenu de la manière suivante:

Selon le mode opératoire décrit dans l'exemple 15, on traite 10 g de benzhydryloxycarbonyl-2 t.butoxycarbonylamino-7 oxo-8 (thiazolyl-5)-3 thia-5 aza-1 bicyclo[4.2.0] octène-2 par 10 $cm^3$ d'acide méthanesulfonique dans 100 $cm^3$ d'acétonitrile. On obtient 8,2 g d'amino-7 benzhydryloxycarbonyl-2 oxo-8 (thiazolyl-5)-3 thia-5 aza-1 bicyclo[4.2.0] octène-2 sous la forme d'un solide jaune.

Spectre infra-rouge ($CHBr_3$), bandes caractéristiques ($cm^{-1}$) 3400, 3300, 1780, 1725, 1620, 1495, 1450, 1220, 755, 740.

Spectre de RMN du proton (350 MHz, $CDCl_3$, δ en ppm, J en Hz)

3,55 et 3,74 (2d, J = 18, 2H, $-SCH_2-$); 4,85 (d, J = 5, 1H, H en 7);
5,05 (d, J = 5, 1H, H en 6); 6,92 (s, 1H, $-C\underline{H}(C_6H_5)_2$); 7 à 7,45 (mt, 10 H aromatiques);
7,55 (s, 1H, H en 4 du thiazole); 8,55 (s, 1H, H en 2 du thiazole).

## Exemple 19

Selon le mode opératoire décrit dans l'exemple 15, on traite 4,9 g de benzhydryloxycarbonyl-2 t.butoxycarbonylamino-7 oxo-8 (phényl-2 thiazolyl-5)-3 thia-5 aza-1 bicyclo[4.2.0] octène-2 par 4,9 $cm^3$ d'acide méthanesulfonique dans 39 $cm^3$ d'acétonitrile. On obtient 4,1 g d'amino-7 benzhydryloxycarbonyl-2 oxo-8 (phényl-2 thiazolyl-5)-3 thia-5 aza-1 bicyclo[4.2.0] octène-2 brut, qui est ensuite acylé selon le mode opératoire décrit dans l'exemple 17 par 1,96 g de (D)N-t.butoxycarbonylphénylglycine en présence de 1,78 g de N,N' dicyclohexylcarbodiimide dans 40 $cm^3$ de chlorure de méthylène sec. Le produit brut obtenu est purifié par chromatographie sur une colonne (heuteur: 30 cm, diamètre: 4 cm) de gel de silice (0,04—0,06 mm) en éluant sous une pression de 0,5 bar (50 kPa) par un mélange de cyclohexane et d'acétate d'éthyle (60—40 en volumes) et en recueillant des fractions de 50 $cm^3$. Les fractions 9 à 33 contenant le produit pur sont réunies et concentrées à sec sous pression réduite (30 mm de mercure; 4 kPa) à 30°C. On obtient 2,8 g de benzhydryloxycarbonyl-2 [(D)αt.butoxycarbonylaminophénylacétamido]-7 oxo-8 (phényl-2 thiazolyl-5)-3 thia-5 aza-1 bicyclo[4.2.0] octène-2 sous la forme d'un solide de couleur crème.

Spectre infra-rouge ($CHBr_3$), bandes caractéristiques ($cm^{-1}$) 3410, 1785, 1720, 1695, 1495, 1225, 760.

Spectre de RMN du proton (350 MHz, $CDCl_3$, δ en ppm, J en Hz)

1,43 (s, 9H, $-C(CH_3)_3$); 3,49 et 3,66 (2d, J = 18, 2H, $-S-CH_2$);
5,04 (d, J = 4,5, 1H, —H en 6);

5,25 (mf, 1H, $\mathrm{Ar{-}\underset{|}{C}H{-}CON}\langle$);

5,67 (d, J = 6, 1H, —NH—COO—); 5,91 (dd, J = 4,5 et 9, 1H, —H en 7);
6,63 (mf, 1H, —CO—NH— en 7); 6,94 (s, 1H, —COO—C<u>H</u>$(C_6H_5)_2$); 7 à 7,5 (mt, aromatiques);
7,48 (s, 1H, H thiazole); 7,73 (dd, J = 7 et 1,5, 2H, —H aromatiques en ortho du thiazole).

On traite 2,8 g de benzhydryloxycarbonyl-2 [(D)$\alpha$-t.butoxycarbonylaminophénylacétamido]-7 oxo-8 (phényl-2 thiazolyl-5)-3 thia-5 aza-1 bicyclo[4.2.0] octène-2 par 28 $cm^3$ d'acide trifluoroacétique en présence de 2 $cm^3$ d'anisol pendant 1 heures entre 0 et 5°C puis on concentre à sec sous pression réduite (2 mm de mercure; 0,27 kPa) à 35°C. Le résidu est trituré avec 35 $cm^3$ d'oxyde d'isopropyle que l'on évapore sous pression réduite (30 mm de mercure; 4 kPa) à 30°C. Cette opération est répétée encore deux fois puis le résidu est repris par 50 $cm^3$ d'oxyde d'isopropyle. L'insoluble est essoré, lavé par 30 $cm^3$ d'oxyde d'isopropyle est 2 fois 30 $cm^3$ d'éther éthylique et séché. On obtient 2 g de trifluoroacétate de [(D)$\alpha$-aminophénylacétamido]-7 carboxy-2 oxo-8 (phényl-2 thiazolyl-5)-3 thia-5 aza-1 bicyclo[4.2.0] octène-2 sous la forme d'un solide jaune pâle.

Spectre infra-rouge (KBr), bandes caractéristiques ($cm^{-1}$) 3220, 3120—2200, 1770, 1700—1680, 1615, 1520, 1450, 1205, 1140, 800, 720.

Spectre de RMN du proton (350 MHz, DMSO $d_6$, $\delta$ en ppm, J en Hz)

3,71 et 3,80 (2d, J = 18, 2H, —S—$CH_2$);

5,05 (s, 1H, $\mathrm{Ar{-}\underset{|}{C}H{-}CON}\langle$);

5,12 (d, J = 5, 1H, —H en 6); 5,8 (mf, 1H, —H en 7); 7,4 à 7,6 (mt, 8H, aromatiques);
7,88 (d, 2H, H aromatiques en ortho du thiazole); 7,90 (s, 1H, —H du thiazole);
8 à 10 (mf étalé, —$NH_2$, —COOH); 9,64 (d, J = 9, 1H, —CONH— en 7).

Exemple 20

Dans une suspension refroidie à —10°C de 2,53 g (D)N-t.butoxycarbonyl (hydroxy-4 phényl) glycine et de 1,33 $cm^3$ de triéthylamine dans 40 $cm^3$ de tétrahydrofuranne on ajoute 1,3 $cm^3$ de chloroformiate d'isobutyle puis on agite le mélange réactionnel à une température comprise entre —10 et —15°C pendant 1 heures avant d'ajouter en 10 minutes une solution de 3,8 g de trifluoroacétate d'amino-7 carboxy-2 oxo-8 (thiazolyl-5)-3 thia-5 aza-1 bicyclo[4.2.0] octène-2 et de 4 $cm^3$ de triéthylamine dans un mélange de 20 $cm^3$ de tétrahydrofuranne et 20 $cm^3$ d'eau distillée. Le mélange réactionnel est agité pendant 1 heures à température comprise entre 0 et 5°C puis 2 heures à 20°C. Après évaporation du tétrahydrofuranne sous pression réduite (30 mm de mercure; 4 kPa) à 30°C on ajoute 50 $cm^3$ de solution saturée de bicarbonate de sodium et lave la phase aqueuse par 100 $cm^3$ d'acétate d'éthyle avant de l'acidifier à pH 2 par de l'acide chlorhydriques 4N, puis de l'extraire par 2 fois 100 $cm^3$ d'acétate d'éthyle. La phase organique est lavée par 100 $cm^3$ d'eau distillée et par 100 $cm^3$ de solution saturée de chlorure de sodium puis séchée et concentrée à sec sous pression réduite (30 mm de mercure; 4 kPa) à 30°C. On obtient 1,2 g [(D)$\alpha$-t.butoxycarbonylamino (hydroxy-4 phényl) acétamido]-7 carboxy-2 oxo-8 (thiazolyl-5)-3 thia-5 aza-1 bicyclo[4.2.0] octène-2 brut sous la forme d'une merigue jaune.

[Rf=0,35; chromatoplaque de gel de silice, éluant: mélange acétate d'éthyle, acétone, eau, acide formique (50—10—5—5 en volumes)] que l'on purifie par conversion en son ester de benzhydryle de la manière suivante:

On redissout le produit brut dans 25 $cm^3$ d'acétonitrile pour l'estérifier par 0,4 g de diphényldiazométhane pendant 1 heure à 20; après concentration à 10 $cm^3$ sous pression réduite (30 mm de mercure; 4 kPa) à 30°C on reprend dans 100 $cm^3$ d'acétate d'éthyle et lave par 50 $cm^3$ d'acide chlorhydrique 4N puis par 50 $cm^3$ de solution saturée de bicarbonate de sodium et 2 fois par 50 $cm^3$ de solution saturée de chlorure de sodium. Après séchage sur sulfate de magnésium on concentre à sec sous pression réduite (30 mm de mercure; 4 kPa) à 30°C puis on chromatographie sur une colonne (hauteur: 20 cm, diamètre: 2 cm) de gel de silice (0,04—0,06 mm) en éluant sous une pression de 0,4 bar (40 kPa) par un mélange de cyclohexane et d'acétate d'éthyle (50—50 en volumes) et en recueillant des fractions de 50 $cm^3$. Les fractions 11 à 24 contenant le produit pur sont réunies et concentrées à sec sous pression réduite (30 mm de mercure; 4 kPa) à 30°C. On obtient 0,16 g de benzhydryloxycarbonyl-2 [(D)$\alpha$-t.butoxycarbonylamino (hydroxy-4 phényl) acétamido]-7 oxo-8 (thiazolyl-5)-3 thia-5 aza-1 bicyclo[4.2.0] octène-2 sous la forme d'un solide jaune amorphe.

Spectre infra-rouge ($CHBr_3$), bandes caractéristiques ($cm^{-1}$) 3560, 3400, 3330, 1785, 1720, 1690, 1610, 1595, 1490, 1450, 1390, 1365, 1220, 755.

Spectre de RMN du proton (350 MHz, $CDCl_3$, $\delta$ en ppm, J en Hz)

1,45 (s, 9H, —$C(CH_3)_3$); 3,43 et 3,63 (2d, J = 18, 2H, —S—$CH_2$—); 5,03 (d, J = 5, 1H, —H en 6);

5,13 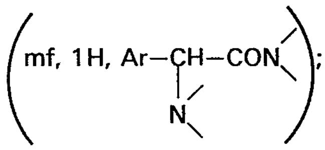


5,60 (mf, 1H, –NH–COO–); 5,87 (dd, J = 5 et 9 et mf, 2H, –H en 7 et –OH);
6,62 (d, J = 9, 1H, –CO–NH–); 6,77 (d, J = 8, 2H, –H aromatiques en ortho du –OH);
6,90 (s, 1H, –COO–C<u>H</u>$(C_6H_5)_2$); 7 à 7,4 (mt, aromatiques);
7,52 (s, 1H, =CH–N= du thiazole); 8,56 (s, 1H, –N=CH–S– du thiazole).

Selon le mode opératoire de l'exemple 17 on traite 0,16 g de benzhydryloxycarbonyl-2 [(D)α-t.butoxycarbonylamino (hydroxy-4 phényl) acétamido]-7 oxo-8 (thiazolyl-5)-3 thia-5 aza-1 bicyclo[4.2.0] octène-2 par 5 $cm^3$ d'acide trifluoroacétique; on obtient 0,082 g de trifluoroacétate de [(D)α-amino (hydroxy-4 phényl) acétamido]-7 carboxy-2 oxo-8 (thiazolyl-5)-3 thia-5 aza-1 bicyclo[4.2.0] octène-2 sous la forme d'un solide de couleur crème.

Spectre infra-rouge (KBr), bandes caractéristiques ($cm^{-1}$) 3300–2200, 1775, 1675, 1610, 1515, 1200, 1135, 840, 800, 720.

Spectre de RMN du proton (350 MHz, DMSO $d_6$, δ en ppm, J en Hz)

3,73 et 3,82 (2d, J = 18, 2H, $-CH_2-S-$);
4,90 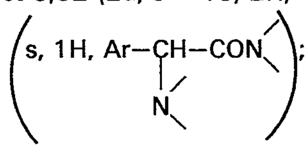


5,15 (s, J = 5, 1H, –H en 6); 5,84 (dd, J = 5 et 9, 1H, –H en 7);
6,80 (s, J = 8, 2H, –H aromatiques en ortho du –OH);
7,30 (d, J = 8, 2H, –H aromatiques en méta du –OH); 7,88 (s, 1H, =CH–N= du thiazole);
8,60 (mf, 3H $-NH_3^{\oplus}$); 9,06 (s, 1H, –N=CH–S–); 9,53 (d, J = 9, 1H, –CO–NH–);
8,90 (mf, 1H, –OH); 13,25 (mf très étalé, –COOH).

En opérant selon le mode opératoire de l'exemple 17 à partir de 3,9 g de trifluoroacétate de [(D)α-amino (hydroxy-4 phényl) acétamido]-7 carboxy-2 oxo-8 (thiazolyl-5)-3 thia-5 aza-1 bicyclo[4.2.0] octène-2 et par traitement par 25 $cm^3$ de résine Amberlite IR 45 (sous forme $OH^-$) dans 100 $cm^3$ d'eau distillée puis filtration et lyophilisation on obtient 1,6 g de [(D)α-amino (hydroxy-4 phényl) acétamido]-7 carboxy-2 oxo-8 (thiazolyl-5)-3 thia-5 aza-1 bicyclo[4.2.0] octène-2 (sel interne) sous forme d'un lyophilisat blanc.

Spectre infra-rouge (KBr), bandes caractéristiques ($cm^{-1}$) 3300–2200, 1765, 1690, 1610, 1520, 1390, 1250, 840.

Le spectre de RMN du proton ($CF_3CO_2D$) est identique à celui du trifluoroacétate pris dans les mêmes conditions.

L'amino-7 carboxy-2 oxo-8 (thiazolyl-5)-3 thia-5 aza-1 bicyclo[4.2.0] octène-2 peut être préparé de la manière suivante:

Selon le mode opératoire décrit dans l'exemple 6 on traite 5,2 g de benzhydryloxycarbonyl-2 t.butoxycarbonylamino-7 oxo-8 (thiazolyl-5)-3 thia-5 aza-1 bicyclo[4.2.0] octène-2 par 80 $cm^3$ d'acide trifluoroacétique. On obtient 4 g de trifluoroacétate d'amino-7 carboxy-2 oxo-8 (thiazolyl-5)-3 thia-5 aza-1 bicyclo[4.2.0] octène-2 sous la forme d'un solide de couleur crème.

Spectre infra-rouge (KBr), bandes caractéristiques ($cm^{-1}$) 3300–2200, 1785, 1680, 1620, 1205, 1180, 1140, 800, 725.

## Exemple 21

On porte au reflux pendant 3 heures une solution de 0,54 g d'(acétylamino-2 thiazolyl-5)-3 benzhydryloxycarbonyl-2 oxo-8 tritylamino-7 oxa-5 aza-1 bicyclo[4.2.0] octène-2 et de 0,14 g d'acide paratoluènesulfonique monohydraté dans 5 $cm^3$ d'acétone. Le mélange réactionnel est dilué par 10 $cm^3$ d'acétate d'éthyle. La solution organique est lavée par 10 $cm^3$ d'une solution demi-saturée de bicarbonate de sodium, puis 10 $cm^3$ de solution saturée de chlorure de sodium, puis séchée sur sulfate de sodium anhydre. On filtre, lave l'insoluble par 2 fois 5 $cm^3$ d'acétate d'éthyle et la solution organique est concentrée à sec sous pression réduite (30 mm de mercure; 4 kPa) à 40°C. Le résidu (0,58 g) est une meringue marron contenant principalement l'amino-7 (acétylamino-2 thiazolyl-5)-3 benzhydryloxycarbonyl-2 oxo-8 oxa-5 aza-1 bicyclo[4.2.0] octène-2 [Rf = 0,11 (chromatoplaque de gel de silice; éluant cyclohexane-acétate d'éthyle 20–80)]. A une solution refroidie à 0°C de 0,58 g du produit précédent, dissous dans 5 $cm^3$ de tétrahydrofuranne sec, on ajoute goutte à goutte pendant 2 minutes 0,117 g de chlorure de thiényl-2 acétyl en solution dans 1 $cm^3$ de tétrahydrofuranne. La solution marron obtenue est laissée sous agitation à 0°C pendant 5 minutes, puis on ajoute goutte à goutte 0,073 g

de triéthylamine en solution dans 1 $cm^3$ de tétrahydrofuranne sec. On laisse sous agitation pendant 1 heures 15 à 0°C. Le mélange est alors filtré et le filtrat est lavé par 25 $cm^3$ d'une solution demi-saturée de bicarbonate de sodium, 25 $cm^3$ d'eau distillée et 25 $cm^3$ de solution saturée de chlorure de sodium. La phase organique est séchée sur sulfate de sodium anhydre; on filtre, puis lave l'insoluble par 2 fois 5 $cm^3$ d'acétate d'éthyle. La solution organique est concentrée à sec sous pression réduite (30 mm de mercure; 4 kPa) à 40°C et le résidu est chromatographié sur gel de silice (0,2—0,06 mm) (diamètre de la colonne: 1,4 cm: hauteur de silice: 15 cm) en éluant par des mélanges cyclohexane-acétate d'éthyle 50—50 (500 $cm^3$), 40—60 (200 $cm^3$), 30—70 (200 $cm^3$) et en recueillant des fractions de 15 $cm^3$. Les fractions 40 à 49 sont réunies et le solvant est évaporé sous pression réduite. On obtient 0,1 g d'(acétylamino-2 thiazolyl-5)-3 benzhydryloxycarbonyl-2 oxo-8 (thiényl-2) acétamido-7 oxa-5 aza-1 bicyclo[4.2.0] octène-2 sous la forme d'une poudre marron.

Spectre de RMN du proton (350 MHz, $CDCl_3$, $\delta$ en ppm, J en Hz)

2,10 (s, 3H, $>N-CO-CH_3$);

3,90 (AB limite, $-CH_2-CO-N<$);

3,80 et 4,20 (mt, $-CH_2-O-$); 5,04 (d, J = 3,5, 1H, —H en 6); 5,94 (dd, J = 3,5 et 10);
6,73 (s, 1H, $-COO-C\underline{H}(C_6H_5)_2$); 6,8 à 6,95 (mt, =CH—CH= du thiophène);
6,9 à 7,6 (mt, aromatiques, —H du thiazole et —S—CH= du thiophène);
7,77 (mf, 1H, —CO—NH—); 13,15 (mf, 1H, $-N\underline{H}-CO-CH_3$).

Une solution de 0,1 g d'(acétylamino-2 thiazolyl-5)-3 benzhydryloxycarbonyl-2 oxo-8 (thiényl-2) acétamido-7 oxa-5 aza-1 bicyclo[4.2.0] octène-2 dans 5 $cm^3$ d'acide formique est chauffée à 50°C avec agitation pendant 30 minutes. Le mélange est concentré à sec sous pression réduite (30 mm de mercure; 4 kPa) à 50°C et le résidu est dissous dans 5 $cm^3$ d'éthanol. La suspension obtenue est agitée à 50°C pendant 5 minutes et le solvant évaporé sous pression réduite (30 mm de mercure; 4 kPa) à 50°C; cette opération est répétée 1 fois et le résidu ainsi obtenu est repris dans 2 $cm^3$ d'éthanol. Le solide en suspension donne, après filtration, 12 mg d'(acétylamino-2 thiazolyl-5)-3 carboxy-2 oxo-8 (thiényl-2) acétamido-7 oxa-5 aza-1 bicyclo[4.2.0] octène-2 sous la forme d'une poudre beige.

Spectre infra-rouge (KBr), bandes caractéristiques ($cm^{-1}$) 3280, 3100, 2220, 1790, 1690, 1655, 1540, 1515, 700.

Spectre de RMN du proton (350 MHz, DMSO $d_6$, $\delta$ en ppm, J en Hz)

2,14 (s, 3H, $>N-CO-CH_3$);

3,76 (s, 2H, $-CH_2-CO-N<$);

4,53 et 4,93 (2d, J = 18, 2H, $-CH_2-O-$); 5,23 (d, J = 3,5, 1H, —H en 6);
5,58 (dd, J = 3,5 et 9,5, 1H, —H en 7); 6,85 à 7 (mt, 2H, =CH—CH= du thiophène);
7,35 (dd, =CH—S— du thiophène); 7,61 (s, 1H, —H du thiazole);
8,97 (d, J = 9,5, 1H, —CO—NH—).

A une solution refroidie à 0°C de 0,80 g d'(amino-2 thiazolyl-5)-3 benzhydryloxycarbonyl-2 oxo-8 tritylamino-7 oxa-5 aza-1 bicyclo[4.2.0] octène-2 dans 10 $cm^3$ de tétrahydrofuranne sec, on ajoute en 3 minutes une solution de 0,11 g de chlorure d'acétyle dans 2 $cm^3$ de tétrahydrofuranne sec, puis une solution de 0,18 $cm^3$ de triéthylamine dans 2 $cm^3$ de tétrahydrofuranne sec. Le mélange est agité pendant 30 minutes à 0°C, puis filtré sur poudre de diatomées. Le filtrat est dilué par 50 $cm^3$ d'acétate d'éthyle et lavé par 50 $cm^3$ d'eau distillée, 50 $cm^3$ d'une solution demi-saturée de bicarbonate de sodium et 50 $cm^3$ de solution saturée de chlorure de sodium. La phase organique est séchée sur sulfate de sodium anhydre; on filtre, lave l'insoluble par 10 $cm^3$ d'acétate d'éthyle, et la solution organique est concentrée sous pression réduite (30 mm de mercure; 4 kPa) à 40°C. Le résidu (0,89 g) est chromatographié sur une colonne de silice (0,04—0,06 mm) (hauteur: 20 cm; diamètre: 2,2 cm) en éluant par un mélange cyclohexane-acétate d'éthyle 40—60 (en volumes) sous 0,4 kPa et en recueillant des fractions de 60 $cm^3$. Les fractions 5 à 11 sont réunies et le solvant est évaporé sous pression réduite (30 mm de mercure; 4 kPa) à 40°C. On obtient 0,55 g d'(acétylamino-2 thiazolyl-5)-3 benzhydryloxycarbonyl-2 oxo-8 tritylamino-7 oxa-5 aza-1 bicyclo[4.2.0] octène-2 sous la forme d'une meringue marron clair.

Spectre infra-rouge (KBr), bandes caractéristiques ($cm^{-1}$) 3340, 1790, 1725, 1700, 1540, 1210.

Spectre de RMN du proton (350 MHz, $CDCl_3$, $\delta$ en ppm, J en Hz)

2,16 (s, 3H, $>N-CO-CH_3$);

3,14 (d, J = 10, 1H, $-N\underline{H}-C(C_6H_5)_3$); 3,89 (d, J = 3,5, 1H, —H en 6);

4,05 et 4,48 (d, J = 18, 2H, $-CH_2-O-$); 4,43 (dd, J = 10 et 3,5, 1H, –H en 7);
6,86 (s, 1H, $-COOC\underline{H}(C_6H_5)_2$; 7,10 (s, 1H, –H du thiazole);
7,10 à 7,65 (mt, aromatiques); 10,93 (mf, 1H, $-NH-CO-CH_3$).

A une solution refroidie à –78°C de 7,14 g de benzhydryloxycarbonyl-2 (diméthylamino-2 vinyl)-3 oxo-8 tritylamino-7 oxa-5 aza-1 bicyclo[4.2.0] octène-2, isomère E, dans 50 $cm^3$ de tétrahydrofuranne sec, on ajoute en 10 minutes 1,72 g de brome en solution dans 7 $cm^3$ de chlorure de méthylène sec; on agite pendant 10 minutes à –78°C puis on laisses remonter la température jusqu'à –40°C et ajoute 0,39 $cm^3$ d'eau distillée. Après 10 minutes, on prélève une fraction aliquote de 5 $cm^3$ du mélange réactionnel, que l'on dilue dans 10 $cm^3$ d'acétate d'éthyle. Cette phase organique est lavée par 5 $cm^3$ d'eau distillée, puis par 5 $cm^3$ d'une solution demi-saturée de chlorure de sodium. Elle est alors séchée sur sulfate de sodium anhydre et concentrée à sec sous pression réduite (30 mm de mercure; 4 kPa) à 20°C. Le résidu (0,61 g) est chromatographié sur une colonne (hauteur: 21 cm, diamètre: 1,8 cm) de gel de silice (0,04—0,06 mm) en éluant sous 40 kPa par un mélange cyclohexane-acétate d'éthyle 75—25 (en volumes) et en recueillant des fractions de 25 $cm^3$. Les fractions 3 et 4 contenant le mélange de épimères de benzhydryloxycarbonyl-2 (bromo-1 oxo-2 éthyl)-3 oxo-8 tritylamino-7 oxa-5 aza-1 bicyclo[4.2.0] octène-2 sont rassemblées et concentrées à sec sous pression réduite (30 mm de mercure; 4 kPa) à 20°C. On obtient 0,03 g de mélange des deux bromoaldéhydes, sous la forme d'une meringue marron.

Position des pics caractéristiques du spectre de RMN du proton (350 MHz, $CDCl_3$, $\delta$ en ppm, J en Hz)

6,22 et 6,41 (s, 2H, >CHBr);

9,27 (s, 2H, $-C\underline{H}O$).

Au mélange réactionnel initial maintenu à –20°C, on ajoute en 10 minutes une solution de 1,23 g de thiourée dans un mélange de 8 $cm^3$ de tétrahydrofuranne et 1,6 $cm^3$ d'eau, puis on laisse remonter la température à 20°C en agitant pendant 30 minutes. Le mélange est alors transféré dans une ampoule à décanter contenant 300 $cm^3$ d'acétate d'éthyle, lavé par 250 $cm^3$ d'une solution demi-saturée de bicarbonate de sodium, 250 $cm^3$ d'eau et 250 $cm^3$ de solution saturée de chlorure de sodium. La phase organique est alors séché sur sulfate de sodium anhydre; on filtre et le filtrat est concentré à sec sous pression réduite (30 mm de mercure; 4 kPa) à 30°C. Le résidu (6,48 g) est chromatographié sur gel de silice (0,04—0,06 mm) en éluant sous 40 kPa par un mélange cyclohexane-acétate d'éthyle 30—70 (en volumes) et en recueillant des fractions de 60 $cm^3$. Les fractions 12 à 21 contenant le produit sont réunies et l'éluant est évaporé sous pression réduite (30 mm de mercure; 4 kPa) à 30°C. On obtient 0,65 g d'(amino-2 thiazolyl-5)-3 benzhydryloxycarbonyl-2 oxo-8 tritylamino-7 oxa-5 aza-1 bicyclo[4.2.0] octène-2 sous la forme d'une meringue marron clair.

Spectre infra-rouge (KBr), bandes caractéristiques ($cm^{-1}$) 1790, 1725, 1490, 1220, 745, 700.

Spectre de RMN du proton (350 MHz, $CDCl_3$, $\delta$ en ppm, J en Hz)

3,12 (d, J= 10, 1H, $-N\underline{H}-C(C_6H_5)_3$); 3,85 (d, J = 3,5, 1H, –H en 6);
4,05 et 4,50 (2d, J = 18, 2H, $-CH_2-O-$); 4,39 (dd, J = 3,5 et 10, 1H, –H en 7);
4,88 (mf, 2H, $-NH_2$); 6,91 (s, $-COOC\underline{H}(C_6H_5)_2$, –H du thiazole); 7,10 à 7,65 (mt, aromatiques).

Le benzhydryloxycarbonyl-2 (diméthylamino-2 vinyl)-3 oxo-8 tritylamino-7 oxa-5 aza-1 bicyclo[4.2.0] octène-2, forme E peut être obtenu de la manière suivante:

On chauffe à 80°C, sous azote, une solution de 4,25 g de benzhydryloxycarbonyl-2 méthyl-3 oxo-8 tritylamino-7 oxa-5 aza-1 bicyclo[4.2.0] octène-2 dans 20 $cm^3$ de diméthylformamide. On ajoute, goutte à goutte pendant 7 minutes, dans la solution maintenue sous agitation à 80°C, 1,55 $cm^3$ de t.butoxy bis-diméthylaminométhane. L'addition terminée, le mélange est conservé sous agitation à 80°C pendant 17 minutes. La solution est diluée par 150 $cm^3$ d'acétate d'éthyle, la phase organique est lavée par 3 fois 60 $cm^3$ d'eau distillée et 60 $cm^3$ d'une solution demi-saturée de chlorure de sodium, séchée sur sulfate de sodium, filtrée et concentrée à sec à 30°C sous pression réduite (20 mm de mercure; 2,7 kPa). Le résidu est trituré dans 150 $cm^3$ d'éther éthylique, la suspension obtenu est filtrée et le filtrat est concentré à sec à 30°C sous pression réduite (20 mm de mercure; 2,7 kPa). On obtient 3,14 g d'un produit brut constitué principalement de benzhydryloxycarbonyl-2 (diméthylamino-2 vinyl)-3 oxo-8 tritylamino-7 oxa-5 aza-1 bicyclo[4.2.0] octène-2, forme E utilisable sans purification supplémentaire.

Spectre infra-rouge (KBr), bandes caractéristiques ($cm^{-1}$) 1780, 1660, 1615, 1490, 1450, 745, 700.

Spectre de RMN du proton (350 MHz, $CDCl_3$, $\delta$ en ppm, J en Hz)

2,77 (s, 6H, $-N(CH_3)_2$); 3,71 (d, J = 3,5, 1HG, H en 6);
4,12 et 4,53 (2d, J = 17, 2H, $-CH_2-O-$); 4,26 (mf, 1H, H en 7);
6,24 et 6,40 (2d, J = 13, 2H, $-CH{=}CH-$); 6,81 (s, 1H, $-COO-C\underline{H}(C_6H_5)_2$).

7,74 g benzhydryloxycarbonyl-2 méthyl-3 oxo-8 tritylamino-7 oxa-5 aza-1 bicyclo[4.2.0] octène-2 sont préparés selon un schéma de synthèse décrit dans le brevet américain 4 108 992 dans lequel on remplace le glyoxylate de t.butyle par le glyoxylate de benzhydryle préparé selon le brevet français 1 495 047.

L'oxacéphalosporine attendue est obtenue sous la forme d'un solide blanc à partir de 13,2 g de tritylamino-3 (propyne-2 yloxy)-4 oxo-2 azétidine.

Spectre infra-rouge (KBr), bandes caractéristiques ($cm^{-1}$) 3340, 1780, 1715, 1620, 1595, 1585, 1490, 1450, 1220, 745, 700.

Spectre de RMN du proton (350 MHz, $CDCl_3$, $\delta$ en ppm, J en Hz)

1,90 (s, 3H, $-CH_3$); 3,75 (d, J = 3,5, 1H, H en 6);
3,87 et 4,08 (2d, J = 18, 2H, $-CH_2-O-$);
4,30 (d, J = 3,5, 1H, H en 7); 6,85 (s, 1H, $-COO-C\underline{H}(C_6H_5)_2$);
7,15 à 7,4 (mt, 26H, aromatiques et $-\underline{H}N-(C_6H_5)_3$).

Exemple 22

A une solution refroidie à 4°C de 10 g d'(amino-2 thiazolyl-5)-3 benzhydryloxycarbonyl-2 [(D) $\alpha$-t.butoxycarbonylaminophénylacétamido]-7 oxo-8 thia-5 aza-1 bicyclo[4.2.0] octène-2 dans 140 $cm^3$ de tétrahydrofuranne sec on ajoute en 5 minutes une solution de 1,22 $cm^3$ de chlorure d'acétyle dans 10 $cm^3$ de tétrahydrofuranne sec puis, après 5 minutes, une solution de 2,15 $cm^3$ de triéthylamine dans 5 $cm^3$ de tétrahydrofuranne. Le mélange réactionnel est agité pendant 4 heures vers 5°C puis filtré, concentré partiellement et versé dans un mélange de 200 $cm^3$ d'acétate d'éthyle et 200 $cm^3$ de solution demi-saturée de bicarbonate de sodium; la phase organique est lavée par 2 fois 100 $cm^3$ d'eau et par 100 $cm^3$ de solution saturée de chlorure de sodium puis séchée sur sulfate de magnésium. Après évaporation du solvant sous pression réduite (30 mm de mercure; 4 kPa) à 40°C, on cristallise le résidu dans 80 $cm^3$ d'acétonitrile. On obtient 2,6 g d'(acétylamino-2 thiazolyl-5)-3 benzhydryloxycarbonyl-2 [(D) $\alpha$- t.butoxycarbonylaminophénylacétamido]-7 oxo-8 thia-5 aza-1 bicyclo[4.2.0] octène-2 sous la forme d'une poudre cristalline beige clair dont les caractéristiques sont identiques à celles du produit obtenu à l'exemple 17.

En opérant comme à l'exemple 17, on peut obtenir ensuite l'(acétylamino-2 thiazolyl-5)-3 carboxy-2 [(D) $\alpha$- t.butoxycarbonylaminophénylacétamido]-7 oxo-8 thia-5 aza-1 bicyclo[4.2.0] octène-2.

A une solution refroidie à −60°C de 11,3 g de benzhydryloxycarbonyl-2 [(D) $\alpha$- t.butoxycarbonylaminophénylacétamido]-7 (diméthylamino-2 vinyl)-3 oxo-8 thia-5 aza-1 bicyclo[4.2.0] octène-2, isomère E, dans 80 $cm^3$ de tétrahydrofuranne sec on ajoute en 10 minutes 2,69 g de brome en solution dans 5 $cm^3$ de chlorure de méthylène sec. On agite pendant 45 minutes en laissant remonter la température jusqu'à −15°C puis ajoute un mélange de 0,6 $cm^3$ d'eau distillée et de 3 $cm^3$ de tétrahydrofuranne. Après 30 minutes à −15°C on ajoute une solution de 1,93 g de thiourée dans un mélange de 18 $cm^3$ de tétrahydrofuranne et 4 $cm^3$ d'eau. Le mélange réactionnel est agité pendant 4 heures entre 5 et 20°C puis dilué par 500 $cm^3$ d'acétate d'éthyle et lavé par 300 $cm^3$ de solution demi-saturée de bicarbonate de sodium, 300 $cm^3$ d'eau et 300 $cm^3$ de solution demi-saturée de chlorure de sodium. Le résidu obtenu [après séchage de la solution sur sulfate de sodium, filtration et évaporation sous pression réduite (30 mm de mercure; 4 kPa) à 40°C] est chromatographié sur une colonne (hauteur: 30 cm; diamètre: 5 cm) de silice (0,04—0,06 mm) en éluant sous 0,4 bar (40 kPa) par un mélange de cyclohexane et d'acétate d'éthyle 35—65 (en volumes) et en recueillant des fractions de 100 $cm^3$. Les fractions 18 à 31 contenant le produit pur sont rassemblées et concentrées à sec sous pression réduite (30 mm de mercure; 4 kPa) à 40°C. On obtient 2,08 g d'(amino-2 thiazolyl-5)-3 benzhydryloxycarbonyl-2 [(D) $\alpha$- t.butoxycarbonylaminophénylacétamido]-7 oxo-8 thia-5 aza-1 bicyclo[4.2.0] octène-2 sous la forme d'une meringue orangée.

Spectre infra-rouge ($CHBr_3$), bandes caractéristiques ($cm^{-1}$) 3480, 3390, 3200, 1785, 1715, 1695, 1600, 1495, 1455, 1390, 1370, 1220, 760, 740.

Spectre de RMN du proton (350 MHz, $CDCl_3$, $\delta$ en ppm, J en Hz)

1,43 (s, 9H, $-C(CH_3)_3$); 3,36 et 3,55 (2d, J = 18, 2H, $-S-CH_2-$); 4,94 (s large, 2H, $-NH_2$);
4,98 (d, J = 5, 1H, −H en 6);

5,24 (mf, 1H, >N−CH−CO−N<);

5,67 (d, J = 6, 1H, −NH−C(=O)−O−);

5,83 (dd, J = 5 et 9, 1H, −H en 7); 6,73 (mf, 1H, −CO−NH−); 6,83 (s, 1H, $-COO-C\underline{H}(C_6H_5)_2$);
6,94 (s, 1H, −H thiazole); 7,10 à 7,50 (mt, 15H, aromatiques).

Exemple 23

A une solution de 2,05 g d'(amino-2 thiazolyl-5)-3 benzhydryloxycarbonyl-2 (dichloro-3,4 phénylthio)acétamido-7 oxo-8 thia-5 aza-1 bicyclo[4.2.0] octène-2 dans 60 $cm^3$ de tétrahydrofuranne refroidie à 3°C, on ajoute 4,02 g de chlorhydrate du chlorure de tritylaminoacétyle puis en 5 minutes 3,36 $cm^3$ de triéthylamine. Après 30 minutes à 3°C le mélange réactionnel est dilué par 250 $cm^3$ d'acétate d'éthyle, lavé successivement par 150 $cm^3$ d'eau distillée, 100 $cm^3$ d'acide chlorhydrique 0,1 N, 100 $cm^3$ de solution saturée de bicarbonate de sodium, 100 $cm^3$ d'eau distillée, 100 $cm^3$ de solution saturée de chlorure de sodium. La phase organique séchée sur sulfate de sodium est concentrée à sec sous pression réduite (30 mm de mercure; 4 kPa) à 40°C et le résidu est chromatographié sur une colonne (hauteur: 30 cm, diamètre: 4 cm) de gel de silice (0,02—0,04 mm) en éluant sous une pression de 0,5 bar (50 kPa) par un mélange de cyclohexane et d'acétate d'éthyle (65/35 en volumes) et en recueillant des fractions de 60 $cm^3$. Les fractions 4 à 35 contenant le produit pur sont réunies et concentrées à sec sous pression réduite (30 mm de mercure; 4 kPa) à 30°C, pour donner 1,51 g de benzhydryloxycarbonyl-2 (dichloro-3,4 phénylthio)acétamido-7 oxo-8 (tritylaminoacétamido-2 thiazolyl-5)-3 thia-5 aza-1 bicyclo[4.2.0] octène-2 sous forme d'une poudre blanche.

Spectre infra-rouge ($CHBr_3$), bandes caractéristiques ($cm^{-1}$) 3350, 1785, 1725, 1685, 1535, 1500, 1460, 1450, 860, 810, 750.

On traite 1,61 g de benzhydryloxycarbonyl-2 (dichloro-3,4 phénylthio)acétamido-7 oxo-8 (tritylaminoacétamido-2 thiazolyl-5)-3 thia-5 aza-1 bicyclo[4.2.0] octène-2 par 30 $cm^3$ d'acide formique et 3 $cm^3$ d'eau distillée à 50°C pendant 30 minutes, puis concentre à sec sous pression réduite ( 2mm de mercure; 0,27 kPa) à 40°C. Le résidu est traité selon le mode opératoire décrit dans l'exemple 24 pour donner 0,46 g de carboxy-2 (dichloro-3,4 phénylthio)acétamido-7 (glycylamino-2 thiazolyl-5)-3 oxo-8 thia-5 aza-1 bicyclo[4.2.0] octène-2 sous le forme d'une poudre jaune.

Spectre de RMN du proton (250 MHz, DMSO $d_6$, $\delta$ en ppm, J en Hz)

3,70 $\left(\text{AB limite, 2H, } -\underset{\underset{O}{\|}}{C}-CH_2-N\langle\right)$;

3,75 à 3,95 $\left(\text{mt, 4H, } -S-CH_2- \text{ et } -S-CH_2-\underset{\underset{O}{\|}}{C}-N\langle\right)$;

5,07 (d, J = 5, 1H, —H en 6); 5,57 (dd, J = 8 et 5, 1H, —H en 7);
7,34 (dd, J = 8,5 et 2, 1H, —H aromatique en 6); 7,46 (s, 1H, —H du thiazole);
7,55 (d, J = 8,5, 1H, —H aromatique en 5); 7,65 (d, J = 2, 1H, —H aromatique en 2);

9,20 $\left(\text{d, J = 8, 1H, } -\underset{\underset{O}{\|}}{C}-NH-\right)$.

L'(amino-2 thiazolyl-5)-3 benzhydryloxycarbonyl-2 (dichloro-3,4 phénylthio)acétamido-7 oxo-8 thia-5 aza-1 bicyclo[4.2.0] octène-2 peut être préparé de la manière suivante:

A une solution de 11,17 g de benzhydryloxycarbonyl-2 (chloracétamido-2 thiazolyl-5)-3 (dichloro-3,4 phénylthio)acétamido-7 oxo-8 thia-5 aza-1 bicyclo[4.2.0] octène-2 dans 100 $cm^3$ de tétrahydrofuranne, on ajoute 1,34 g de bicarbonate de sodium puis une solution de 1,21 g de thiourée dans un mélange de 10 $cm^3$ de tétrahydrofuranne et de 10 $cm^3$ d'eau distillée et agite 3 jours à 25°C. Le mélange réactionnel est dilué par 300 $cm^3$ d'acétate d'éthyle, lavé 2 fois par 200 $cm^3$ d'eau distillée et par 100 $cm^3$ de solution saturée de chlorure des sodium. La phase organique est séchée sur sulfate de sodium et concentrée à sec sous pression réduite (30 mm de mercure; 4 kPa) à 40°C. Le résidu est chromatographié sur une colonne (diamètre: 3 cm) de 140 g de gel de silice (0,05—0,2 mm) en éluant par un mélange de cyclohexane et d'acétate d'éthyle (20/80 en volumes) et en recueillant des fractions de 120 $cm^3$. Les fractions 5 à 14 sont réunies et concentrées à sec sous pression réduite (30 mm de mercure; 4 kPa) à 30°C pour donner 8 g d'(amino-2 thiazolyl-5)-3 benzhydryloxycarbonyl-2 (dichloro-3,4 phénylthio)acétamido-7 oxo-8 thia-5 aza-1 bicyclo[4.2.0] octène-2 sous la forme d'une meringue jaune pâle.

Rf = 0,26 (chromatoplaque de gel de silice éluée par un mélange de cyclohexane et d'acétate d'éthyle 20/80 en volumes).

Exemple 24

A une solution de 0,77 g de benzhydryloxycarbonyl-2 (chloracétamido-2 thiazolyl-5)-3 (dichloro-3,4 phénylthio)acétamido-7 oxo-8 oxyde-5 thia-5 aza-1 bicyclo[4.2.0] octène-2 dans 10 $cm^3$ de N,N

diméthylformamide à 20°C, on ajoute 0,17 $cm^3$ de N,N diisopropyléthylamine et 0,265 g de N t.butoxycarbonylcystéamine en solution dans 5 $cm^3$ de N,N diméthylformamide. Le mélange réactionnel est agité 17 heures à 20°C puis dilué par 100 $cm^3$ d'acétate d'éthyle et 100 $cm^3$ d'eau distillée. La phase organique est lavée par 100 $cm^3$ de solution saturée de chlorure de sodium, séchée sur sulfate de sodium et concentrée à sec sous pression réduite (30 mm de mercure; 4 kPa) à 40°C. Le solide jaune obtenu est concentré par 10 $cm^3$ d'éther éthylique filtré et cristallisé de 10 $cm^3$ d'acétonitrile pour donner 0,42 g de benzhydryloxycarbonyl-2 [(t.butoxycarbonylamino-2 éthylthioacétamido)-2 thiazolyl-5]-3 (dichloro-3,4 phénylthio)acétamido-7 oxo-8 oxyde-5 thia-5 aza-1 bicyclo[4.2.0] octène-2 sous la forme d'un solide cristallin jaune.

Spectre infra-rouge (KBr), bandes caractéristiques ($cm^{-1}$) 1795, 1720, 1690, 1540, 1510, 1170, 870, 815, 760, 705.

Spectre de RMN du proton (250 MHz, DMSO $d_6$, $\delta$ en ppm, J en Hz)

1,39 (s, 9H, $-C(CH_3)_3$); 2,67 (t, J = 7, 2H, $-S-CH_2-CH_2-$);
3,17 (td, J = 7 et 6, 2H, $-S-CH_2-CH_2-NH-$);
3,42 (s, 2H, $>N-CO-CH_2S-CH_2-$);
3,74 et 4,31 (2d, J = 18, 2H, $-S-CH_2-$);
3,9 et 4,01 (2d, J = 15, 2H, $-Ar-S-CH_2-CO-N<$);
5,01 (d, J = 5, 1H, –H en 6); 5,97 (dd, J = 5 et 9, 1H, –H en 7);
6,87 (s, 1H, $-COO-CH(C_6H_5)_2$); 6,93 (t, J = 6, 1H, $-CH_2-NH-COO$);
7,05 à 7,35 (mt, 10H, aromatiques);
7,36 (s et dd, J = 8,5 et 2, 2H, –H du thiazole et –H aromatique en 6 respectivement);
7,55 (d, J = 8,9, 1H, –H aromatique en 5); 7,67 (d, J = 2, 1H, –H aromatique en 2);
8,71 (d, J = 9, $-C(=O)-NH-$);
12,26 (mf, 1H, –NHCO–).

Une solution de 3,08 g de benzhydryloxycarbonyl-2 [(t.butoxycarbonylamino-2 éthylthioacétamido)-2 thiazolyl-5]-3 (dichloro-3,4 phénylthio)acétamido-7 oxo-8 oxyde-5 thia-5 aza-1 bicyclo[4.2.0] octène-2 dans un mélange de 50 $cm^3$ de dichlorométhane et 5 $cm^3$ de N,N diméthylacétamide refroidie à 0°C est traitée par 0,9 g de trichlorure de phosphore pendant 30 minutes. Le mélange réactionnel est versé dans un mélange de 100 $cm^3$ d'eau distillée et 200 $cm^3$ d'acétate d'éthyle. La phase organique est lavée par 100 $cm^3$ de solution saturée de chlorure de sodium, séchée sur sulfate de sodium et concentrée à sec sous pression réduite (30 mm de mercure; 4 kPa) à 40°C. Le résidu est chromatographié sur une colonne (diamètre: 2 cm) de 70 g de gel de silice (0,05–0,2 mm) en éluant par un mélange de cyclohexane et d'acétate d'éthyle (20/80 en volumes) et en recueillant des fractions de 60 $cm^3$. Les fractions 2 à 6 sont réunies et concentrées à sec sous pression réduite (30 mm de mercure; 4 kPa) à 30°C pour donner 1,7 g de benzhydryloxycarbonyl-2 [(t.butoxycarbonylamino-2 éthylthioacétamido)-2 thiazolyl-5]-3 (dichloro-3,4 phénylthio)acétamido-7 oxo-8 thia-5 aza-1 bicyclo[4.2.0] octène-2 sous la forme d'une meringue jaune.

Rf = 0,33 (chromatoplaque de gel de silice éluant acétate d'éthyle).

Spectre infra-rouge (KBr), bandes caractéristiques ($cm^{-1}$) 1795, 1720, 1690, 1540, 1510, 870, 815, 760, 705.

Spectre de RMN du proton (250 MHz, $CDCl_3$, $\delta$ en ppm, J en Hz)

1,44 (s, 9H, $-C(CH_3)_3$); 2,77 (t, J = 7, 2H, $-S-CH_2-CH_2-$);
3,36 (mt, 2H, $-S-CH_2-CH_2-NH-$);
3,38 (s, 2H, $>N-C(=O)-CH_2-S-CH_2-$);
3,51 et 3,65 (2d, J = 18, 2H, $-S-CH_2-$);
3,66 et 3,76 (2d, J = 15, 2H, $Ar-S-CH_2-C(=O)-N<$);

5,02 (d, J = 5, 1H, –H en 6); 5,1 (mf, 1H, $-CH_2-N\underline{H}-COO-$); 5,86 (dd, J = 9 et 5, 1H, –H en 7); 6,91 (s, 1H, $-COO-C\underline{H}(C_6H_5)_2$);

7,05 à 7,35 (mt, aromatiques, –H aromatique en 6, >NH);

7,11 (s, –H du thiazole); 7,38 (d, J = 8,5, 1H, –H aromatique en 5);

7,48 (d, J = 2, 1H, –H aromatique en 2);

7,83 (d, J = 9, 1H, $-\underset{\underset{O}{\|}}{C}-NH-$).

Une solution de 1,7 g de benzhydryloxycarbonyl-2 [(t.butoxycarbonylamino-2 éthylthioacétamido)-2 thiazolyl-5]-3 (dichloro-3,4 phénylthio)acétamido-7 oxo-8 thia-5 aza-1 bicyclo[4.2.0] octène-2 dans 50 $cm^3$ d'acide formique est chauffée à 50°C pendant 30 minutes puis concentrée à sec sous pression réduite (2 mm de mercure; 0,27 kPa) à 40°C. L'huile résiduelle est reprise dans 20 $cm^3$ d'acétone que l'on concentre à sec sous pression réduite (40 mm de mercure; 5,3 kPa) à 50°C; cette opération est répétée une nouvelle fois puis le résidu est repris dans 20 $cm^3$ d'acétone bouillante. On filtre à chaud et lave le solide recueilli par 20 $cm^3$ d'acétone et 20 $cm^3$ d'éther éthylique pour obtenir 1,15 g d'[(amino-2 éthylthioacétamido)-2 thiazolyl-5]-3 carboxy-2 (dichloro-3,4 phénylthio)acétamido-7 oxo-8 thia-5 aza-1 bicyclo[4.2.0] octène-2 sous la forme d'un solide jaune.

Spectre infra-rouge (KBr), bandes caractéristiques ($cm^{-1}$) 3260, 3150, 3100–2200, 1765, 1760, 1600, 1550, 1460, 880, 810.

Spectre de RMN du proton (250 MHz, DMSO $d_6$, $\delta$ en ppm, J en Hz)

2,87 (mt, 2H, $-S-C\underline{H}_2-CH_2-$);

3,09 (mt, 2H, $-S-CH_2-C\underline{H}_2-N<$);

3,42 (s, large, 2H, $>N-\underset{\underset{O}{\|}}{C}-C\underline{H}_2-S-CH_2-$);

3,66 et 3,76 (2d, J = 18, 2H, $-S-CH_2-$);

3,78 et 3,88 (2d, J = 15, 2H, $Ar-S-CH_2-\underset{\underset{O}{\|}}{C}-N<$);

5,06 (d, J = 5, 1H, –H en 6); 5,55 (dd, J = 5 et 9, 1H, –H en 7);

7,33 (dd, J = 8,5 et 2, 1H, –H aromatique en 6); 7,48 (s, 1H, –H thiazole);

7,53 (d, J = 8,5, 1H, –H aromatique en 5); 7,64 (d, J = 2, 1H, –H aromatique en 2);

9,20 (d, J = 9, 1H, $-\underset{\underset{O}{\|}}{C}-NH-$).

Le benzhydryloxycarbonyl-2 (chloracétamido-2 thiazolyl-5)-3 (dichloro-3,4 phénylthio)acétamido-7 oxo-8 oxyde-5 thia-5 aza-1 bicyclo[4.2.0] octène-2 peut être obtenu de la manière suivante:

Une solution de 2 g de benzhydryloxycarbonyl-2 (chloracétamido-2 thiazolyl-5)-3 (dichloro-3,4 phénylthio)acétamido-7 oxo-8 thia-5 aza-1 bicyclo[4.2.0] octène-2 dans 50 $cm^3$ de dichlorométhane est refroidie à 0°C et traitée par une solution de 0,45 g d'acide métachloroperbenzoïque à 85% dans 10 $cm^3$ de dichlorométhane. Après 30 minutes à une température comprise entre 0 et 5°C on dilue le mélange réactionnel par 100 $cm^3$ de dichlorométhane et ajoute 100 $cm^3$ de solution saturée de bicarbonate de sodium. Le solide qui précipite est essoré et repris dans 200 $cm^3$ de dichlorométhane. On ajoute à cette solution la phase organique décantée, lavée par 100 $cm^3$ d'eau puis 100 $cm^3$ de solution saturée de chlorure de sodium et séchée sur sulfate de sodium, puis concentre à sec sous pression réduite jusqu'à un volume résiduel de 10 $cm^3$. Le solide est essoré, lavé par 20 $cm^3$ d'acétate d'éthyle puis par 20 $cm^3$ d'éther éthylique. On obtient 1,48 g de benzhydryloxycarbonyl-2 (chloracétamido-2 thiazolyl-5)-3 (dichloro-3,4 phénylthio)acétamido-7 oxo-8 oxyde-5 thia-5 aza-1 bicyclo[4.2.0] octène-2 sous la forme d'un solide crème.

Spectre infra-rouge (KBr), bandes caractéristiques ($cm^{-1}$) 3280, 3180, 3100, 2500, 1780, 1715, 1685, 1650, 1540, 1510, 1460, 1220, 850, 810, 755, 700.

Spectre de RMN du proton (250 MHz, DMSO $d_6$, $\delta$ en ppm, J en Hz)

3,74 et 4,33 (2d, J = 18, 2H, $-S-CH_2-$);

3,90 et 4,33 $\left(2d, J = 15, 2H, -S-CH_2-CO-N\langle\right)$;

4,38 $\left(s, 2H, \rangle N-CO-CH_2-Cl\right)$;

5,01 (d, J = 5, 1H, –H en 6); 5,98 (dd, J = 5 et 9, 1H, en 7); 6,88 (s, 1H, $-COOC\underline{H}(C_6H_5)_2$); 7,05 à 7,35 (mt, 10H, aromatiques); 7,37 (dd, J = 8,5 et 2, 1H, –H aromatiques en 6); 7,39 (s, 1H, –H du thiazole); 7,56 (d, J = 8,5, 1H, –H aromatiques en 5); 7,67 (d, J = 2, 1H, –H aromatique en 2); 8,72 (d, J = 9, 1H, –CO–NH–);

12,5 $\left(\text{mf étalé}, 1H, \rangle NH\right)$.

Le benzhydryloxycarbonyl-2 (chloracétamido-2 thiazolyl-5)-3 (dichloro-3,4 phénylthio)acétamido-7 oxo-8 thia-5 aza-1 bicyclo[4.2.0] octène-2 peut être obtenu de la manière suivante:

On ajoute en 40 minutes à une suspension de 19,2 g de benzhydryloxycarbonyl-2 t.butoxycarbonylamino-7 (chloracétamido-2 thiazolyl-5)-3 oxo-8 thia-5 aza-1 bicyclo[4.2.0] octène-2 dans 290 $cm^3$ d'acétonitrile à 37°C une solution de 11,4 g d'acide p.toluènesulfonique (hydrate) dans 70 $cm^3$ d'acétonitrile. On agite 3 heures à 40°C puis ajoute 5,7 g d'acide p.toluènesulfonique agite encore 1 heures avant de diluer le mélange réactionnel par 500 $cm^3$ d'acétate d'éthyle et 300 $cm^3$ de solution saturée de bicarbonate de sodium. La phase organique est lavée 2 fois par 250 $cm^3$ d'eau distillée et par 250 $cm^3$ de solution demi-saturée de chlorure de sodium et séchée sur sulfate de sodium puis refroidie à 0°C avant addition de 4,2 $cm^3$ de triéthylamine. On ajoute ensuite en 10 minutes une solution de 7,6 g de chlorure de dichloro-3,4 phénylthioacétyle dans 50 $cm^3$ d'acétate d'éthyle. On laisse agiter pendant 35 minutes en laissant remonter la température jusqu'à 18°C puis dilue par 100 $cm^3$ d'acétate d'éthyle et 400 $cm^3$ d'eau distillée. La couche organique est lavée par un mélange de 150 $cm^3$ de solution saturée de chlorure de sodium et de 150 $cm^3$ de solution saturée de bicarbonate de sodium puis par 300 $cm^3$ d'acide chlorhydrique normal et par 300 $cm^3$ de solution saturée de chlorure de sodium. Après sur sulfate de magnésium, la solution est concentrée à sec sous pression réduite (30 mm de mercure; 4 kPa) à 40°C et le résidu est chromatographié sur une colonne (hauteur: 77 cm, diamètre: 4,5 cm) de gel de silice (0,2–0,06 mm) en éluant par des mélanges de cyclohexane et d'acétate d'éthyle 50/50 en volumes (1,8 litre) puis 20/80 en volumes et en recueillant des fractions de 600 $cm^3$. Les fractions 4 à 6 contenant le produit sont réunies et concentrées sous pression réduite (30 mm de mercure; 4 kPa) à 30°C pour donner 14,6 g de solide que l'on recristallise dans 50 $cm^3$ d'acétonitrile pour obtenir 9,2 g de benzhydryloxycarbonyl-2 (chloroacétamido-2 thiazolyl-5)-3 (dichloro-3,4 phénylthio)acétamido-7 oxo-8 thia-5 aza-1 bicyclo[4.2.0] octène-2 sous la forme de cristaux crèmes.

PF = 148°C.

Le benzhydryloxycarbonyl-2 t.butoxycarbonylamino-7 (chloroacétamido-2 thiazolyl-5)-3 oxo-8 thia-5 aza-1 bicyclo[4.2.0] octène-2 peut être obtenu de la manière suivante:

On ajoute à une solution de 60 g d'(amino-2 thiazolyl-4)-3 benzhydryloxycarbonyl-2 t.butoxycarbonylamino-7 oxo-8 thia-5 aza-1 bicyclo[4.2.0] octène-2 dans 350 $cm^3$ de tétrahydrofuranne refroidie à 0°C successivement 12,37 g de chlorure de chloracétyle puis en 6 minutes 16,3 $cm^3$ de triéthylamine. Après 1 heure à 0°C le mélange réactionnel est dilué par 800 $cm^3$ de solution demi-saturée de bicarbonate de sodium et l'on extrait 3 fois par 200 $cm^3$ d'acétate d'éthyle. Les phases organiques jointes sont rassemblées et lavées par 250 $cm^3$ de solution saturée de chlorure de sodium et séchées puis concentrées à sec sous pression réduite (30 mm de mercure; 4 kPa) à 40°C pour donner 78 g de meringue organgée que l'on cristallise dans 300 $cm^3$ d'acétonitrile pour obtenir 37,4 g de cristaux crèmes de benzhydryloxycarbonyl-2 t.butoxycarbonylamino-7 (chloracétamido-2 thiazolyl-4)-3 oxo-8 thia-5 aza-1 bicyclo[4.2.0] octène-2.

PF = 244°C.

## Exemple 25

En traitant selon le mode opératoire de l'exemple 24 4,2 g de benzhydryloxycarbonyl-2 (chloracétamido-2 thiazolyl-5)-3 (dichloro-3,4 phénylthio)acétamido-7 oxo-8 oxyde-5 thia-5 aza-1 bicyclo[4.2.0] octène-2 par 3,13 g de (L) N-t.butoxycarbonylaminocystéinate de benzhydryle en présence de 0,93 $cm^3$ de N,N diisopropyléthylamine on obtient 7,61 g de benzhydryloxycarbonyl-2 {[(L) benzhydryloxycarbonyl-2 t.butoxycarbonylamino-2 éthylthioacétamido]-2 thiazolyl-5}-3 (dichloro-3,4 phénylthio)acétamido-7 oxo-8 oxyde-5 thia-5 aza-1 bicyclo[4.2.0] octène-2 brut sous la forme d'une huile brune que l'on réduit par action de 0,87 $cm^3$ de trichlorure de phosphore selon le mode opératoire de l'exemple 24 pour obtenir 3,8 g de benzhydryloxycarbonyl-2 {[(L) benzhydryloxycarbonyl-2 t.butoxycarbonylamino-2 éthylthioacétamido]-2 thiazolyl-5}-3 (dichloro-3,4 phénylthio)acétamido-7 oxo-8 thia-5 aza-1 bicyclo[4.2.0] octène-2 sous la forme d'une meringue jaune.

Rf = 0,63 (chromatoplaque de gel de silice éluée par un mélange de cyclohexane et d'acétate d'éthyle 20/80 en volumes).

3,80 g de benzhydryloxycarbonyl-2 {[(L) benzhydryloxycarbonyl-2 t.butoxycarbonylamino-2 éthylthioacétamido]-2 thiazolyl-5}-3 (dichloro-3,4 phénylthio)acétamido-7 oxo-8 thia-5 aza-1 bicyclo[4.2.0] octène-2 sont traités par 50 $cm^3$ d'acide formique selon le mode opératoire décrit dans l'exemple 24 pour donner 1,79 g de {[(L)amino-2 carboxy-2 éthylthioacétamido]-2 thiazolyl-5}-3 carboxy-2 (dichloro-3,4 phénylthio)acétamido-7 oxo-8 thia-5 aza-1 bicyclo[4.2.0] octène-2 sous la forme d'une poudre crème.

Spectre infra-rouge (KBr), bandes caractéristiques ($cm^{-1}$) 3300–2200, 1770, 1660, 1550, 1460, 870, 810.

Spectre de RMN du proton (250 MHz, DMSO $d_6$, $\delta$ en ppm, J en Hz)

2,98 et 3,16 (2dd, J = 14 et 7,5 et J = 14 et 4 respectivement, $-S-C\underline{H}_2-CH(NH_2)COOH$);

3,50 (AB limite, J = 15, 2H, $-S-CH_2-CO-N<$);

3,68 (dd, J = 7,5 et 4, 1H, $-CH(N<)-COO-$);

3,73 et 3,86 (2d, J = 18, 2H, $-S-CH_2-$);

3,77 et 3,86 (2d, J = 15, 2H, $-S-CH_2-C(=O)-N<$);

5,13 (d, J = 5, 1H, –H en 6); 5,67 (dd, J = 5 et 9, 1H, –H en 7);
7,33 (dd, J = 8,5 et 2, 1H, –H aromatique en 6); 7,51 (s, 1H, –H du thiazole);
7,53 (d, J = 8,5, 1H, –H aromatique en 5); 7,63 (d, J = 2, 1H, –H aromatique en 2);

9,25 (d, J = 9, 1H, $-C(=O)-NH-$).

Exemple 26

On agite à 20°C pendant 12 heures un mélange de 1,32 g de benzhydryloxycarbonyl-2 oxo-8 [(pyridyl-3 méthyl)-2 thiazolyl-5]-3 (thiényl-2 acétamido)-7 thia-5 aza-1 bicyclo[4.2.0] octène-2, 1,25 $cm^3$ d'iodure de méthyle et 7 $cm^3$ de diméthylformamide. Le mélange est dilué par 100 $cm^3$ d'oxyde d'éthyle, et le mélange est agité pendant 15 minutes. La liqueur surnageante est décantée, et le résidu est agité pendant 15 minutes avec 100 $cm^3$ d'oxyde d'éthyle. Le solide est filtré, lavé par deux fois 20 $cm^3$ d'oxyde d'éthyle et séché sous pression réduite (0,1 mm de mercure; 0,013 kPa) à 20°C. On obtient ainsi 2,3 g d'iodure de benzhydryloxycarbonyl-2 [(méthyl-1 pyridinio-3) méthyl-2 thiazolyl-5]-3 oxo-8 (thiényl-2 acétamido)-7 thia-5 aza-1 bicyclo[4.2.0] octène-2 sous la forme d'une poudre orangée.

Spectre infra-rouge (KBr), bandes caractéristiques ($cm^{-1}$) 3260, 3180, 1780, 1730, 1665, 1510, 1225, 750, 705, 675.

Spectre de RMN du proton (350 MHz, DMSO $d_6$, $\delta$ en ppm, J en Hz)

3,73 et 3,81 (2d, J = 15, 2H, $-CH_2-C(=O)-N<$);

3,8 et 3,9 (2d, J = 18, 2H, $-S-CH_2-$);

4,36 (s, 3H, $>\overset{+}{N}-CH_3$);

4,44 (s, 2H, $-CH_2-$); 5,23 (d, J = 5, 1H, –H en 6); 5,83 (dd, J = 9 et 5, 1H, –H en 7);
6,86 (s, 1H, $-COO-C\underline{H}(C_6H_5)_2$); 6,9 à 7 (mt, 2H, –H en 3 et –H en 4 du thiophène);
6,9 à 7,4 (mt, aromatiques et –H en 5 du thiophène); 7,64 (s, 1H, –H du thiazole);
8,12 (dd, J = 8 et 5, 1H, –H en 5 de la pyridine); 8,46 (d, J = 8, 1H, –H en 4 de la pyridine);

8,94 (d, J = 5, 1H, –H en 6 de la pyridine); 9,05 (s, 1H, –H en 2 de la pyridine);

9,24 (d, J = 9, 1H, $-\overset{O}{\overset{\|}{C}}-NH-$).

A une solution refroidie à 3°C de 1,5 g d'iodure de benzhydryloxycarbonyl-2 [(méthyl-1 pyridino-3) méthyl-2 thiazolyl-5]-3 oxo-8 (thiényl-2 acétamido)-7 thia-5 aza-1 bicyclo[4.2.0] octène-2 dans 1,8 $cm^3$ d'anisole, on ajoute 18 $cm^3$ d'acide trifluoracétique. Le mélange est agité à 3°C pendant 15 minutes, puis 30 minutes à 20°C. On le concentre sous pression réduite (0,1 mm de mercure; 0,013 kPa) à 20°C. Le résidu est repris dans 50 $cm^3$ d'acétate d'éthyle et la solution concentrée à sec sous pression réduite (0,1 mm de mercure; 0,013 kPa). L'opération est réitérée et le résidu repris dans 50 $cm^3$ d'oxyde d'éthyle. On agite le mélange hétérogène pendant 15 minutes à 22°C, filtre et sèche le solide sous pression réduite (0,1 mm de mercure; 0,013 kPa). On obtient 1,05 g de trifluoracétate de carboxy-2 [(méthyl-1 pyridinio-3 méthyl)-2 thiazolyl-5]-3 oxo-8 (thiényl-2 acétamido)-7 thia-5 aza-1 bicyclo[4.2.0] octène-2 sous la forme d'une poudre jaune.

Spectre infra-rouge (KBr), bandes caractéristiques ($cm^{-1}$) 3300, 1780, 1680, 1510, 1180, 800, 720, 680.

Spectre de RMN du proton (350 MHz, DMSO $d_6$, $\delta$ en ppm, J en Hz)

3,7 à 3,85 (mt, 4H, $-CH_2-\overset{O}{\overset{\|}{C}}-N<$ et $-S-CH_2$);

4,35 (s, 3H, $>\overset{+}{N}-CH_3$);

4,62 (s, 2H, $-CH_2-$); 5,18 (d, J = 5, 1H, –H en 6); 5,75 (dd, J = 8 et 5, 1H, –H en 7);
6,9 à 7 (mt, 2H, –H en 3 et –H en 4 du thiophène);
7,34 (dd, J = 5 et 1, 1H, –H en 5 du thiophène); 7,75 (s, 1H, –H du thiazole);
8,11 (dd, J = 8 et 5, 1H, –H en 5 de la pyridine); 8,56 (d, J = 8, 1H, –H en 4 de la pyridine);
8,92 (d, J = 5, 1H, –H en 6 de la pyridine); 9,08 (s large, 1H, –H en 2 de la pyridine);

9,19 (d, J = 8, 1H, $-\overset{O}{\overset{\|}{C}}-NH-$).

Le benzhydryloxycarbonyl-2 oxo-8 [(pyridyl-3 méthyl)-2 thiazolyl-5]-3 (thiényl-2 acétamido)-7 thia-5 aza-1 bicyclo[4.2.0] octène-2 peut être préparé de la manière suivante:

A une solution de 6,36 g d'amino-7 benzhydryloxycarbonyl-2 oxo-8 [(pyridyl-3 méthyl)-2 thiazolyl-5]-3 thia-5 aza-1 bicyclo[4.2.0] octène-2 dans 200 $cm^3$ de tétrahydrofuranne sec, on ajoute 1,4 $cm^3$ de chlorure de thiényl-2 acétyle puis 1,58 $cm^3$ de triéthylamine. On agite le mélange à 20°C pendant 1 heure 20 minutes, puis on le verse dans un mélange acétate d'éthyle-solution saturée de bicarbonate de sodium 65–35 (en volumes). La phase organique est décantée, puis lavée successivement par 200 $cm^3$ d'eau distillée, 200 $cm^3$ d'une solution saturée de chlorure de sodium. Elle est séchée sur sulfate de sodium anhydre. Le séchant est filtré, lavé par 10 $cm^3$ d'acétate d'éthyle et le filtrat est concentré à sec sous pression réduite (20 mm de mercure; 2,7 kPa) à 30°C. Le résidu est purifié par chromatographie sur une colonne (hauteur: 24 cm, diamètre: 3 cm) de gel de silice (0,05–0,2 mm) en éluant par 2,5 litres d'acétate d'éthyle et en recueillant des fractions de 60 $cm^3$. Les fractions 12 à 35 sont réunies et concentrées à sec sous pression réduite (30 mm de mercure; 4 kPa) à 30°C pour donner 4,45 g de benzhydryloxycarbonyl-2 oxo-8 [(pyridyl-3 méthyl)-2 thiazolyl-5]-3 (thiényl-2 acétamido)-7 thia-5 aza-1 bicyclo[4.2.0] octène-2 sous la forme d'une meringue jaune.

Spectre infra-rouge (KBr), bandes caractéristiques ($cm^{-1}$) 3300, 3200, 1785, 1730, 1685, 1580, 1520, 1495, 1480, 1450, 1425, 1225, 760, 700.

Spectre de RMN du proton (250 MHz, DMSO $d_6$, $\delta$ en ppm, J en Hz)

3,78 (AB limite, J = 16, 2H, $-CH_2-CO-N<$);

3,78 et 3,9 (2d, J = 18, 2H, $-S-CH_2-$); 4,14 et 4,24 (2d, J = 17, 2H, $-CH_2-$);
5,21 (d, J = 5, 1H, –H en 6); 5,83 (dd, J = 8 et 5, 1H, –H en 7);
6,85 (s, 1H, $-COO-\underline{CH}(C_6H_5)_2$); 6,9 à 7 (mt, 2H, –H en 3 et –H en 4 du thiophène);
7,05 à 7,4 (mt, 12H, aromatiques + –H en 5 du thiophène et –H en 5 de la pyridine);
7,58 (s, 1H, –H du thiazole); 7,64 (d large, J = 7,5, 1H, –H en 4 de la pyridine);
8,50 (mt, 2H, –H en 2 et –H en 6 de la pyridine); 9,23 (d, J = 8, 1H, –CO–NH–).

L'amino-7 benzhydryloxycarbonyl-2 oxo-8 [(pyridyl-3 méthyl)-2 thiazolyl-5]-3 thia-5 aza-1 bicyclo[4.2.0] octène-2 peut être préparé de la manière suivante:

A une solution de 10 g de benzhydryloxycarbonyl-2 t.butoxycarbonylamino-7 oxo-8 [(pyridyl-3 méthyl)-2 thiazolyl-5]-3 thia-5 aza-1 bicyclo[4.2.0] octène-2 dans 100 $cm^3$ d'acétonitrile, on ajoute 10 $cm^3$ d'une solution d'acide méthansulfonique. On agite le mélange pendant 5 minutes et on le verse dans 500 $cm^3$ d'un mélange 50/50 (en volumes) d'une solution saturée de bicarbonate de sodium et d'acétate d'éthyle. La phase organique est décantée, puis lavée successivemennt par 200 $cm^3$ d'eau distillée, puis 200 $cm^3$ d'une solution saturée de chlorure de sodium. Elle est séchée sur sulfate de magnésium anhydre, filtrée et le filtrat est concentré à sec sous pression réduite (30 mm de mercure; 4 kPa) à 30°C. On obtient 6,36 g d'amino-7 benzhydryloxycarbonyl-2 oxo-8 [(pyridyl-3 méthyl)-2 thiazolyl-5]-3 thia-5 aza-1 bicyclo[4.2.0] octène-2 sous la forme d'une meringue marron.

Spectre infra-rouge ($CHBr_3$), bandes caractéristiques ($cm^{-1}$) 3400, 3340, 1780, 1725, 1620, 1495, 1480, 1450, 1425, 1220, 760.

Spectre de RMN du proton (250 MHz, DMSO $d_6$, $\delta$ en ppm, J en Hz)

3,72 et 3,83 (2d, J = 18, 2H, $-S-CH_2-$); 4,13 et 4,23 (2d, J = 16, 2H, $-CH_2-$);
4,89 (d, J = 5, 1H, –H en 7); 5,08 (d, J = 5, 1H, –H en 6);
6,8 (s, 1H, $-COO-\underline{C}\underline{H}(C_6H_5)_2$); 7,05 à 7,4 (mt, aromatiques, $-NH_2$ et –H en 5 de la pyridine);
7,53 (s, 1H, –H du thiazole); 7,64 (ddd, J = 8–2,5 et 1, 1H, –H en 4 de la pyridine);
8,47 (dd, J = 5 et 1, 1H, –H en 6 de la pyridine); 8,49 (d, J = 2, 1H, –H en 2 de la pyridine).

Le benzhydryloxycarbonyl-2 t.butoxycarbonylamino-7 oxo-8 [(pyridyl-3 méthyl)-2 thiazolyl-5]-3 thia-5 aza-1 bicyclo[4.2.0] octène-2 peut être préparé de la manière suivante:

On ajoute une solution de 88,1 g de benzhydryloxycarbonyl-2 (bromo-1 oxo-2 éthyl)-3 t.butoxycarbonylamino-7 oxo-8 thia-5 aza-1 bicyclo[4.2.0] octène-2 (mélange des bromoaldéhydres épimères) dans 150 $cm^3$ de étrahydrofuranne anhydre à une solution de 25 g de pyridyl-3 thioformamide dans 200 $cm^3$ de N,N'-diméthylacétaamide. On agite le mélange pendant 6 heures à 20°C, puis on le coule dans 2 litres d'un mélange eau-acétate d'éthyle 50/50 (en volumes). La phase organique est décantée puis lavée successivement par 500 $cm^3$ d'une solution saturée de bicarbonate de sodium, 250 $cm^3$ d'eau et 250 $cm^3$ d'une solution saturée de chlorure de sodium. Elle est séchée sur sulfate de magnésium anhydre, filtrée et le filtrat est concentré à sec sous pression réduite (30 mm de mercure; 4 kPa). Le résidu est purifièpar chromatographie sur une colonne (hauteur: 60 cm, diamètre: 6 cm) de gel de silice (0,05–0,2 mm) en éluant par 7 litres d'acéate d'éthyle et en recueillant des fractions de 120 $cm^3$. Le contenu des fractions 18 à 56 est concentré à sec sous pression réduite (30 mm de mercure; 4 kPa) à 30°C et on obtient 26,74 g de benzhydryloxycarbonyl-2 t.butoxycarbonylamino-7 oxo-8 [(pyridyl-3 méthyl)-2 thiazolyl-5]-3 thia-5 aza-1 bicyclo[4.2.0] octène-2 sous la forme d'une meringue crème.

Spectre infra-rouge ($CHBr_3$), bandes caractéristiques ($cm^{-1}$) 3420, 1785, 1720, 1500, 1455, 1425, 1390, 1240, 760.

Spectre de RMN du proton (250 MHz, $CDCl_3$, $\delta$ en ppm, J en Hz)

1,47 (s, 9H, $-C(CH_3)_3$); 3,48 et 3,67 (2d, J = 18, 2H, $-S-CH_2-$);
4,1 (AB limite, J = 16, 2H, $-CH_2-$); 5,04 (d, J = 5, 1H, –H en 6);

5,41 $\left(d,\ J = 9,\ 1H,\ -\underset{\underset{O}{\|}}{C}-NH-\right)$.

5,71 (dd, J = 5 et 9, 1H, –H en 7); 6,9 (s, 1H, $-COO-\underline{C}\underline{H}(C_6H_5)_2$);
7,05 à 7,4 (mt, aromatiques et –H en 5 de la pyridine); 7,39 (s, 1H, –H du thiazole);
7,53 (ddd, J = 8–2,5 et 1, 1H, –H en 4 de la pyridine);
8,50 (d, J = 2,5, 1H, –H en 2 de la pyridine); 8,53 (dd, J = 5 et 1, 1H, –H en 6 de la pyridine).

Le pyridyl-3 thioacétamido peut être préparé de la manière suivante:

Dans une solution de 50 g de pyridyl-3 acétonitrile, 54 $cm^3$ de triéthylamine dans 500 $cm^3$ d'éthanol absolu, on fait barboter un courant d'acide sulfhydrique pendant 6 heures. On laisse reposer pendant 48 heures puis on fait passer un courant d'azote. Le mélange est concentré à sec sous pression réduite (30 mm de mercure; 4 kPa) et l'huile résiduelle est reprise dans 150 $cm^3$ d'éthanol. On chauffe au reflux, filtre et la cristallisation se développe en refroidissant. Les cristaux sont filtrés, lavés par 10 $cm^3$ d'éthanol puis séchés sous pression réduite (0,1 mm de mercure; 0,013 kPa). On obtient 52 g de pyridyl-3 thioacétamide sous la forme d'une poudre cristalline blanche.

F. inst. 135–136°C Kofler.

Spectre de RMN du proton (60 MHz, DMSO $d_6$, $\delta$ en ppm, J en Hz)

3,9 (s, 2H, $-CH_2-$); 7,38 (q, J = 8 et 5, 1H, –H en 5); 7,75 (dd, J = 8 et 2, 1H, –H en 4);
8,50 (dd, J = 5 et 2, 1H, –H en 6); 8,60 (d, J = 2, 1H, –H en 2); 9,50 (s large, 2H, $-NH_2$).

Exemple 27

On agite pendant 72 heures à 23°C une solution de 0,3 g de benzhydryloxycarbonyl-2 oxo-8 [(pyridyl-3 amino)-2 thiazolyl-5]-3 (thiényl-2 acétamido)-7 thia-5 aza-1 bicyclo[4.2.0] octène-2 et 0,3 $cm^3$ d'iodure de méthyle dans 6 $cm^3$ de N,N-diméthylformamide. Le mélange réactionnel est dilué par 50 $cm^3$ de dichlorométhane et la phase organique est lavée par 2 fois d'eau distillée, puis séchée sur sulfate de magnésium; elle est filtrée et le filtrat est concentré à sec sous pression réduite (30 mm de mercure; 4 kPa) à 30°C. Le résidu est dissout dans 3 $cm^3$ d'acétonitrile et la solution obtenue est versée sur 20 $cm^3$ d'oxyde d'isopropyle. Le précipité est filtré, lavé par 2 fois 5 $cm^3$ d'oxyde d'isopropyle et séché sous pression réduite (0,5 mm de mercure; 0,067 kPa). On obtient 0,3 g de benzhydryloxycarbonyl-2 oxo-8 [(méthyl-1 pyridinio-3) amino-2 thiazolyl-5]-3 (thiényl-2 acétamido)-7 thia-5 aza-1 bicyclo[4.2.0] octène-2 sous la forme d'une poudre jaune.

Spectre infra-rouge (KBr), bandes caractéristiques ($cm^{-1}$) 3200–3100, 2500, 1780, 1730, 1670, 1530, 1510, 765, 750, 705.

Spectre de RMN du proton (250 MHz, DMSO $d_6$, $\delta$ en ppm, J en Hz)

3,79 (AB limite, J = 15, 2H, $-CH_2-CO-NH-$); 3,82 et 3,95 (2d, J = 18, 2H, $-S-CH_2-$),

4,4 (s, 3H, $>\overset{+}{N}-CH_3$);

5,23 (d, J = 5, 1H, –H en 6); 5,83 (dd, J = 5 et 9, 1H, –H en 7);

6,91 (s, 1H, $-COO-\underline{CH}(C_6H_5)_2$); 6,95 à 7,05 (mt, –H en 3 et –H en 4 du thiophène);

6,95 à 7,35 (mt, aromatiques et $>N-H$);

7,27 (s, –H du thiazole); 7,38 (dd, J = 4,5 et 1, 1H, –H en 5 du thiophène);

8,05 (dd, J = 7,5 et 5, 1H, –H en 5 de la pyridine);

8,40 (dd, J = 8 et 1, 1H, –H en 4 de la pyridine);

8,60 (d large, J = 5, 1H, –H en 6 de la pyridine); 9,16 (s large, 1H, –H en 2 de la pyridine);

9,26 (d, J = 9, 1H, $-CO-NH-$).

On agite à 50°C pendant 30 minutes une solution de 0,3 g de benzhydryloxycarbonyl-2 oxo-8 [(méthyl-1 pyridinio-3) amino-2 thiazolyl-5]-3 (thiényl-2 acétamido)-7 thia-5 aza-1 bicyclo[4.2.0] octène-2, 1 $cm^3$ d'anisole dans 5 $cm^3$ d'acide formique. Le mélange est concentré à sec sous pression réduite (0,1 mm de mercure; 0,013 kPa) et le résidu est repris par 3 fois 30 $cm^3$ d'éthanol et concentré à sec sous pression réduite (0,1 mm de mercure; 0,013 kPa) à 30°C à chaque fois. Le résidu final est repris dans 30 $cm^3$ d'acétone, lavé par 2 fois 30 $cm^3$ d'oxyde d'éthyle et séché sous pression réduite (0,1 mm de mercure; 0,013 kPa). On obtient ainsi 0,13 g de carboxylato-2 [(méthyl-1 pyridinio-3) amino-2 thiazolyl-5]-3 oxo-8 (thiényl-2 acétamido)-7 thia-5 aza-1 bicyclo[4.2.0] octène-2 sous la forme d'un solide ocre.

Spectre infra-rouge (KBr), bandes caractéristiques ($cm^{-1}$) 3320, 3140, 2000, 1770, 1675, 1600, 1525, 1505, 710, 675.

Spectre de RMN du proton (250 MHz, DMSO $d_6$, $\delta$ en ppm, J en Hz)

3,77 (s, 2H, $-CH_2-CO-NH-$); 3,81 et 3,93 (2d, J = 18, 2H, $-S-CH_2-$);

4,36 (s, 3H, $>\underset{+}{N}-CH_3$);

5,17 (d, J = 5, 1H, –H en 6); 5,73 (dd, J = 9 et 5, 1H, –H en 7);

6,9 à 7 (mt, 2H, –H en 3 et –H en 4 du thiophène);

7,33 (dd, J = 4,5 et 1, 1H, –H en 5 du thiophène); 7,49 (s, 1H, –H du thiazole);

8 (dd, J = 7,5 et 5, 1H, –H en 5 de la pyridine);

8,47 (d large, J = 7,5, 1H, –H en 4 de la pyridine);

8,55 (d, J = 5, 1H, –H en 6 de la pyridine); 9,17 (d, J = 9, 1H, $-CO-NH-$);

9,33 (s large, 1H, –H en 2 de la pyridine);

11,55 (mf, 1H, $>NH$);

de 15 à 12 (mf étalé, 1H, $-COOH$).

Le benzhydryloxycarbonyl-2 oxo-8 [(pyridyl-3 amino)-2 thiazolyl-5]-3 (thiényl-2 acétamido)-7 thia-5 aza-1 bicyclo[4.2.0] octène-2 peut être obtenu de la manière suivante:

A une solution refroidie à 5°C de 3,85 g d'amino-7 benzhydryloxycarbonyl-2 oxo-8 [(pyridyl-3 amino)-2 thiazolyl-5]-3 thia-5 aza-1 bicyclo[4.2.0] octène-2 dans 100 $cm^3$ de tétrahydrofuranne sec, on

ajoute successivement 0,88 $cm^3$ de chlorure de thiényl-2 acétyle et 0,95 $cm^3$ de triéthylamine, puis on agite 35 minutes à cette température. On enlève le bain réfrigérant et on agite le mélange pendant 1 heure 30 minutes à 23°C. On verse le mélange réactionnel sur 400 $cm^3$ d'un mélange acétate d'éthyle-solution saturée de bicarbonate de sodium (50/50 en volumes). On laisse décanter et la phase organique est lavée successivement par 200 $cm^3$ d'eau distillée et 200 $cm^3$ d'une solution saturée de chlorure de sodium. Elle est séchée sur sulfate de magnésium anhydre, filtrée et le filtrat est concentré à sec sous pression réduite (30 mm de mercure; 4 kPa) à 30°C. Le résidu est chromatographié sur une colonne (hauteur: 30 cm, diamètre: 3 cm) de gel de silice (0,6—0,04 mm) en éluant par 2 litres d'acétate d'éthyle et en recueillant des fractions de 100 $cm^3$. Les fractions 5 à 8 contenant le produit pur sont réunies et concentrées sous pression réduite (30 mm de mercure; 4 kPa) à 30°C pour donner 1,3 g de benzhydryloxycarbonyl-2 oxo-8 [(pyridyl-3 amino)-2 thiazolyl-5]-3 (thiényl-2 acétamido)-7 thia-5 aza-1 bicyclo[4.2.0] octène-2 sous la forme d'une meringue jaune.

Spectre infra-rouge (KBr), bandes caractéristiques ($cm^{-1}$) 3380, 3150, 2000, 1780, 1730, 1670, 1590, 1550, 1530, 1490, 1225, 760, 750, 700.

Spectre de RMN du proton (250 MHz, $CDCl_3$, $\delta$ en ppm, J en Hz)

3,45 et 3,60 (2d, J = 18, 2H, $-S-CH_2-$);

3,86 (s, 2H, $-CH_2-\underset{\underset{O}{\|}}{C}-NH-$).

5,05 (d, J = 5, 1H, —H en 6); 5,89 (dd, J = 5 et 9, 1H, —H en 7);

6,71 (d, J = 9, 1H, $-\underset{\underset{O}{\|}}{C}-NH-$).

6,95 (s, 1H, $-COO-\underline{CH}(C_6H_5)_2$);
6,95 à 7,35 (mt, aromatiques, —H en 3, —H en 4 et —H en 5 du thiophène, —H en 5 de la pyridine);
7,80 (ddd, J = 8, 2 et 1, 1H, —H en 4 de la pyridine);
8,30 (dd, J = 5 et 1, 1H, —H en 6 de la pyridine);
8,37 (mf, 1H, $>N-H$).
8,47 (d, J = 2, 1H, —H en 2 de la pyridine).

L'amino-7 benzhydryloxycarbonyl-2 [(pyridyl-3 amino)-2 thiazolyl-5]-3 oxo-8 thia-5 aza-1 bicyclo[4.2.0] octène-2 peut être préparé de la manière suivante:

A une solution de 37,22 g de benzhydryloxycarbonyl-2 t.butoxycarbonylamino-7 [(pyridyl-3 amino)-2 thiazolyl-5]-3 oxo-8 thia-5 aza-1 bicyclo[4.2.0] octène-2 dans 372 $cm^3$ d'acétonitrile on ajoute en 5 minutes 37,7 $cm^3$ d'acide méthanesulfonique. Le mélange est agité pendant 5 minutes puis on ajoute la solution réactionnelle dans un mélange de 870 $cm^3$ d'une solution saturée de bicarbonate de sodium, 1740 $cm^3$ d'eau distillée et 580 $cm^3$ de chlorure de méthylène. On agite pendant 10 minutes et on filtre le précipité que l'on lave par 4 fois 100 $cm^3$ d'eau distillée. Le produit est séché et on obtient 27,66 g d'amino-7 benzhydryloxycarbonyl-2 [(pyridyl-3 amino)-2 thiazolyl-5]-3 oxo-8 thia-5 aza-1 bicyclo[4.2.0] octène-2 sous la forme d'une poudre cristalline jaune pâle.

Spectre infra-rouge (KBr), bandes caractéristiques ($cm^{-1}$) 3380, 3150, 2000, 1780, 1730, 1670, 1590, 1550, 1530, 1490, 1225, 800, 760, 750, 700.

Spectre de RMN du proton (250 MHz, DMSO $d_6$, $\delta$ en ppm, J en Hz)

2,4 (mf, 2H, $-NH_2$); 3,78 (AB limite, J = 18, 2H, $-S-CH_2$); 4,88 (d large, J = 5, 1H, —H en 7);
5,1 (d, J = 5, 1H, —H en 6); 6,85 (s, 1H, $-COO-\underline{CH}(C_6H_5)_2$);
7 à 7,35 (mt, 11H, aromatiques et —H du thiazole); 7,34 (mt, 1H, —H en 5 de la pyridine);
8,06 (ddd, J = 8—2,5 et 1, 1H, —H en 4 de la pyridine);
8,17 (dd, J = 5 et 1, 1H, —H en 6 de la pyridine);
8,68 (d, J = 2,5, 1H, —H en 2 de la pyridine); 10,38 (mf, 1H, —NH—).

Le benzhydryloxycarbonyl-2 t.butoxycarbonylamino-7 [(pyridyl-3 amino)-2 thiazolyl-5]-3 oxo-8 thia-5 aza-1 bicyclo[4.2.0] octène-2 peut être préparé de la manière suivante:

A une solution refroidie à —75°C de 107,1 g de benzhydryloxycarbonyl-2 t.butoxycarbonylamino-7 (diméthylamino-2 vinyl)-3 oxo-8 thia-5 aza-1 bicyclo[4.2.0] octène-2, forme E, dans 600 $cm^3$ de tétrahydrofuranne sec, on ajoute goutte à goutte une solution de 35,96 g de brome dans 40 $cm^3$ de dichlorométhane sec. Le mélange est agité à cette température pendant 10 minutes, puis on ajoute

30,64 g de pyridyl-3 thiourée en solution dans 400 $cm^3$ d'un mélange eau-tétrahydrofuranne (50/50 en volumes). On enlève le bain réfrigérant et on agite à 20°C pendant 17 heures. La solution brune est diluée par 1,5 litre d'acétate d'éthyle puis lavée successivement par 1 litre d'eau distillée, 1 litre d'une solution de bicarbonate de sodium demi-saturée, 1 litre d'eau distillée et 500 $cm^3$ d'une solution saturée de chlorure de sodium. La phase organique est séchée sur sulfate de magnésium anhydre. Après évaporation du solvant sous pression réduite (30 mm de mercure; 4 kPa) à 30°C, le résidu est chromatographié sur colonne (hauteur: 46 cm, diamètre: 8,1 cm) de gel de silice (0,06—0,20 mm) en éluant successivement par 5 litres d'un mélange cyclohexan-acétate d'éthyle (50/50 en volumes), 5 litres d'un mélange cyclohexane acétate d'éthyle (25/75 en volumes) et 7 litres d'acétate d'éthyle en recueillant des fractions de 1 litre. Les fractions 8 à 17 contenant le produit pur sont rassemblées et concentrées jusqu'à un volume résiduel de 250 $cm^3$. On obtient une suspension jaune conservée à 5°C pendant 12 heures. Le solide est filtré, lavé par 3 fois 100 $cm^3$ d'éther éthyline et séché sous pression réduite (1 mm de mercure; 0,13 kPa). On obtient ainsi 14,5 g de benzhydryloxycarbonyl-2 t.butoxycarbonylamino-7 [(pyridyl-3 amino)-2 thiazolyl-5]-3 oxo-8 thia-5 aza-1 bicyclo[4.2.0] octène-2 sous la forme d'une poudre jaune.

Spectre infra-rouge (KBr), bandes caractéristiques ($cm^{-1}$) 3330, 3100, 2500, 1790, 1720, 1590, 1530, 1500, 1370, 1160, 760, 745, 705.

Spectre de RMN du proton (250 MHz, DMSO $d_6$, $\delta$ en ppm, J en Hz)

1,45 (s, 9H, $-C(CH_3)_3$); 3,76 et 3,88 (2d, J = 18, 2H, $-S-CH_2-$);
5,18 (d, J = 5, 1H, −H en 6); 5,59 (dd, J = 9 et 5, 1H, −H en 7);
6,88 (s, 1H, $-COO-\underline{CH}(C_6H_5)_2$); 7 à 7,4 (mt, 11H, aromatiques et −H du thiazole);
7,34 (mt, 1H, −H en 5 de la pyridine);
8,07 (ddd, J = 8—2,5 et 1, 1H, −H en 4 de la pyridine); 8,11 (d, J = 9, 1H, −CONH−);
8,19 (dd, J = 5 et 1, 1H, −H en 6 de la pyridine);
8,69 (d, J = 2, 1H, −H en 2 de la pyridine); 10,45 (s, 1H, =N−H).

## Exemple 28

On agite pendant 24 heures à 20°C un mélange de 1,19 g de bromure de phénacyle, 1,99 g de benzhydryloxycarbonyl-2 oxo-8 [(pyridyl-3 amino)-2 thiazolyl-5]-3 (thiényl-2 acétamido)-7 thia-5 aza-1 bicyclo[4.2.0] octène-2 dans 10 $cm^3$ de N,N-diméthylformamide. Le mélange est dilué par 200 $cm^3$ d'oxyde d'éthyle. La suspension obtenue est agitée à 20°C pendant 30 minutes, puis on filtre, lave par 20 $cm^3$ d'oxyde d'éthyle et sèche sous pression réduite (0,1 mm de mercure; 0,013 kPa). On obtient ainsi 2,3 g de bromure de benzhydryloxycarbonyl-2 oxo-8 [(phénacyl-1 pyridinio-3) amino-2 thiazolyl-5]-3 (thiényl-2 acétamido)-7 thia-5 aza-1 bicyclo[4.2.0] octène-2 sous la forme d'une poudre jaune.

Spectre infra-rouge (KBr), bandes caractéristiques ($cm^{-1}$) 3200, 3120, 2500, 1780, 1725, 1695, 1665, 1600, 1570, 1530, 1505, 1450, 1230, 760, 700, 690, 665.

Spectre de RMN du proton (250 MHz, DMSO $d_6$, $\delta$ en ppm, J en Hz)

3,78 (AB limite, J = 16, 2H, 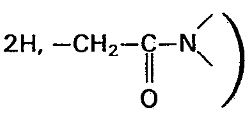 );

3,80 et 3,93 (2d, J = 18, 2H, $-S-CH_2-$); 5,22 (d, J = 5, 1H, −H en 6);
5,82 (dd, J = 8 et 5, 1H, −H en 7);

6,58 (AB limite, J = 18, 2H, 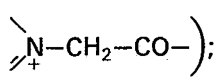 );

6,89 (s, 1H, $-COO-\underline{CH}(C_6H_5)_2$); 6,95 à 7,05 (mt, −H en 3 et −H en 4 du thiophène);
6,95 à 7,35 (mt, aromatiques); 7,23 (s, 1H, −H du thiazole);
7,37 (dd, J = 5 et 1, 1H, −H en 5 du thiophène);
7,68 (t, J = 7,5, 2H, −H aromatiques en méta du phénacyl);
7,82 (t, J = 7,5, 1H, −H aromatiques es para du phénacyl);
8,11 (d, J = 7,5, 2H, −H aromatiques en ortho du phénacyl);
8,18 (dd, J = 8 et 5, 1H, −H en 5 de la pyridine);
8,56 (d large, J = 8, 1H, −H en 4 de la pyridine); 8,62 (d, J = 5, 1H, −H en 6 de la pyridine);

9,25 (d, J = 8, 1H, 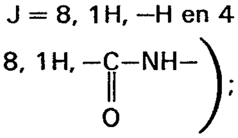 );

9,31 (s large, 1H, −H en 2 de la pyridine).

A une solution refroidie à 3°C de 2,3 g de bromure de benzhydryloxycarbonyl 2 oxo-8 [(phénacyl-1 pyridinio-3) amino-2 thiazolyl-5]-3 (thiényl-2 acétamido)-7 thia-5 aza-1 bicyclo[4.2.0] octène-2 dans 2,6 $cm^3$ d'anisole, on ajoute 26 $cm^3$ d'acide trifluoracétique. On agite pendant 15 minutes à 3°C, puis 30 minutes à 22°C. Le mélange est concentré à sec sous pression réduite (0,1 mm de mercure; 0,013 kPa) à 20°C et le résidu est repris dans 20 $cm^3$ d'acétate d'éthyle puis à nouveau concentré à sec sous pression réduite (0,1 mm de mercure; 0,013 kPa) à 20°C. On réitère cette opération deux fois en substituant l'acétate d'éthyle par l'éthanol. On dilue par 20 $cm^3$ d'oxyde d'éthyle, filtre le solide jaune, lave par 20 $cm^3$ d'oxyde d'éthyle et sèche sous pression réduite (0,1 mm de mercure; 0,013 kPa) pour donner 1,9 g de bromure de carboxy-2 oxo-8 [(phénacyl-1 pyridinio-3 amino)-2 thiazolyl-5]-3 (thiényl-2 acétamido)-7 thia-5 aza-1 bicyclo[4.2.0] octène-2 sous la forme d'une poudre jaune.

Spectre infra-rouge (KBr), bandes caractéristiques ($cm^{-1}$) 3280, 3120, 2100, 1775, 1700, 1670, 1600, 1575, 1500, 1510, 705, 690, 665.

Spectre de RMN du proton (350 MHz, DMSO $d_6$, $\delta$ en ppm, J en Hz)

3,78 (AB limite, 2H, 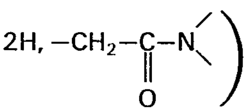);

3,81 et 3,93 (2d, J = 18, 2H, $-S-CH_2-$); 5,18 (d, J = 5, 1H, –H en 6);
5,73 (dd, J = 8 et 5, 1H, –H en 7);

6,53 (s large, 2H, 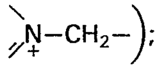);

6,93 à 7 (mt, 2H, –H en 3 et –H en 4 du thiophène);
7,35 (dd, J = 5 et 1,5, 1H, –H en 5 du thiophène); 7,46 (s, 1H, –H du thiazole);
7,67 (t, J = 8, 2H, –H aromatiques en méta du phénacyl);
7,79 (t, J = 8, 2H, –H aromatiques en para du phénacyl);
8,06 (d, J = 8, 2H, –H aromatiques en ortho du phénacyl);
8,15 (dd, J = 8,5 et 6, 1H, –H en 5 de la pyridine);
8,6 à 8,7 (mt, 2H, –H en 4 et –H en 6 de la pyridine);

9,19 (d, J = 9, 1H, 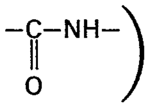);

9,45 (s, 1H, –H en 2 de la pyridine);

11,62 (mf, 1H, 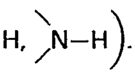).

Exemple 29

On agite une solution de 1,99 g de bromure de benzhydryloxycarbonyl-2 oxo-8 [(pyridyl-3 amino)-2 thiazolyl-5]-3 (thiényl-2 acétamido)-7 thia-5 aza-1 bicyclo[4.2.0] octène-2, 0,879 $cm^3$ de bromacétate de t.butyle dans 10 $cm^3$ de N,N-diméthylformamide à 22°C pendant 24 heures. Le mélange réactionnel est dilué par 100 $cm^3$ d'oxyde d'éthyle. On décante la phase liquide et le résidu est repris par 100 $cm^3$ d'oxyde d'éthyle. On agite 15 minutes à 20°C puis on filtre la phase solide, lave le précipité par 2 fois 20 $cm^3$ d'oxyde d'éthyle, puis sèche sous pression réduite (0,1 mm de mercure; 0,013 kPa) à 20°C. On obtient ainsi 2,3 g de bromure de benzhydryloxycarbonyl-2 [(t.butoxycarbonylméthyl-1 pyridinio-3) amino-2 thiazolyl-5]-3 oxo-8 (thiényl-2 acétamido)-7 thia-5 aza-1 bicyclo[4.2.0] octène-2 sous la forme d'une poudre jaune.

Spectre infra-rouge (KBr), bandes caractéristiques ($cm^{-1}$) 3200, 3100, 2500, 1780, 1740, 1670, 1600, 1570, 1520, 1505, 1455, 1370, 1240, 1180, 750, 705, 670.

Spectre de RMN du proton (350 MHz, DMSO $d_6$, $\delta$ en ppm, J en Hz)

1,5 (s, 9H, $-C(CH_3)_3$);

3,77 et 3,83 (2d, J = 16, 2H, 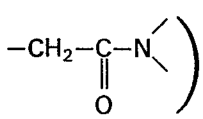);

3,81 et 3,93 (2d, J = 19, 2H, $-S-CH_2-$); 5,24 (d, J = 5, 1H, –H en 6);

5,62 et 5,69 (AB limite, J = 16, 2H, 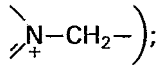);

5,81 (dd, J = 8,5 et 5, 1H, –H en 7); 6,89 (s, 1H, $-COO-C\underline{H}(C_6H_5)_2$);
6,90 à 7 (mt, –H en 3 et –H en 4 du thiophène); 6,90 à 7,35 (mt, aromatiques);
7,28 (s, –H du thiazole); 7,37 (dd, J = 5 et 1, –H en 5 du thiophène);
8,15 (dd, J = 8,5 et 6, 1H, –H en 5 de la pyridine);
8,56 (dd, J = 8,5 et 1,5, 1H, –H en 4 de la pyridine);
8,67 (d, J = 6, 1H, –H en 6 de la pyridine);

9,24 $\left(d,\ J = 8{,}5,\ 1H,\ -\underset{\underset{O}{\|}}{C}-NH-\right)$.

9,28 (s large, 1H, –H en 2 de la pyridine).

A une solution refroidie à 3°C de 2,3 g de bromure de benzhydryloxycarbonyl-2 [(t.butoxycarbonylméthyl-1 pyridinio-3) amino-2 thiazolyl-5]-3 oxo-8 (thiényl-2 acétamido)-7 thia-5 aza-1 bicyclo[4.2.0] octène-2 dans 2,3 $cm^3$ d'anisole, on ajoute 29 $cm^3$ d'acide trifluoracétique. On agite à 4°C pendant 30 minutes et à 20°C pendant 60 minutes. Le mélange est concentré à sec sous pression réduite (0,1 mm de mercure; 0,013 kPa) à 30°C et le résidu repris par 50 $cm^3$ d'acétate d'éthyle puis la solution obtenue est à nouveau concentrée à sec sous pression réduite (0,1 mm de mercure; 0,013 kPa). L'opération est réitérée et le solide jaune obtenu est lavé par 50 $cm^3$ d'oxyde d'éthyle, puis séché sous pression réduite (0,1 mm de mercure; 0,013 kPa) à 20°C. On obtient 1,3 g de trifluoracétate de carboxy-2 [(carboxyméthyl-1 pyridinio-3) amino-2 thiazolyl-5]-3 oxo-8 (thiényl-2 acétamido)-7 thia-5 aza-1 bicyclo[4.2.0] octène-2 sous la forme d'une poudre jaune.

Spectre infra-rouge (KBr), bandes caractéristiques ($cm^{-1}$) 3260, 3220, 3150, 2150, 1775, 1740, 1670, 1600, 1530, 1512, 1185, 1145, 800, 709.

Spectre de RMN du proton (250 MHz, DMSO $d_6$, $\delta$ en ppm, J en Hz)

3,78 $\left(AB\ limite,\ J = 15,\ 2H,\ -CH_2-\underset{\underset{O}{\|}}{C}-N\lessgtr\right)$;

3,81 et 3,95 (2d, J = 18, 2H, $-S-CH_2-$); 5,18 (d, J = 5, 1H, –H en 6);

3,53 $\left(s\ large,\ 2H,\ \gtrdot\overset{+}{N}-CH_2-\right)$;

5,72 (dd, J = 8 et 5, 1H, –H en 7); 6,9 à 7 (mt, 2H, –H en 3 et –H en 4 du thiophène);
7,37 (dd, J = 5 et 1, 1H, –H en 5 du thiophène); 7,50 (s, 1H, –H du thiazole);
8,07 (dd, J = 8 et 5,5, 1H, –H en 5 de la pyridine);
8,50 à 8,65 (mt, 2H, –H en 4 et –H en 6 de la pyridine);

9,21 $\left(d,\ J = 8,\ 1H,\ -\underset{\underset{O}{\|}}{C}-NH-\right)$.

9,4 (s, large, 1H, –H en 2 de la pyridine);

11,77 $\left(mf,\ 1H,\ \gtrdot NH\right)$.

## Exemple 30

Une solution de 0,79 g de benzhydryloxycarbonyl-2 (dichloro-3,4 phénylthio)acétamido-7 (nicotinoylamino-2 thiazolyl-5)-3 oxo-8 thia-5 aza-1 bicyclo[4.2.0] octène-2 et de 0,16 g d'iodure de méthyle dans 3 $cm^3$ de N,N diméthylformamide est agitée 17 heures à 25°C puis versée dans 100 $cm^3$ d'éther éthylique. On essore le solide et le lave 3 fois par 20 $cm^3$ d'éther éthylique pour obtenir après séchage 0,71 g de benzhydryloxycarbonyl-2 (dichloro-3,4 phénylthio) acétamido-7 [(méthylpyridinio-3) carbonylamino-2 thiazolyl-5]-3 oxo-8 thia-5 aza-1 bicyclo[4.2.0] octène-2 sous la forme d'un solide jaune.

Spectre infra-rouge (KBr), bandes caractéristiques ($cm^{-1}$) 3200, 3100–2500, 1785, 1725, 1660, 1550, 1500, 810, 750, 700.

Une solution de 0,71 g d'iodure de benzhydryloxycarbonyl-2 (dichloro-3,4 phénylthio)acétamido-7 [(méthylpyridinio-3) carbonylamino-2 thiazolyl-]-3 oxo-8 thia-5 aza-1 bicyclo[4.2.0] octène-2 dans 20 $cm^3$ d'acide formique est chauffée à 50°C pendant 30 minutes puis concentrée à sec sous pression réduite et traitée selon le mode opératoire décrit dans l'exemple 24 pour donner 0,28 g de carboxyla-

to-2 (dichloro-3,4 phénylthio)acétamido-7 [(méthylpyridinio-3) carbonylamino-2 thiazolyl-5]-3 oxo-8 thia-5 aza-1 bicyclo[4.2.0] octène-2 sous la forme d'un solide jaune.

Spectre de RMN du proton (250 MHz, DMSO $d_6$, $\delta$ en ppm, J en Hz)

3,76 et 3,92 (2d, J = 18, 2H, $-S-CH_2-$);

3,81 et 3,89 $\left(2d, J = 15, 2H, -CH_2-CO-N\langle\right)$;

4,43 $\left(s, 3H, \rangle\overset{+}{N}-CH_3\right)$;

5,15 (d, J = 5, 1H, −H en 6); 5,71 (dd, J = 9 et 5, 1H, −H en 7);
7,35 (dd, J = 8,5 et 2, 1H, −H aromatique en 6); 7,56 (d, J = 8,5, 1H, −H aromatique en 5);
7,63 (s, 1H, −H du thiazole); 7,64 (d, J = 2, 1H, −H aromatique en 2);
8,21 (dd, J = 7,5 et 5,5, 1H, −H en 5 de la pyridine);
9,04 (d, J = 7,5, 1H, −H en 4 de la pyridine); 9,07 (d, J = 5,5, 1H, −H en 6 de la pyridine);

9,25 $\left(d, J = 9, 1H, -\underset{\underset{O}{\|}}{C}-NH-\right)$.

9,53 (s large, 1H, −H en 2 de la pyridine).

Le benzhydryloxycarbonyl-2 (dichloro-3,4 phénylthio) acétamido-7 (nicotinoylamino-2 thiazolyl-5)-3 oxo-8 thia-5 aza-1 bicyclo[4.2.0] octène-2 peut être obtenu de la manière suivante:

A une solution de 6,43 g d'amino-7 benzhydryloxycarbonyl-2 (nicotinoylamino-2 thiazolyl-5)-3 oxo-8 thia-5 aza-1 bicyclo[4.2.0] octène-2 dans 25 $cm^3$ de N,N diméthylacétamide à 0°C, on ajoute en 10 minutes une solution de chlorure de dichloro-3,4 phénylthioacétyle (préparée par action de 8,4 $cm^3$ de chlorure d'oxalyle sur 3,99 g d'acide correspondant dans 48 $cm^3$ d'éther éthylique et remplacement du solvant) dans 20 $cm^3$ d'acétate d'éthyle. Après 1 heures à 0°C puis 40 minutes à température comprise entre 0 et 20°C le mélange réactionnel est versé dans 250 $cm^3$ d'acétate d'éthyle et lavé 3 fois par 100 $cm^3$ d'eau distillée puis par 100 $cm^3$ de solution saturée de chlorure de sodium. La phase organique est séchée et concentrée à sec sous pression réduite (30 mm de mercure; 4 kPa) à 40°C. Le résidu est filtré sur une colonne de 40 g de silice (0,02—0,5 mm) en éluant par 2 litres de mélange de dichlorométhane et de méthanol (95/5 en volumes). Le résidu obtenu après concentration à sec du solvant sous pression réduite (30 mm de mercure; 4 kPa) à 30°C est cristallisé dans 20 $cm^3$ de dichlorométhane pour donner 2,9 g de benzhydryloxycarbonyl-2 (dichloro-3,4 phénylthio)acétamido-7 (nicotinoylamino-2 thiazolyl-5)-3 oxo-8 thia-5 aza-1 bicyclo[4.2.0] octène-2 sous la forme d'un solide cristallin jaune pâle.

Rf = 0,2 (chromatoplaque de gel de silice, éluant acétate d'éthyle).

L'amino-7 benzhydryloxycarbonyl-2 (nicotinoylamino-2 thiazolyl-5)-3 oxo-8 thia-5 aza-1 bicyclo[4.2.0] octène-2 peut être préparé de la manière suivante:

9 g de benzhydryloxycarbonyl-2 t.butoxycarbonylamino-7 (nicotinoylamino-2 thiazolyl-5)-3 oxo-8 thia-5 aza-1 bicyclo[4.2.0] octène-2 dans 134 $cm^3$ d'acétonitrile sont traités par 7,6 g d'acide p.toluènesulfonique selon le mode opératoire décrit dans l'exemple 24 pour donner 6,43 g d'amino-7 benzhydryloxycarbonyl-2 (nicotinoylamino-2 thiazolyl-5)-3 oxo-8 thia-5 aza-1 bicyclo[4.2.0] octène-2 sous la forme d'une poudre crème.

Rf 0,23 (chromatoplaque de gel de silice, éluant acétate d'éthyle).

Le benzhydryloxycarbonyl-2 t.butoxycarbonylamino-2 (nicotinoylamino-2 thiazolyl-5)-3 oxo-8 thia-5 aza-1 bicyclo[4.2.0] octène-2 peut être préparé de la manière suivante:

On ajoute à une solution de 11,3 g d'(amino-2 thiazolyl-5)-3 benzhydryloxycarbonyl-2 t.butoxycarbonylamino-7 oxo-8 thia-5 aza-1 bicyclo[4.2.0] octène-2 dans 300 $cm^3$ de tétrahydrofuranne refroidie à 3°C, 7,12 g de chlorhydrate de chlorure de nicotinoyle, puis en 10 minutes, 11,12 $cm^3$ de triéthylamine. On agite 1 heure à 3°C, puis 2 heures en laissant remonter la température à 20°C. On verse le mélange réactionnel dans 400 $cm^3$ d'acétate d'éthyle et lave 3 fois par 120 $cm^3$ d'eau distillée et 100 $cm^3$ de solution saturée de chlorure de sodium. La phase organique est séchée et concentrée à sec sous pression réduite (30 mm de mercure; 4 kPa) à 40°C. Le résidu est cristallisé dans 20 $cm^3$ d'acétate d'éthyle pour donner 7,4 g de benzhydryloxycarbonyl-2 t.butoxycarbonylamino-2 (nicotinoylamino-2 thiazolyl-5)-3 oxo-8 thia-5 aza-1 bicyclo[4.2.0] octène-2 sous la forme d'un solide jaune.

Rf = 0,24 (chromatoplaque de gel de silice, éluant: mélange de cyclohexane et d'acétate d'éthyle 20/80 en volumes).

La présente invention concerne également les compositions pharmaceutiques qui contiennent comme produit actif au moins un produit de formule générale (I), sous forme libre ou sous forme de sel, en association avec un ou plusieurs adjuvants ou diluants pharmaceutiquement acceptables. Ces compositions peuvent être utilisées par voie orale, parentérale ou topique.

Comme compositions solides pour administration orale peuvent être utilisés des comprimés, des pilules, des poudres ou des granulés. Dans ces compositions, le produit actif selon l'invention est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale peuvent être des solutions stériles aqueuses ou non aqueuses, des suspensions ou des émulsions. Comme solvant ou véhicule, on peut employer le propylèneglycol, un polyéthylèneglykol, des huiles végétales, en particulier l'huile d'olive et des esters organiques injectables, par exemple l'oléate d'éthyle. Ces compositions peuvent également contenir des adjuvants, en particulier des agents mouillants, émulsifiants ou dispersants. La stérilisation peut se faire de plusieurs façons, par exemple à l'aide d'un filtre aseptisant, en incorporant à la composition des agents stérilisants, par irradiation ou par chauffage. Elles peuvent être également préparées sous forme de compositions solides stériles qui seront dissoutes au moment de l'emploi dans de l'eau stérile ou tout autre milieu stérile injectable.

Les compositions pour administration topique peuvent être des crèmes, pommades, lotions, suspensions, solutions, dans lesquels le produit actif peut être associé à des épaississants, agents favorisant la pénétration, colorants et autres ingrédients habituels.

En thérapeutique humaine, les médicaments selon la présente invention sont particulièrement utiles dans le traitement des infections d'origine bactérienne.

D'une façon générale, le médecin déterminera la posologie qu'il estime la plus appropriée en fonction de l'âge, du poids, du degré de l'infection et des autres facteurs propres au sujet à traiter. Généralement, les doses sont comprises entre 2 et 8 g par jour de produit actif par voie intramusculaire ou intraveineuse pour un adulte.

L'exemple suivant, donné à titre non limitatif, illustre une composition selon la présente invention.

Exemple A

On prépare une solution injectable ayant la composition suivante:

| | |
|---|---|
| — sel de sodium du carboxy-2 oxo-8 (phényl-2 thiazolyl-5)-3 (thiényl-2 acétamido)-7 thia-5 aza-1 bicyclo[4.2.0] octène-2 | 261 mg |
| — chlorure de sodium | 1,5 mg |
| — soluté injectable | 2 $cm^3$ |

Cette solution contient 250 mg de produit actif (compté en acide libre).

Exemple B

On prépare une solution injectable ayant la composition suivante:

| | |
|---|---|
| — sel de sodium de l'(acétamido-2 thiazolyl-5)-3 carboxy-2 oxo-8 phénylacétamido-7 thia-5 aza-1 bicyclo[4.2.0] octène-2 | 262 mg |
| — chlorure de sodium | 1,5 mg |
| — soluté injectable | 2 $cm^3$ |

Cette solution contient 250 mg de produit actif (compté en acide libre).

Exemple C

On prépare 100 $cm^3$ d'une solution aqueuse isotonique contenant 2,47 g de bicarbonate de sodium et, comme produit actif, 10 g de [(L'amino-2 carboxy-2 éthylthioacétamido)-2 thiazolyl-5]-3 carboxy-2 (dichloro-3,4 phénylthio) acétamido-7 oxo-8 thia-5 aza-1 bicyclo[4.2.0] octène-2. Après filtration sur filtre bactériologique, on répartit aseptiquement cette solution en ampoules (à raison de 10 $cm^3$ par ampoule), on lyophilise et on scelle les ampoules.

Chaque ampoule contient l'équivalent de 1 g du produit actif, sous forme de son sel de sodium.

**Revendications pour les Etats contractants BE, CH, DE, FR, GB, IT, LI, LU, NL, SE**

1. Un dérivé de la céphalosporine caractérisé en ce qu'il répond à la formule générale

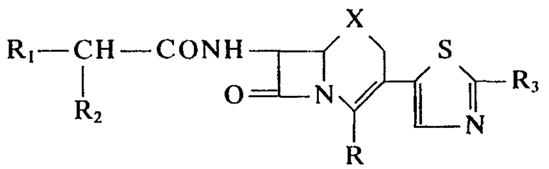

dans laquelle
le symbole $R_1$ représente un radical hétérocyclyle choisi parmi thiényl, furyle, dithiol-1,3 one-2 yle-4 ou un radical phényle, p.hydroxyphényle, phénoxy ou dichlorophénylthio
le symbole $R_2$ représente un atome d'hydrogène ou
le symbole $R_1$ est un radical phényle ou p.hydroxyphényle et le symbole $R_2$ est un radical amino,
le symbole $R_2$ représente un atome d'hydrogène, un radical phényle (éventuellement substitué par un radical acylamino), alcoylthio, alcoylamino, dialcoylamino ou anilino, un radical acylamino, benzoylamino ou thénoylamino, éventuellement substitué sur l'atome d'azote (par un radical alcoyle ou phényle), un radical alcoyloxycarbonylamino, dialcoylaminoéthylamino, dialcoylaminométhylèneamino ou alcoylidènehydrazo, un radical acétamido substitué par un radical amino, amino-2 éthylthio ou L'amino-2 carboxy-2 éthylthio, ou un radical de structure $-AR'_3$ dans lequel A représente un radical bivalent choisi parmi $-CH_2-$, $-NH-$ ou $-NHCO-$ et $R'_3$ représente un radical méthyl-1 pyridinio-3 (ou $-4$), benzoylméthyl-1 pyridinio-3 (ou $-4$) ou carboxyméthyl-1 pyridinio-3 (ou $-4$)
le symbole R représente un radical carboxy ou représente un radical carboxylato lorsque $R'_3$ est un radical pyridinio substitué,
le symbole X représente un atome d'oxygène ou de soufre,
étant entendu que les radicaux et portions alcoyles ou acyles cités ci-dessus sont droits ou ramifiés et contiennent 1 à 4 atomes de carbone, sous ses formes D, L et D,L lorsque $R_2$ est autre que l'atome d'hydrogène, ainsi que ses sels métalliques, ses sels d'addition avec les bases azotées et, lorsqu'ils existent, ses sels d'addition avec les acides.

2. Un produit selon la revendication 1 caractérisé en ce que le symbole X représente un atome d'oxygène et les symboles $R_1$, $R_2$, $R_3$ et R sont définis comme dans la revendication 1, ou bien le symbole R est défini comme dans la revendication 1, le symbole X est un atome de soufre et soit les symboles $R_1$ et $R_2$ sont définis comme dans la revendication 1 et le symbole $R_3$ représente un radical phényle, acylaminophényle, alcoylthio ou anilino, un radical benzoylamino ou thénoylamino éventuellement substitué sur l'atome d'azote (par un radical alcoyle ou phényle), un radical acylamino substitué sur l'atome d'azote (par un radical alcoyle ou phényle), un radical alcoyloxycarbonylamino, dialcoylaminoéthylamino, dialcoylaminométhylèneamino ou alcoylidènehydrazo, un radical acétamido substitué par un radical amino, amino-2 éthylthio ou L'amino-2 carboxy-2 éthylthio, ou un radical de structure $-AR'_3$ dans lequel A représente un radical bivalent choisi parmi $-CH_2-$, $-NH-$ ou $-NHCO-$ et $R'_3$ représente un radical méthyl-1 pyridinio-3 (ou $-4$), benzoylméthyl-1 pyridinio-3 (ou $-4$) ou carboxyméthyl-1 pyridinio-3 (ou $-4$), soit le symbole $R_1$ est un radical dithiol-1,3 one-2 yle-4, le symbole $R_2$ est un atome d'hydrogène et $R_3$ est un atome d'hydrogène, un radical alcoylamino, dialcoylamino ou acylamino, sous ses formes D, L et D,L lorsque $R_2$ est autre qu'un atome d'hydrogène, ainsi que ses sels métalliques, ses sels d'addition avec les bases azotées et, lorsqu'ils existent, ses sels d'addition avec les acides.

3. Un produit selon la revendication 1, caractérisé en ce que le symbole X est un atome de soufre, le symbole R est un radical carboxy, le symbole $R_3$ représente un atome d'hydrogène ou un radical alcoylamino, dialcoylamino ou acylamino, et le symbole $R_1$ représente un radical thiényle, furyle, phényle, p.hydroxyphényle, phénoxy ou dichloro-3,4 phénylthio et le symbole $R_2$ représente un atome d'hydrogène ou bien le symbole $R_1$ représente un radical phényle ou p.hydroxyphényle et le symbole $R_2$ représente un radical amino, sous ses formes D, L et D,L lorsque $R_2$ est autre qu'un atome d'hydrogène, ainsi que ses sels métalliques, ses sels d'addition avec les bases azotées et lorsqu'ils existens ses sels d'addition avec les acides.

4. Un produit selon la revendication 1, caractérisé en ce que les symboles X et R sont définis comme dans la revendication 1, le symbole $R_1$ est un radical thiényle et le symbole $R_2$ est un atome d'hydrogène, ou bien le symbole $R_1$ est un radical phényle ou p.hydroxyphényle et le symbole $R_2$ est un radical amino, le symbole $R_3$ représente un atome d'hydrogène, un radical phényle, acylamino éventuellement substitué sur l'atome d'azote par un radical alcoyle, un radical dialcoylaminoéthylamino, un radical amino-2 éthylthioacétamido, L'amino-2 carboxy-2 éthylthioacétamido ou un radical de structure $-A-R'_3$ dans laquelle A et $R'_3$ sont définis comme dans la revendication 1, sous ses formes D, L et D,L lorsque $R_2$ est amino, ainsi que ses sels métalliques, ses sels d'addition avec les bases azotées et lorsqu'ils existens ses sels d'addition avec les acides.

5. Un procédé de préparation d'un produit selon la revendication 1, caractérisé en ce que l'on fait agir un acide de formule générale

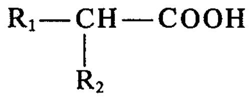

(dans laquelle $R_1$ et $R_2$ sont définis comme dans la revendication 1, et dont la fonction amine que peut représenter $R_2$ est préalablement protégée), ou un dérivé réactif de cet acide, sur un dérivé d'amino-7 céphalosphorine de formule générale

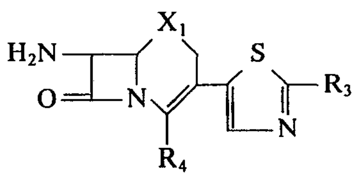

dans laquelle $R_3$ qui est défini comme dans la revendication 1 est protégé lorsqu'il contient un radical amino, $R_4$ est un radical carboxy, un radical carboxy protégé, ou un radical carboxylato, et $X_1$ est défini comme X dans la revendication 1, ou représente un radical sulfinyle, puis le cas échéant réduit le sulfoxyde obtenu, élimine les radicaux protecteurs, et transforme éventuellement le produit obtenu en un sel métallique, en un sel d'addition avec une base azotée ou, lorsqu'il existe, en sel d'addition avec un acide.

6. U procédé selon la revendication 5 caractérisé en ce que l'on fait agir à titre de dérivé réactif un halogénure, l'anhydride, un anhydride mixte ou un ester réactif de l'acide de formule générale

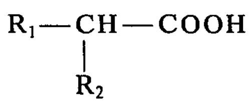

dans laquelle $R_1$ et $R_2$ sont définis comme dans la revendication 5.

7. Un procédé selon la revendication 5 caractérisé en ce que l'on utilise une amino-7 céphalosporine dans la formule de laquelle $R_4$ est un radical carboxy protété par un groupement méthoxyméthyle, t.butyle, trichloro-2,2,2 éthyle, benzhydryle, p.nitrobenzyle ou p.méthoxybenzyle.

8. Un procédé de préparation d'un produit selon la revendication 1, pour lequel $R_3$ est défini comme dans la revendication 1, à l'exception de représenter un radical $-AR'_3$ et R représente un radical carboxy, caractérisé en ce que l'on fait agir un produit de formule générale

$R_3$ CS $NH_2$

dans laquelle $R_3$ est défini comme ci-dessus, sur un dérivé de la céphalosphorine de formule générale

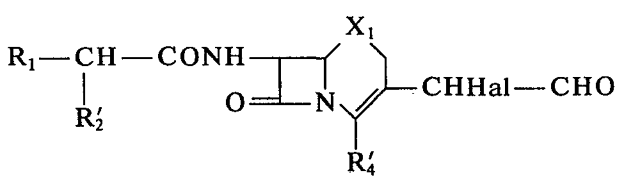

dans laquelle $R_1$ et $X_1$ sont définis comme dans la revendication 5, $R'_2$ est un atome d'hydrogène ou un radical amino protégé, $R'_4$ est un radical carboxy protégé et Hal représente un atome d'halogène, puis éventuellement un agent de déshydratation, puis le cas échéant réduit le sulfoxyde obtenu, élimine les groupements protecteurs et transforme éventuellement le produit obtenu en un sel métallique, en un sel d'addition avec une base azotée ou, lorsqu'il existe, en un sel d'addition avec un acide.

9. Un procédé e préparation selon la revendication 8, caractérisé en ce que l'on met en oeuvre un dérivé de la céphalosporine de formule générale

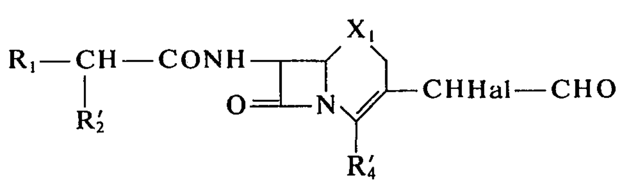

dans laquelle $R_1$, $R'_2$, $R'_4$ et X sont définis comme dans la revendication 8 et Hal représente un atome de chlore, de brome ou d'iode.

10. Un procédé selon la revendication 8, caractérisé en ce que l'on met en oeuvre une céphalosporine de formule générale

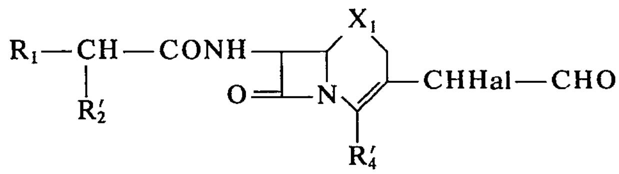

dans laquelle $R_1$, $R'_4$, $X_1$ et Hal sont définis comme dans la revendication 8 et $R'_2$ représente un radical amino protégé par un groupement t.butoxycarbonyle, trichloro-2,2,2 éthoxycarbonyle, trityle, benzyle, benzyloxycarbonyle, formyle, trifluoracétyle, p.nitrobenzyloxycarbonyle, p.méthoxybenzyloxycarbonyle, diphénylphosphinoyle ou par un radical de formule générale

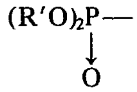

dans laquelle le symbole R' est alcoyle, trichloro-2,2,2 éthyle, phényle ou benzyle (ces deux derniers éventuellement substitués par un atome d'halogène ou par un radical alcoyle, alcoyloxy ou nitro) ou les deux symboles R' forment ensemble un radical alcoylène contenant 2 ou 3 atomes de carbone.

11. Un procédé de préparation d'un produit selon la revendication 1, pour lequel $R_3$ représente un radical acylamino, benzoylamino ou thénoylamino éventuellement substitués sur l'atome d'azote, ou représente un radical alcoyloxycarbonylamino, dialcoylaminométhylèneamino, un radical acétamido substitué ou un radical de structure $-AR'_3$ dans laquelle A est –NHCO– et $R'_3$ est défini comme dans la revendication 1, caractérisé en ce que l'on traite une amine de formule générale

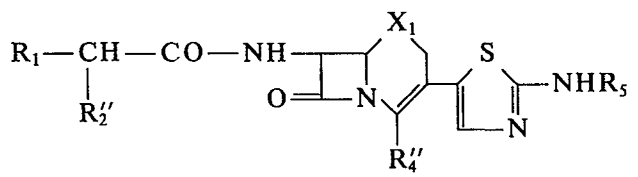

dans laquelle $R_1$ et $X_1$ sont définis comme dans la revendication 5, $R''_2$ représente un radical amino protégé, $R''_4$ est un radical carboxy libre ou protégé et $R_5$ représente un atome d'hydrogène ou un radical alcoyle ou phényle, par toute méthode connue pour former un amide, un carbamate ou une amidine, sans toucher au reste de la molécule, puis réduit le cas échéant le sulfoxyde obtenu, élimine éventuellement les radicaux protecteurs et transforme éventuellement le produit obtenu en un sel métallique, en sel d'addition avec une base azotée ou, lorsqu'il existe, en sel d'addition avec un acide.

12. Un procédé de préparation d'un produit selon la revendication 1, pour lequel $R_3$ est un radical de structure $-AR'_3$ et R un radical carboxylato, caractérisé en ce que l'on fait agir un halogénure de méthyle, de benzoylméthyle ou de carboxyméthyle (dont la fonction acide est libre ou protégée) sur un dérivé de céphalosporine de formule générale

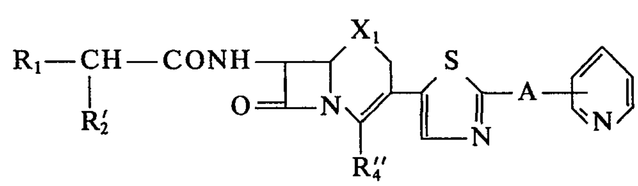

dans laquelle le radical A est substitué en position –3 ou –4 du noyau pyridyle, $R_1$ et A sont définis comme dans la revendication 1, $R'_2$ et $R''_4$ sont définis comme dans la revendication 11, $X_1$ est défini comme dans la revendication 5, puis le cas échéant réduit le sulfoxyde obtenu, élimine les radicaux protecteurs et transforme éventuellement le produit obtenu en un sel métallique, en sel d'addition avec une base azotée ou en sel d'addition avec un acide fort, lorsqu'ils existent.

13. Un procédé de préparation d'un produit selon la revendication 1, pour lequel $R_3$ représente un radical acétamido substitué par un radical amino-2 éthylthio ou L'amino-2 carboxy-2 éthylthio et R représente un radical carboxy, caractérisé en ce que l'on fait agir la cystéamine ou la cystéine dont la fonction amine et le cas échéant acide sont préalablement protégées, sur un dérivé d'halogénoacétamidothiazolyl céphalosporine de formule générale

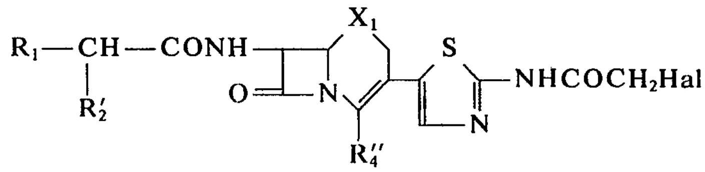

dans laquelle $R_1$ et $X_1$ sont définis comme dans la revendication 5, $R'_2$ et $R''_4$ sont définis comme dans la revendication 11, Hal représente un atome de chlore, de brome ou d'iode, puis réduit le cas échéant le produit obtenu, élimine les radicaux protecteurs et transforme éventuellement le produit obtenu en un sel métallique, en un sel d'addition avec une base azotée ou en un sel d'addition avec un acide.

14. Composition pharmaceutique caractérisée en ce qu'elle contient au moins un produit selon la revendication 1, en association avec un ou plusieurs diluants ou adjuvants compatibles et pharmaceutiquement acceptables.

**Revendication pour l'Etat contractant AT**

Procédé de préparation d'un dérivé de la céphalosporine de formule générale

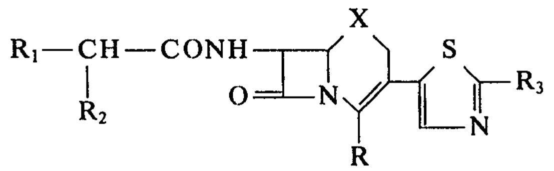

dans laquelle
le symbole $R_1$ représente un radical hétérocyclyle choisi parmi thiényle, furyle, dithiol-1,3 one-2 yle-4 ou un radical phényle, p.hydroxyphényle, phénoxy ou dichlorophénylthio
le symbole $R_2$ représente un atome d'hydrogène ou
le symbole $R_1$ est un radical phényle ou p.hydroxyphényle et le symbole $R_2$ est un radical amino,
le symbole $R_3$ représente un atome d'hydrogène, un radical phényle (éventuellement substitué par un radical acylamino), alcoylthio, alcoylamino, dialcoylamino ou anilino, un radical acylamino, benzoylamino ou thénoylamino, éventuellement substitué sur l'atome d'azote (par un radical alcoyle ou phényle), un radical alcoyloxycarbonylamino, dialcoylaminoéthylamino, dialcoylaminométhylèneamino ou alcoylidènehydrazo, un radical acétamido substitué par un radical amino, amino-2 éthylthio ou L'amino-2 carboxy-2 éthylthio, ou un radical de structure $-AR'_3$ dans lequel A représente un radical bivalent choisi parmi $-CH_2-$, $-NH-$ ou $-NHCO-$ et $R'_3$ représente un radical méthyl-1 pyridinio-3 (ou -4), benzoylméthyl-1 pyridinio-3 (ou -4) ou carboxyméthyl-1 pyridinio-3 (ou -4)
le symbole R représente un radical carboxyy ou représente un radical carboxylato lorsque $R'_3$ est un radical pyridinio substitué,
le symbole X représente un atome d'oxygène ou de soufre, étant entendu que les radicaux et portions alcoyles ou acyles cités ci-dessus sont droits ou ramifiés et contiennent 1 à 4 atomes de carbone, sous ses formes D, L et D,L lorsque $R_2$ est autre que l'atome d'hydrogène, ainsi que de ses sels métalliques, ses sels d'addition avec les bases azotées et, lorsqu'ils existent, ses sels d'addition avec les acides, caractérisé en ce que l'on fait agir un acide de formule générale

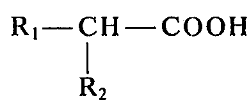

(dans laquelle $R_1$ et $R_2$ sont définis comme ci-dessus, et dont la fonction amine que peut représenter $R_2$ est préalablement protégée), ou un dérivé réactif de cet acide, sur un dérivé d'amino-7 céphalosporine de formule générale

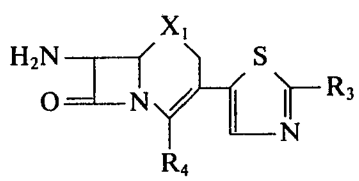

dans laquelle $R_3$ qui est défini comme dans la revendication 1 est protégé lorsqu'il contient un radical amino, $R_4$ est un radical carboxy, un radical carboxy protégé, ou un radical carboxylato, et $X_1$ est défini comme X dans la revendication 1, ou représente un radical sulfinyle, puis le cas échéant réduit le sulfoxyde obtenu, élimine les radicaux protecteurs, et transforme éventuellement le produit obtenu en un

sel métallique, en un sel d'addition avec une base azotée ou, lorsqu'il existe, en sel d'addition avec un acide, ou bien lorsque $R_3$ est défini comme précédemment, à l'exception de représenter un radical $-Ar'_3$ et R représente un radical carboxy, on fait agir un produit de formule générale

$R_3$ CS $NH_2$

dans laquelle $R_3$ est défini comme ci-dessus, sur un dérivé de la céphalosporine de formule générale

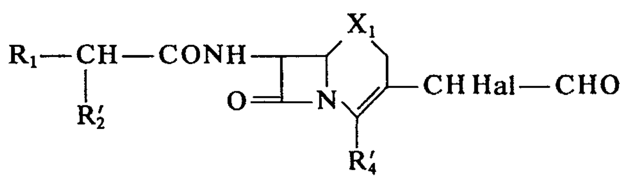

dans laquelle $R_1$ et $X_1$ sont définis comme précédemment, $R'_2$ est un atome d'hydrogène ou un radical amino protégé, $R'_4$ est un radical carboxy protégé et Hal représente un atome d'halogène, puis éventuellement un agent de déshydratation, puis le cas échéant réduit le sulfoxyde obtenu, élimine les groupements protecteurs et transforme éventuellement le produit obtenu en un sel métallique, en un sel d'addition avec une base azotée ou, lorsqu'il existe, en un sel d'addition avec un acide, ou bien lorsque $R_3$ représente un radical acylamino, benzoylamino ou thénoylamino éventuellement substitués sur l'atome d'azote, ou représente un radical alcoyloxycarbonylamino, dialcoylaminométhylèneamino, un radical acétamido substitué ou un radical de structure $-AR'_3$ dans laquelle A est $-NHCO-$ et $R'_3$ est défini comme précédemment, on traite une amine de formule générale

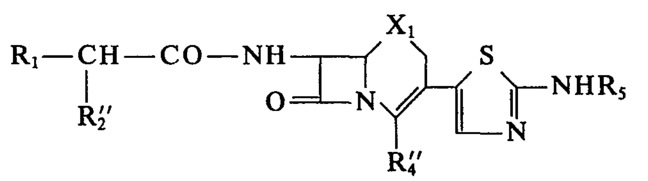

dans laquelle $R_1$ et $X_1$ sont définis comme précédemment, $R''_2$ représente un radical amino protégé, $R''_4$ est un radical carboxy libre ou protégé et $R_5$ représente un atome d'hydrogène ou un radical alcoyle ou phényle, par toute méthode connue pour former un amide, un carbamate ou une amidine, sans toucher au reste de la molécule, puis réduit le cas échéant le sulfoxyde obtenu, élimine éventuellement les radicaux protecteurs et transforme éventuellement le produit obtenu en un sel métallique, en sel d'addition avec une base azotée ou, lorsqu'il existe, en sel d'addition avec un acide, ou bien lorsque $R_3$ est un radical de structure $-AR'_3$ et R un radical carboxylato, on fait agir un halogénure de méthyle, de benzoylméthyle ou de carboxyméthyle (dont la fonction acide est libre ou protégée) sur un dérivé de céphalosporine de formule générale

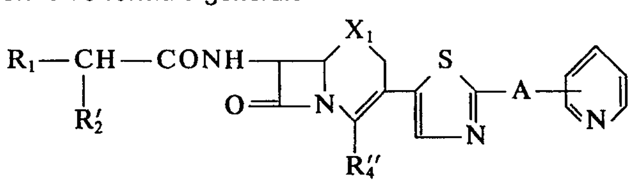

dans laquelle le radical A est substitué en position -3 ou -4 du noyau pyridyle, $R_1$, $R'_2$, $R''_4$, $X_1$ et A sont définis comme précédemment, puis le cas échéant réduit le sulfoxyde obtenu, élimine les radicaux protecteurs et transforme éventuellement le produit obtenu en un sel métallique, en sel d'addition avec une base azotée ou en sel d'addition avec un acide fort, lorsqu'ils existent, ou bien lorsque $R_3$ représente un radical acétamido substitué par un radical amino-2 éthylthio ou L'amino-2 carboxy-2 éthylthio et R représente un radical carboxy, on fait agir la cystéamine ou la cystéine dont la fonction amine et le cas échéant acide sont préalablement protégées, sur un dérivé d'halogénoacétamidothiazolyl céphalosporine de formule générale

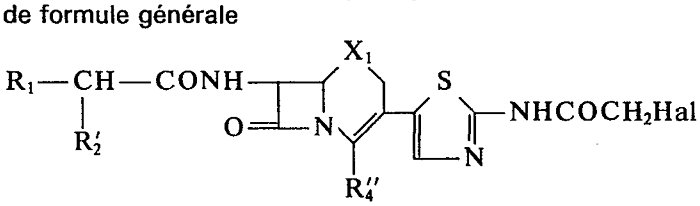

dans laquelle $R_1$, $R'_2$, $R''_4$ et $X_1$ sont définis comme précédemment, Hal représente un atome de chlore, de brome ou d'iode, puis réduit le cas échéant le produit obtenu, élimine les radicaux protecteurs et transforme éventuellement le produit obtenu en un sel métallique, en un sel d'addition avec une base azotée ou en un sel d'addition avec un acide.

**Patentansprüche für die Vertragsstaaten BE, CH, DE, FR, GB, IT, LI, LU, NL, SE**

1. Ein Derivat des Cephalosporins, dadurch gekennzeichnet, daß es der allgemeinen Formel

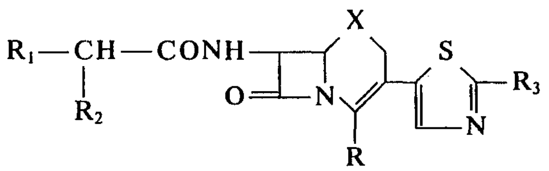

worin
das Symbol $R_1$ einen Heterocyclylrest ausgewählt unter Thienyl, Furyl, Dithiol-1,3-on-2-yl-4 oder einen Phenyl-, p-Hydroxyphenyl-, Phenoxy- oder Dichlorphenylthiorest bedeutet,
das Symbol $R_2$ ein Wasserstoffatom bedeutet oder
das Symbol $R_1$ ein Phenyl- oder p-Hydroxyphenylrest ist und
das Symbol $R_2$ ein Aminorest ist,
das Symbol $R_3$ ein Wasserstoffatom, einen Phenylrest (gegebenenfalls substituiert durch einen Acylaminorest), Alkylthio, Alkylamino, Dialkylamino oder Anilino, einen Acylamino-, Benzoylamino- oder Thenoylaminorest bedeutet, der gegebenenfalls am Stickstoffatom substituiert ist (durch einen Alkyl- oder Phenylrest), einen Alkyloxycarbonylamino-, Dialkylaminoethylamino-, Dialkylaminomethylamino- oder Alkylidenhydrazorest bedeutet, einen Acetamidorest substituiert durch einen Amino-, 2-Aminoethylthio- oder L-2-Amino-2-carboxy-ethylthiorest, oder einen Rest der Struktur $-AR'_3$ bedeutet, worin A einen zweiwertigen Rest ausgewählt unter $-CH_2-$, $-NH-$ oder $-NHCO-$ bedeutet und $R'_3$ einen 1-Methyl-pyridinio-3- (oder -4-), 1-Benzoylmethyl-pyridinio-3- (oder -4-), oder 1-Carboxymethyl-pyridinio-3- (oder -4-) -rest bedeutet,
das Symbol R einen Carboxyrest bedeutet oder einen Carboxylatorest bedeutet, wenn $R'_3$ ein substituierter Pyridiniorest ist,
das Symbol X ein Schwefel- oder Sauerstoffatom bedeutet,
wobei die vorstehend erwähnten Alkyl- oder Acylreste und -teile gerade oder verzweigt sind und 1 bis 4 Kohlenstoffatome enthalten, unter seinen D-, L- und D,L-Formen, wenn $R_2$ anders als ein Wasserstoffatom ist, sowie seine Metallsalze, seine Additionssalze mit Stickstoffbasen und, falls sie existieren, seine Additionssalze mit Säuren.

2. Ein Produkt gemäß Anspruch 1, dadurch gekennzeichnet, daß das Symbol X ein Sauerstoffatom bedeutet und die Symbole $R_1$, $R_2$, $R_3$ und R wie in Anspruch 1 definiert sind, oder das Symbol R wie in Anspruch 1 definiert ist, das Symbol X ein Schwefelatom ist und entweder die Symbole $R_1$ und $R_2$ wie in Anspruch 1 definiert sind und das Symbol $R_3$ einen Phenyl-, Acylaminophenyl-, Alkylthio- oder Anilinorest, einen Benzoylamino- oder Thenoylaminorest gegebenenfalls substituiert am Stickstoffatom (durch einen Alkyl- oder Phenylrest), einen Acylaminorest substituiert am Stickstoffatom (durch einen Alkyl- oder Phenylrest), einen Alkyloxycarbonylamino-, Dialkylaminoethylamino-, Dialkylaminomethylenamino- oder Alkylidenhydrazorest, einen Acetamidorest substituiert durch einen Amino-, 2-Aminoethylthio- oder L-2-Amino-2-carboxyethylthiorest, oder einen Rest der Struktur $-AR'_3$ bedeutet, worin A einen zweiwertigen Rest ausgewählt unter $-CH_2-$, $-NH-$ oder $-NHCO-$ bedeutet und $R'_3$ einen 1-Methyl-pyridinio-3- (oder -4-), 1-Benzoylmethyl-pyridinio-3- (oder -4-) oder 1-Carboxymethyl-pyridinio-3- (oder -4-) -rest bedeutet, oder das Symbol $R_1$ ist ein Dithiol-1,3-on-2-yl-4-rest, das Symbol $R_2$ ist ein Wasserstoffatom und $R_3$ ist ein Wasserstoffatom, ein Alkylamino-, Dialkylamino- oder Acylaminorest, unter seinen D-, L- und D,L-Formen, wenn $R_2$ anders als ein Wasserstoffatom ist, sowie seine Metallsalze, seine Additionssalze mit Stickstoffbasen und, falls sie existieren, seine Additionssalze mit Säuren.

3. Ein Produkt gemäß Anspruch 1, dadurch gekennzeichnet, daß das Symbol X ein Schwefelatom ist, das Symbol R ein Carboxyrest ist, das Symbol $R_3$ ein Wasserstoffatom oder ein Alkylamino-, Dialkylamino- oder Acylaminorest ist und das Symbol $R_1$ einen Thienyl-, Furyl-, Phenyl-, p-Hydroxyphenyl-, Phenoxy- oder 3,4-Dichlorphenylthiorest bedeutet und das Symbol $R_2$ ein Wasserstoffatom bedeutet oder das Symbol $R_1$ einen Phenyl- oder p-Hydroxyphenylrest bedeutet und das Symbol $R_2$ einen Aminorest bedeutet, unter seinen D-, L- und D,L-Formen, wenn $R_2$ anders als ein Wasserstoffatom ist, sowie seine Metallsalze, seine Additionssalze mit Stickstoffbasen und, falls sie existieren, seine Additionssalze mit Säuren.

4. Ein Produkt gemäß Anspruch 1, dadurch gekennzeichnet, daß die Symbole X und R wie in Anspruch 1 definiert sind, das Symbol $R_1$ ein Thienylrest ist und das Symbol $R_2$ ein Wasserstoffatom ist, oder das Symbol $R_1$ ein Phenyl- oder p-Hydroxyphenyrest ist und das Symbol $R_2$ ein Aminorest ist, das Symbol $R_3$ ein Wasserstoffatom, einen Phenyl-, Acylaminorest gegebenenfalls substituiert am Stickstoffatom durch einen Alkylrest, einen Dialkylaminoethylaminorest, einen 2-Aminoethylthioacetamidorest, 2-L-Amino-2-carboxyethylthioacetamidorest oder einen Rest der Struktur $-A-R'_3$ bedeutet, worin A und $R'_3$ wie in Anspruch 1 definiert sind, unter seinen D-, L- und D,L-Formen, wenn $R_2$ Amino ist, sowie seine Metallsalze, seine Additionssalze mit Stickstoffbasen und, falls sie existieren, seine Additionssalze mit Säuren.

5. Verfahren zur Herstellung des Produkts gemäß Anspruch 1, dadurch gekennzeichnet, daß man eine Säure der allgemeinen Formel

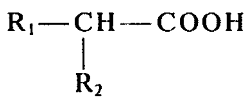

(worin $R_1$ und $R_2$ wie in Anspruch 1 definiert sind und deren Aminfunktion, welche $R_2$ bedeuten kann, vorher geschützt wurde), oder ein reaktives Derivat dieser Säure auf ein 7-Aminocephalosporinderivat der allgemeinen Formel

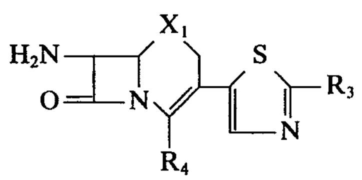

einwirken läßt, worin $R_3$, das wie in Anspruch 1 definiert ist, geschützt ist, wenn es einen Aminorest enthält, $R_4$ ein Carboxyrest, ein geschützter Carboxyrest oder ein Carboxylatorest ist und $X_1$ wie X in Anspruch 1 definiert ist oder einen Sulfinylrest bedeutet, und daß man dann gegebenenfalls das erhaltene Sulfoxid reduziert, die Schutzgruppen entfernt und gegebenenfalls das erhaltene Produkt in ein Metallsalz, in ein Additionssalz mit einer Stickstoffbase oder, falls es existiert, in ein Additionssalz mit einer Säure überführt.

6. Verfahren gemäß Anspruch 5, dadurch gekennzeichnet, daß man als reaktives Derivat ein Halogenid, das Anhydrid, ein gemischtes Anhydrid oder einen reaktiven Ester der Säure der allgemeinen Formel

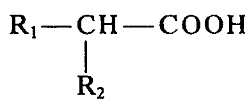

worin $R_1$ und $R_2$ wie in Anspruch 5 definiert sind, zur Reaktion bringt.

7. Verfahren gemäß Anspruch 5, dadurch gekennzeichnet, daß man ein 7-Aminocephalosporin verwendet, in dessen Formel $R_4$ ein Carboxyrest ist, geschützt durch eine Methoxymethyl-, tert.-Butyl-, 2,2,2-Trichlorethyl-, Benzhydryl-, p-Nitrobenzyl- oder p-Methoxybenzylgruppe.

8. Verfahren zur Herstellung eines Produkts gemäß Anspruch 1, wobei $R_3$ wie in Anspruch 1 definiert ist, mit Ausnahme der Bedeutung eines Restes $-AR'_3$, und R einen Carboxyrest bedeutet, dadurch gekennzeichnet, daß man ein Produkt der allgemeinen Formel

$R_3$ CS $NH_2$

worin $R_3$ wie vorstehend definiert ist, auf ein Derivat des Cephalosporins der allgemeinen Formel

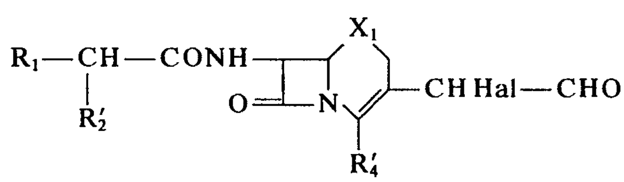

worin $R_1$ und $X_1$ wie in Anspruch 5 definiert sind, $R'_2$ ein Wasserstoffatom oder ein geschützter Aminorest ist, $R'_4$ ein geschützter Carboxyrest ist und Hal ein Halogenatom bedeutet, einwirken läßt, und daß man dann gegebenenfalls ein Dehydratisierungsmittel einwirken läßt, und daß man dann gegebenenfalls das erhaltene Sulfoxid reduziert, die Schutzgruppen entfernt und das erhaltene Produkt gegebenenfalls in ein Metallsalz, in ein Additionssalz mit einer Stickstoffbase oder, falls es existiert, in ein Additionssalz mit einer Säure überführt.

9. Herstellungsverfahren gemäß Anspruch 8, dadurch gekennzeichnet, daß man ein Derivat des Cephalosporins der allgemeinen Formel

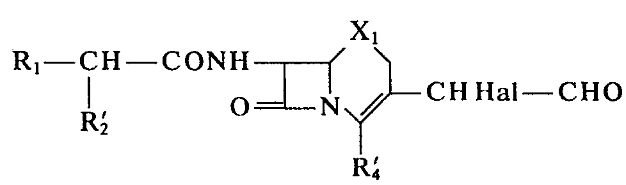

einsetzt, worin $R_1$, $R'_2$, $R'_4$ und X wie in Anspruch 8 definiert sind und Hal ein Chlor-, Brom- oder Jodatom bedeutet.

10. Verfahren gemäß Anspruch 8, dadurch gekennzeichnet, daß man ein Cephalosporin der allgemeinen Formel

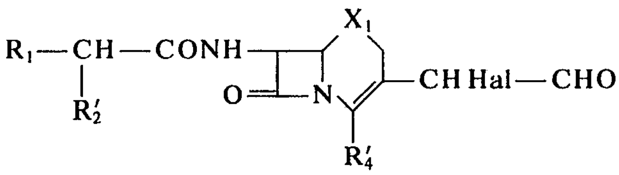

einsetzt, worin $R_1$, $R'_4$, $X_1$ und Hal wie in Anspruch 8 definiert sind und $R'_2$ einen Aminorest bedeutet, der durch eine tert.-Butoxycarbonyl-, 2,2,2-Trichlorethoxycarbonyl-, Trityl-, Benzyl-, Benzyloxycarbonyl-, Formyl-, Trifluoracetyl-, p-Nitrobenzyloxycarbonyl-, p-Methoxybenzyloxycarbonyl-, Diphenylphosphinoylgruppe oder durch einen Rest der allgemeinen Formel

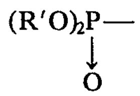

geschützt ist, worin das Symbol R' Alkyl, 2,2,2-Trichlorethyl, Phenyl oder Benzyl ist (die beiden letzteren gegebenenfalls substituiert durch ein Halogenatom oder durch einen Alkyl-, Alkyloxy- oder Nitrorest) oder die beiden Symbole R' bilden zusammen einen Alkylenrest mit 2 oder 3 Kohlenstoffatomen.

11. Verfahren zur Herstellung eines Produkts gemäß Anspruch 1, wobei $R_3$ einen Acylamino-, Benzoylamino- oder Thenoylaminorest bedeutet, die gegebenenfalls am Stickstoffatom substituiert sind, oder einen Alkyloxycarbonylamino-, Dialkylaminomethylenaminorest, einen substituierten Acetamidorest oder einen Rest der Struktur $-AR'_3$ bedeutet, worin A für –NHCO– steht und $R'_3$ wie in Anspruch 1 definiert ist, dadurch gekennzeichnet, daß man ein Amin der allgemeinen Formel

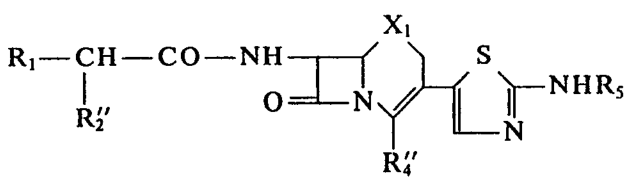

worin $R_1$ und $X_1$ wie in Anspruch 5 definiert sind, $R''_2$ einen geschützten Aminorest bedeutet, $R''_4$ ein freier oder geschützter Carboxylrest ist und $R_5$ ein Wasserstoffatom oder einen Alkyl- oder Phenylrest bedeutet, nach jeder Methode, die zur Bildung eines Amids, eines Carbamats oder eines Amidins bekannt ist, behandelt, ohne daß der Rest des Moleküls davon berührt wird, daß man dann gegebenenfalls das erhaltene Sulfoxid reduziert, gegebenenfalls die Schutzgruppen entfernt und gegebenenfalls das erhaltene Produkt in ein Metallsalz, ein Additionssalz mit einer Stickstoffbase oder, falls es existiert, in ein Additionssalz mit einer Säure überführt.

12. Verfahren zur Herstellung eines Produkts gemäß Anspruch 1, wobei $R_3$ ein Rest der Struktur $-AR'_3$ und R ein Carboxylatorest ist, dadurch gekennzeichnet, daß man ein Methyl-, Benzoylmethyl- oder Carboxymethylhalogenid (dessen saure Funktion frei oder geschützt ist) auf ein Cephalosporinderivat der allgemeinen Formel

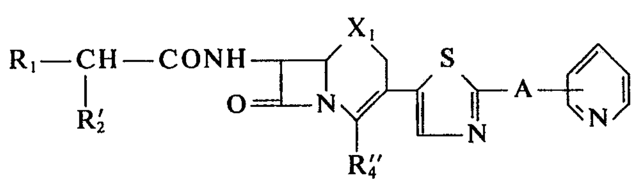

einwirken läßt, worin der Rest A in 3- oder 4-Stellung des Pyridinrests substituiert ist, $R_1$ und A wie in Anspruch 1 definiert sind, $R'_2$ und $R''_4$ wie in Anspruch 11 definiert sind, $X_1$ wie in Anspruch 5 definiert ist, daß man dann gegebenenfalls das erhaltene Sulfoxid reduziert, die Schutzgruppen entfernt und das erhaltene Produkt gegebenenfalls in ein Metallsalz, ein Additionssalz mit einer Stickstoffbase oder ein Additionssalz mit einer starken Säure, falls sie existieren, überführt.

13. Verfahren zur Herstellung eines Produkts gemäß Anspruch 1, wobei $R_3$ eine Acetamidorest substituiert durch einen 2-Aminoethylthio- oder L-2-Amino-2-carboxyethylthiorest bedeutet und R einen Carboxyrest bedeutet, dadurch gekennzeichnet, daß man Cysteamin oder Cystein, dessen Aminfunktion und gegebenenfalls Säurefunktion vorher geschützt wurde, auf ein Halogenacetamidothiazolyl-Cephalosporin der allgemeinen Formel

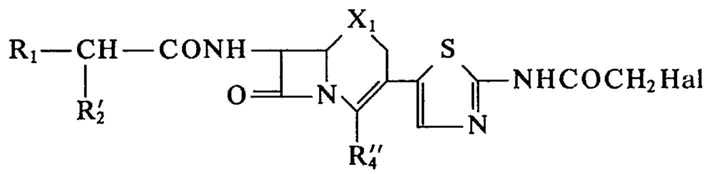

einwirken läßt, worin $R_1$ und $X_1$ wie in Anspruch 5 definiert sind, $R'_2$ und $R''_4$ wie in Anspruch 1 definiert sind, Hal ein Chlor-, Brom- oder Jodatom bedeutet, daß man dann gegebenenfalls das erhaltene Produkt reduziert, die Schutzgruppen entfernt und das erhaltene Produkt gegebenenfalls in ein Metallsalz, in ein Additionssalz mit einer Stickstoffbase oder in ein Additionssalz mit einer Säure überführt.

14. Pharmazeutische Zusammensetzung, dadurch gekennzeichnet, daß sie wenigstens ein Produkt gemäß Anspruch 1 zusammen mit einem oder mehreren verträglichen und pharmazeutisch annehmbaren Verdünnungsmitteln oder Hilfsmitteln enthält.

**Patentanspruch für den Vertragsstaat AT**

Verfahren zur Herstellung eines Cephalosporinderivats der allgemeinen Formel

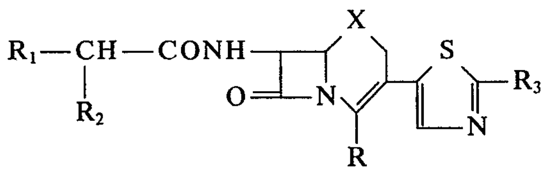

in welcher
das Symbol $R_1$ einen Heterocyclylrest, ausgwählt aus Thienyl, Furyl, 1,3-Dithiol-2-on-4-yl, oder einen Phenyl-, p-Hydroxyphenyl-, Phenoxy- oder Dichlorphenylthiorest darstellt,
das Symbol $R_2$ ein Wasserstoffatom darstellt oder
das Symbol $R_1$ ein Phenyl- oder p-Hydroxyphenylrest ist und das Symbol $R_2$ ein Aminorest ist,
das Symbol $R_3$ ein Wasserstoffatom, einen Phenylrest (gegebenenfalls substituiert durch einen Acylaminorest), einen Alkylthio-, Alkylamino-, Dialkylamino- oder Anilinorest, einen Acylamino-, Benzoylamino- oder Thenoylaminorest, gegebenenfalls substituiert am Stickstoffatom (durch einen Alkyl- oder Phenylrest), einen Alkyloxycarbonylamino-, Dialkylaminoäthylamino-, Dialkylaminomethylenamino- oder Alkylidenhydrazorest, einen Acetamidorest, substituiert durch einen Amino-, 2-Amino-äthylthio- oder 2-L-Amino-2-carboxyäthylthiorest, oder einen Rest der Formel $-AR'_3$, worin A einen zweiwertigen Rest, ausgewählt aus $-CH_2-$, $-NH-$ oder $-NHCO-$ darstellt und $R'_3$ einen 1-Methyl-3- (oder -4-) -pyridinio-, 1-Benzoylmethyl-3- (oder -4-) -pyridinio- oder 1-Carboxymethyl-3- (oder -4-) -pyridiniorest bedeutet, darstellt,
das Symbol R einen Carboxyrest darstellt oder für einen Carboxylatrest steht, wenn $R'_3$ ein substituierter Pyridiniorest ist,
das Symbol X ein Sauerstoff- oder Schwefelatom darstellt,
wobei die oben erwähnten Alkyl- oder Acylreste und -teile selbstverständlich geradkettig oder verzweigt sind und 1 bis 4 Kohlenstoffatome enthalten, in den Formel D, L und D,L, wenn $R_2$ von einem Wasserstoffatom verschieden ist, sowie von ihren Metallsalzen, ihren Additionssalzen mit Stickstoffbasen und, falls existent, von ihren Additionssalzen mit Säuren, dadurch gekennzeichnet, daß man eine Säure der allgemeinen Formel

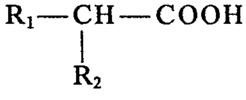

(in welcher $R_1$ und $R_2$ die obige Bedeutung haben und deren Aminfunktion, die $R_2$ darstellen kann, zuvor geschützt wurde) oder ein reaktionsfähiges Derivat dieser Säure, auf ein 7-Amino-cephalosporin-derivat der allgemeinen Formel

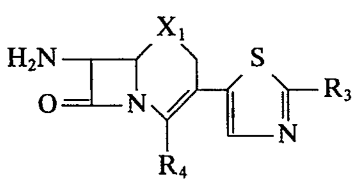

in welcher $R_3$, das die im Anspruch 1 angegebene Bedeutung hat, geschützt ist, wenn es einen Aminorest enthält, $R_4$ ein Carboxyrest, ein geschützter Carboxyrest oder ein Carboxylatrest ist und $X_1$ die Bedeutung von X in Anspruch 1 hat oder einen Sulfinylrest darstellt, einwirken läßt, dann zutreffendenfalls das erhaltene Sulfoxid reduziert, die Schutzgruppen entfernt und gegebenenfalls das erhaltene Produkt in ein Metallsalz, ein Additionssalz mit einer Stickstoffbase oder, falls existent, in ein Additionssalz mit einer Säure überführt, oder, wenn $R_3$ die obige Bedeutung mit Ausnahme eines Restes $-AR'_3$ hat und R einen Carboxyrest darstellt, läßt man eine Verbindung der allgemeinen Formel

$R_3\ CS\ NH_2$

in welcher $R_3$ die obige Bedeutung hat, auf ein Cephalosporinderivat der allgemeinen Formel

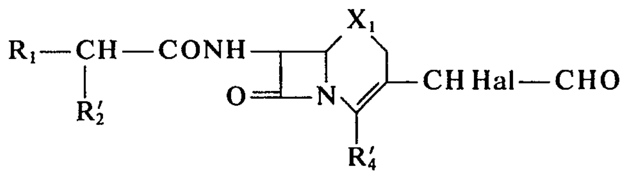

in welcher $R_1$ und $X_1$ die obige Bedeutung haben, $R'_2$ ein Wasserstoffatom oder ein geschützter Aminorest ist, $R'_4$ ein geschützter Carboxyrest ist und Hal ein Halogenatom darstellt, und dann gegebenenfalls ein Dehydratationsmittel einwirken, reduziert dann zutreffendenfalls das erhaltene Sulfoxid, entfernt die Schutzgruppen und überführt das erhaltene Produkt gegebenenfalls in ein Metallsalz, ein Additionssalz mit einer Stickstoffbase oder, falls existent, in ein Additionssalz mit einer Säure, oder, wenn $R_3$ einen Acylamino-, Benzoylamino- oder Thenoylaminorest, gegebenenfalls substituiert am Stickstoffbase, darstellt oder einen Alkyloxycarbonylamino-, Dialkylaminomethylenaminorest, einen substituierten Acetamidorest oder einen Rest der Formel $-AR'_3$, in welcher A $-NHCO-$ ist, darstellt und $R'_3$ die obige Bedeutung hat, behandelt man ein Amin der allgemeinen Formel

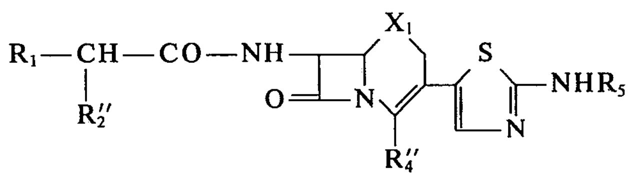

in welcher $R_1$ und $X_1$ die obige Bedeutung haben, $R''_2$ einen geschützten Aminorest darstellt, $R''_4$ ein freier oder geschützter Carboxyrest ist und $R_5$ ein Wasserstoffatom oder ein Alkyl- oder Phenylrest ist, nach jeder bekannten Methode zur Bildung eines Amids, eines Carbamats oder eines Amidins, wobei der Rest des Moleküls nicht angegriffen wird, reduziert dann zutreffendenfalls das erhaltene Sulfoxid, entfernt gegebenenfalls die Schutzgruppen und wandelt gegebenenfalls die erhaltene Verbindung in ein Metallsalz, ein Additionssalz mit einer Stickstoffbase oder, falls existent, in ein Additionssalz mit einer Säure um, oder, wenn $R_3$ ein Rest der Formel $-AR'_3$ ist und R ein Carboxylatrest ist, läßt man ein Methyl-, Benzoylmethyl- oder Carboxymethylhalogenid (dessen Säurefunktion frei oder geschützt ist) auf ein Cephalosporinderivat der allgemeinen Formel

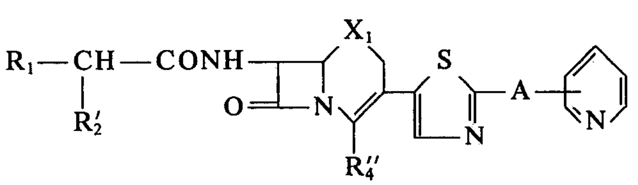

in welcher der Rest A in 3- oder 4-Stellung des Pyridylkerns substituiert ist, $R_1$, $R'_2$, $R''_4$, $X_1$ und A die obige Bedeutung haben, einwirken und reduziert dann zutreffendenfalls das erhaltene Sulfoxid, entfernt die Schutzgruppen und überführt gegebenenfalls das erhaltene Produkt in ein Metallsalz, ein Additionssalz mit einer Stickstoffbase oder ein Salz mit einer starken Säure, falls existent, oder, wenn $R_3$ einen Acetamidorest, substituiert durch einen 2-Aminoäthylthio- oder 2-L-Amino-2-carboxy-äthylthiorest, darstellt und R einen Carboxyrest darstellt, läßt man das Cysteamin oder Cystein, dessen Aminfunktion und gegebenenfalls Säurefunktion zuvor geschützt wurden, auf ein Halogenacetamidothiazolyl-cephalosporinderivat der allgemeinen Formel

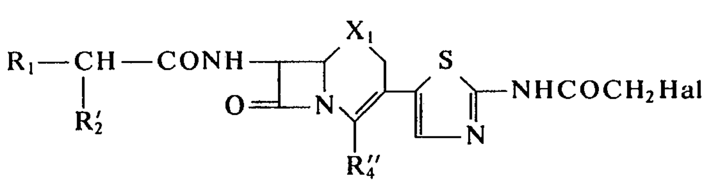

in welcher $R_1$, $R'_2$, $R''_4$ und $X_1$ die obige Bedeutung haben, Hal ein Chlor-, Brom- oder Jodatom darstellt einwirken, reduziert dann zutreffendenfalls das erhaltene Produkt, entfernt die Schutzgruppen und überführt gegebenenfalls das erhaltene Produkt in ein Metallsalz, in ein Additionssalz mit einer Stickstoffbase oder in ein Additionssalz mit einer Säure.

**Claims for the Contracting States BE, CH, DE, FR, GB, IT, LI, LU, NL, SE**

1. A cephalosporin derivative, characterised in that it corresponds to the general formula

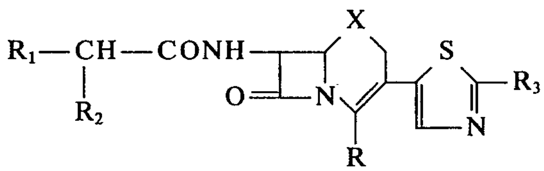

in which
the symbol $R_1$ represents a heterocyclyl radical chosen from among thienyl, furyl or 1,3-dithiol-2-on-4-yl or a phenyl, p-hydroxyphenyl, phenoxy or dichlorophenylthio radical and
the symbol $R_2$ represents a hydrogen atom or
the symbol $R_1$ is a phenyl or p-hydroxyphenyl radical and the symbole $R_2$ is an amino radical,
the symbol $R_3$ represents a hydrogen atom, a phenyl radical (optionally substituted by an acylamino radical), an alkylthio, alkylamino, dialkylamino or anilino radical, an acylamino, benzoylamino or thenoylamino radical, optionally substituted at the nitrogen atom (by an alkyl or phenyl radical), an alkoxycarbonylamino, dialkylaminoethylamino, dialkylaminomethyleneamino or alkylidenehydrazo radical, an acetamido radical substituted by an amino, 2-aminoethylthio or L-2-amino-2-carboxyethylthio radical, or a radical of the structure $-AR'_3$ in which A represents a bivalent radical chosen from among $-CH_2-$, $-NH-$ or $-NHCO-$ and $R'_3$ represents a 1-methyl-3- (or -4-) pyridinio or 1-benzoylmethyl-3- (or -4-) pyridinio or 1-carboxymethyl-3- (or -4-) pyridinio radical,
the symbol R represents a carboxyl radical or represents a carboxylato radical if $R'_3$ is a substituted pyridinio radical, and
the symbol X represents an oxygen or sulphur atom, it being understood that the alkyl or acyl radicals and portions mentioned above are straight or branched and contain 1 to 4 carbon atoms,
in its D, L and D,L forms if $R_2$ is other than a hydrogen atom, as well as its metal salts, its addition salts with nitrogen-containing bases and, where they exist, its addition salts with acids.

2. A product according to Claim 1, characterised in that the symbol X represents an oxygen atom and the symbols $R_1$, $R_2$, $R_3$ and R are defined as in Claim 1, or the symbol R is defined as in Claim 1, the symbol X is a sulphur atom and either the symbols $R_1$ and $R_2$ are defined as in Claim 1 and the symbol $R_3$ represents a phenyl, acylaminophenyl, alkylthio or anilino radical, a benzoylamino or thenoylamino radical which is optionally substituted at the nitrogen atom (by an alkyl or phenyl radical), an acylamino radical which is substituted at the nitrogen atom (by an alkyl or phenyl radical), an alkoxycarbonylamino, dialkylaminoethylamino, dialkylaminomethyleneamino or alkylidenehydrazo radical, an acetamido radical substituted by an amino, 2-aminoethylthio or L-2-amino-2-carboxy-ethylthio radical, or a radical of the structure $-AR'_3$, in which A represents a bivalent radical chosen from among $-CH_2-$, $-NH-$, $-NHCO-$ and $R'_3$ represents a 1-methyl-3-(or -4-) pyridinio, 1-benzoylmethyl-3- (or -4-) pyridinio or 1-carboxymethyl-3- (or -4-) pyridinio radical, or the symbol $R_1$ is a 1,3-dithiol-2-on-4-yl radical, the symbol $R_2$ is a hydogen atom and $R_3$ is a hydrogen atom or an alkylamino, dialkylamino or acylamino radical, in its D, L and D,L forms if $R_2$ is other than a hydrogen atom, as well as its metal salts, its addition salts with nitrogen-containing bases and, where they exist, its addition salts with acids.

3. A product according to Claim 1, characterised in that the symbol X is a sulphur atom, the symbol R is a carboxyl radical, the symbol $R_3$ represents a hydrogen atom or an alkylamino, dialkylamino or acylamino radical and the symbol $R_1$ represents a thienyl, furyl, phenyl, p-hydroxyphenyl, phenoxy or 3,4-dichloro-phenylthio radical and the symbol $R_2$ represents a hydrogen atom, or the symbol $R_1$ represents a phenyl or p-hydroxyphenyl radical and the symbol $R_2$ represents an amino radical, in its D, L and D,L forms if $R_2$ is other than a hydrogen atom, as well as its metal salts, its addition salts with nitrogen-containing bases and, where they exist, its addition salts with acids.

4. A product according to Claim 1, characterised in that the symbols X and R are defined as in Claim 1, the symbol $R_1$ is a thienyl radical and the symbol $R_2$ is a hydrogen atom, or the symbol $R_1$ is a phenyl or p-hydroxyphenyl radical and the symbol $R_2$ is an amino radical, the symbol $R_3$ represents a hydrogen atom, a phenyl radical, an acylamino radical optionally substituted at the nitrogen atom by an alkyl radical, a dialkylaminoethylamino radical, a 2-amino-ethylthioacetamido radical, a L-2-amino-2-carboxyethylthioacetamido radical or a radical of the structure $-A-R'_3$, in which A and $R'_3$ are defined as in Claim 1, in its D, L and D,L forms if $R_2$ is amino, as well as its metal salts, its addition salts with nitrogen-containing bases and, where they exist, it addition salts with acids.

5. A process for the preparation of a product according to Claim 1, characterised in that an acid of the general formula

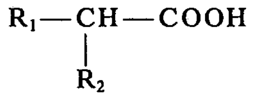

(in which $R_1$ and $R_2$ are defined as in Claim 1 and in which the amine group which $R_2$ can represent is protected beforehand) or a reactive derivative of this acid is reacted with a 7-amino-cephalosporin derivative of the general formula

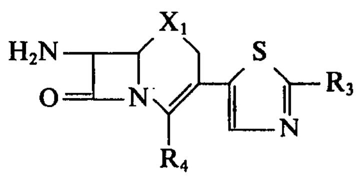

in which $R_3$, which is defined as in Claim 1, is protected if it contains an amino radical, $R_4$ is a carboxyl radical, a protected carboxyl radical or a carboxylato radical and $X_1$ is defined like X in Claim 1 or represents a sulphinyl radical after which, where appropriate, the sulphoxide obtained is reduced, the protective radicals are removed and the product obtained is optionally converted to a metal salt, an addition salt with a nitrogen-containing base or, where it exits, an addition salt with an acid.

A process according to Claim 5, characterised in that the reactive derivative employed is a halide, the anhydride, a mixed anhydride or a reactive ester of the acid of the general formula

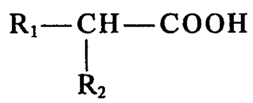

in which $R_1$ and $R_2$ are defined as in Claim 5.

7. A process according to Claim 5, characterised in that a 7-amino-cephalosporin is employed, in the formula of which $R_4$ is a carboxyl radical protected by a methoxymethyl, t.-butyl, 2,2,2-trichloroethyl, benzhydryl, p-nitrobenzyl or p-methoxybenzyl group.

8. A process for the preparation of a product according to Claim 1, for which $R_3$ is defined as in Claim 1 except for representing a $-AR'_3$ radical and R represents a carboxyl radical, characterised in that a product of the general formula

$R_3CSNH_2$

in which $R_3$ is defined as above is reacted with a cephalosporin derivative of the general formula

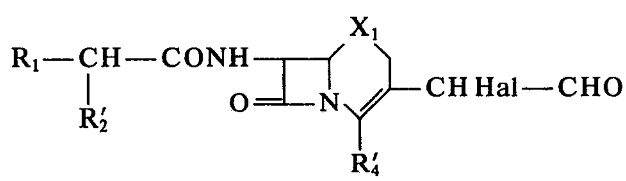

in which $R_1$ and $X_1$ are defined as in Claim 5, $R'_2$ is a hydrogen atom or a protected amino radical, $R'_4$ is a protected carboxyl radical and Hal represents a halogen atom followed, where appropriate, by a dehydrating agent after which, where necessary, the sulphoxide obtained is reduced, the protective groups are removed and the product obtained is optionlly converted to a metal salt, an addition salt with a nitrogen-containing base or, where it exists, an addition salt with an acid.

9. A process of preparation according to Claim 8, characterised in that a cephalosporin derivative of the general formula

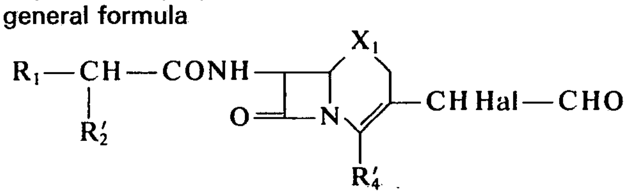

is employed, in which $R_1$, $R'_2$, $R'_4$ and X are defined as in Claim 8 and Hal represents a chlorine, bromine or iodine atom.

10. A process according to Claim 8, characterised in that a cephalosporin of the general formula

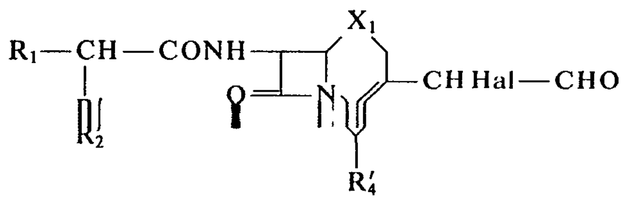

is employed, in which $R_1$, $R'_4$, $X_1$ and Hal are defined as in Claim 8 and $R'_2$ represents an amino radical protected by a t.-butoxycarbonyl, 2,2,2-trichloroethoxycarbonyl, trityl, benzyl, benzyloxycarbonyl, formyl, trifluoroacetyl, p-nitrobenzyloxycarbonyl, p-methoxybenzyloxycarbonyl or diphenylphosphinoyl group or by a radical of the general formula

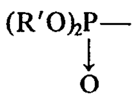

in which the symbol R' is alkyl, 2,2,2-trichloroethyl, phenyl or benzyl (the latter two being optionally substituted by a halogen atom by an alkyl, alkoxy or nitro radical) or the two symbols R' together form an alkylene radical containing 2 or carbon atoms.

11. A process for the preparation of a product according to Claim 1, for which $R_3$ represents an acylamino, benzoylamino or thenoylamino radical optionally substituted at the nitrogen atom or represents an alkoxycarbonylamino or dialkylaminomethyleneamino radical, a substituted acetamido radical or a radical of the structure $-AR'_3$, in which A is –NHCO– and $R'_3$ is defined as in Claim 1, characterised in that an amine of the general formula

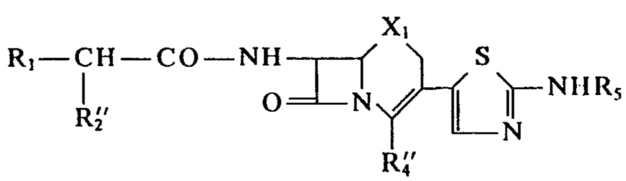

in which $R_1$ and $X_1$ are defined as in Claim 5, $R''_2$ represents a protected amino radical, $R_4''$ is a free or protected carboxyl radical and $R_5$ represents a hydrogen atom or an alkyl or phenyl radical is treated by any known method to form an amide, a carbamate or an amidine, without affecting the remainder of the molecule after which, where appropriate, the sulphoxide obtained is reduced, the protective radicals are removed where appropriate and the product obtained is optionally converted to a metal salt, an addition salt with a nitrogen-containing base or, where it exists, an addition salt with an acid.

12. A process for the preparation of a product according to Claim 1, for which $R_3$ is a radical of the structure $-AR'_3$ and R is a carboxylato radical, characterised in that a methyl halide, benzoylmethyl halide or carboxymethyl halide (wherein the acid group is free or protected) is reacted with a cephalosporin derivative of the general formula

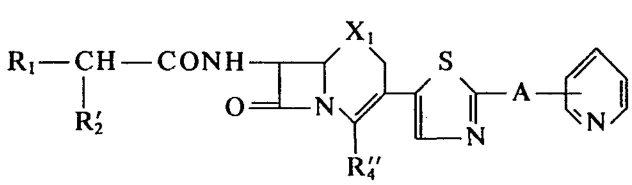

in which the radical A is substituted in the 3- or 4-position of the pyridyl ring, $R_1$ and A are defined as in Claim 1, $R'_2$ and $R''_4$ are defined as in Claim 11 and $X_1$ is defined as in Claim 5, after which, where appropriate, the sulphoxide obtained is reduced, the protective radicals are removed and the product obtained is optionally converted to a metal salt, an addition salt with a nitrogen-containing base or an addition salt with a strong acid, if these exist.

13. A process for the preparation of a product according to Claim 1, for which $R_3$ represents an acetamido radical substituted by a 2-amino-ethylthio or L-2-amino-2-carboxy-ethylthio radical and R represents a carboxyl radical, characterised in that cysteamine or cysteine, in which the amine group and, where appropriate, the acid group are protected beforehand, is reacted with a halogeno-acetamidothiazolyl-cephalosporin derivative of the general formula

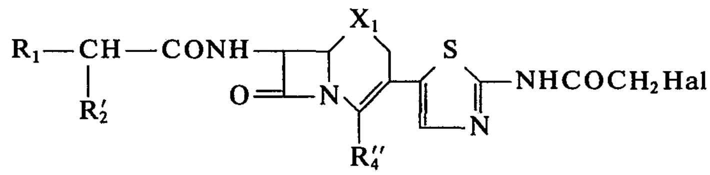

in which $R_1$ and $X_1$ are defined as in Claim 5, $R'_2$ and $R''_4$ are defined as in Claim 11 and Hal represents a chlorine, bromine or iodine atom, after which, where appropriate, the product obtained is reduced, the protective radicals are removed and the product obtained is optionally converted to a metal salt, an addition salt with a nitrogen-containing base or an addition salt with an acid.

14. Pharmaceutical composition, characterised in that it contains at least one product according to Claim 1, in association with one or more compatible and pharmaceutically acceptable diluents and adjuvants.

**Claim for the Contracting State AT**

Process for the preparation of a cephalosporin derivate of the general formula

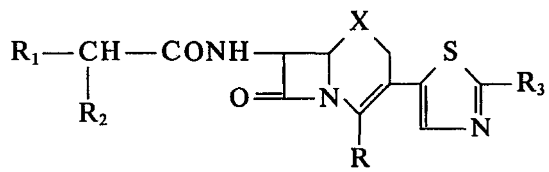

in which
the symbol $R_1$ represents a heterocyclyl radical chosen from among thienyl, furyl or 1,3-dithiol-2-on-4-yl or a phenyl, p-hdroxyphenyl, phenoxy or dichlorophenylthio radical and
the symbol $R_2$ represents a hydrogen atom or
the symbol $R_1$ is a phenyl or p-hydroxyphenyl radical and the symbol $R_2$ is an amino radical,
the symbol $R_3$ represents a hydrogen atom, a phenyl radical (optionally substituted by an acylamino radical), an alkylthio, alkylamino, dialkylamino or anilino radical, an acylamino, benzoylamino or thenoylamino radical, optionally substituted at the nitrogen atom (by an alkyl or phenyl radical), an alkoxycarbonylamino, dialkylaminoethylamino, dialkylaminomethyleneamino or alkylidenehydrazo radical, an acetamido radical substituted by an amino, 2-aminoethylthio or L-2-amino-2-carboxyethylthio radical, or a radical of the structure $-AR'_3$in which A represents a bivalent radical chosen from among $-CH_2-$, $-NH-$ or $-NHCO-$ and $R'_3$ represents a 1-methyl-3-(or -4-) pyridinio or 1-benzoylmethyl-3- (or -4-) pyridinio or 1-carboxymethyl-3- (or -4-) pyridinio radical,
the symbol R represents a carboxyl radical or represents a carboxylato radical if $R'_3$ is a substituted pyridinio radical, and
the symbol X represents an oxygen or sulphur atom, it being understood that the alkyl or acyl radicals and portions mentioned above are straight or branched and contain 1 to 4 carbon atoms,
in its D, L and D,L forms if $R_2$ is other than a hydrogen atom, as well as its metal salts, its addition salts with nitrogen-containing bases and, where they exist, its addition salts with acids, characterised in that an acid of the general formula

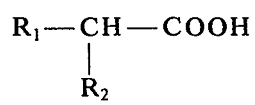

(in which $R_1$ and $R_2$ are defined as above and in which the amine group which $R_2$ can represent is protected beforehand) or a reactive derivative of this acid is reacted with a 7-amino-cephalosporin derivative of the general formula

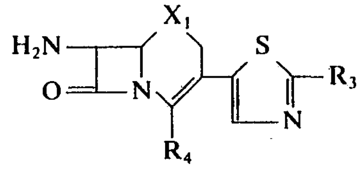

in which $R_3$, which is defined as in Claim 1, is protected if it contains an amino radical, $R_4$ is a carboxyl radical, a protected carboxyl radical or a carboxylato radical and $X_1$ is defined like X in Claim 1 or represents a sulphinyl radical after which, where appropriate, the sulphoxide obtained is reduced, the protective radicals are removed and the product obtained is optionally converted to a metal salt, an addition salt with a nitrogen-containing base or, where it exists, an addition salt with an acid, or if $R_3$ is defined as above except for representing a $-AR'_3$ radical and R represents a carboxyl radical, a product of the general formula

$R_3CSNH_2$

in which $R_3$ is defined as above is reacted with a cephalosporin derivative of the general formula

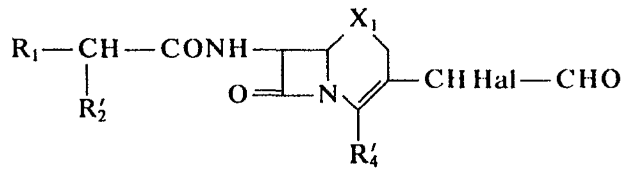

in which $R_1$ and $X_1$ are defined as above, $R'_2$ is a hydrogen atom or a protected amino radical, $R'_4$ is a protected carboxyl radical and Hal represents a halogen atom followed, where appropriate, by a dehydrating agent after which, where necessary, the sulphoxide obtained is reduced, the protective groups are removed and the product obtained is optionally converted to a metal salt, an addition salt with a nitrogen-containing base or, where it exists, an addition salt with an acid, or if $R_3$ represents an acylamino, benzoylamino or thenoylamino radical optionally substituted at the nitrogen atom or represents an alkoxycarbonylamino or dialkylaminomethylamino radical, a substituted acetamido radical or a radical of the structure $-AR'_3$, in which A is $-NHCO-$ and $R'_3$ is defined as above, an amine of the general formula

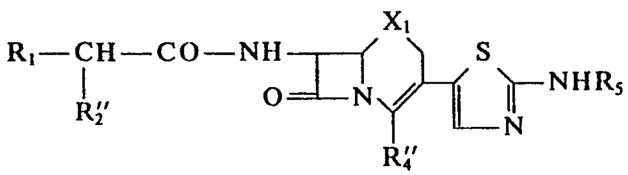

in which $R_1$ and $X_1$ are defined as above, $R''_2$ represents a protected amino radical, $R''_4$ is a free or protected carboxyl radical and $R_5$ represents a hydrogen atom or an alkyl or phenyl radical is treated by any known method to form an amide, a carbamate or an amidine, without affecting the remainder of the molecule after which, where appropriate, the sulphoxide obtained is reduced, the protective radicals are removed where appropriate and the product obtained is optionally converted to a metal salt, an addition salt with a nitrogen-containing base or, where it exists, an addition salt with an acid, or if $R_3$ is a radical of the structure $-AR'_3$ and R is a carboxylato radical, a methyl halide, benzoylmethyl halide or carboxymethyl halide (wherein the acid group is free or protected) is reacted with a cephalosporin derivative of the general formula

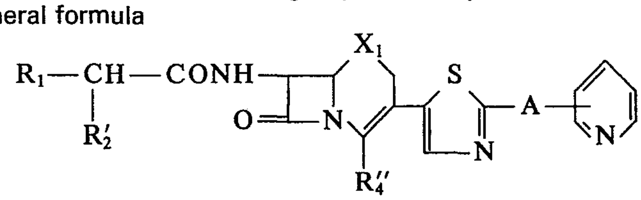

in which the radical A is substituted in the 3- or 4-position of the pyridyl ring and $R_1$, $R'_2$, $R''_4$, $X_1$ and A are defined as above after which, where appropriate, the sulphoxide obtained is reduced, the protective radicals are removed and the product obtained is optionally converted to a metal salt, an addition salt with a nitrogen-containing base or an addition salt with a strong acid, where these exist, or, if $R_3$ represents an acetamido radical substituted by a 2-amino-ethylthio or L-2-amino-2-carboxy-ethylthio radical and R represents a carboxyl radical, cysteamine or cysteine, the amino group and, where appropriate, the acid group of which have beforehand been protected, is reacted with a halogenoacetamido-thiazolyl-cephalosporin derivative of the general formula

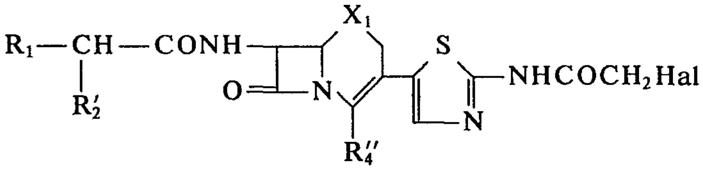

in which $R_1$, $R'_2$, $R''_4$ and $X_1$ are defined as above and Hal represents a chlorine, bromine or iodine atom, after which, where appropriate, the product obtained is reduced, the protective radicals are removed and the product obtained is optionally converted to a metal salt, an addition salt with a nitrogen-containing base or an addition salt with an acid.